# EUROPEAN PATENT SPECIFICATION

(11) **EP 3 282 496 B1**
(45) Date of publication and mention of the grant of the patent: **26.04.2023**
(21) Application number: 16776444.8
(22) Date of filing: 29.03.2016
(51) Int. Cl.: C07C 211/54, C07C 211/58, C07C 211/61, C07C 255/51, C07D 209/14, C07D 307/91, H01L 51/54

(54) **ORGANIC ELECTROLUMINESCENT ELEMENT**
ORGANISCHES ELEKTROLUMINESZENTES ELEMENT
ÉLÉMENT ÉLECTROLUMINESCENT ORGANIQUE

(30) Priority: 10.04.2015 JP 2015080611
(43) Date of publication of application: 14.02.2018
(73) Proprietor: Hodogaya Chemical Co., Ltd., Chuo-ku Tokyo 104-0028 (JP)
(72) Inventor: HAYASHI, Shuichi, Tokyo 104-0028 (JP); TOGASHI, Kazunori, Tokyo 104-0028 (JP); MOCHIZUKI, Shunji, Tokyo 104-0028 (JP); NAITO, Keigo, Tokyo 104-0028 (JP)
(74) Representative: Müller-Boré & Partner Patentanwälte PartG mbB
(86) International application number: PCT/JP2016/060129
(87) International publication number: WO 2016/163276

(56) References cited:
- EP-A1- 2 924 753
- EP-A1- 2 963 696
- EP-A1- 2 999 019
- EP-A1- 3 002 796
- EP-A1- 3 002 797
- EP-A1- 3 229 284
- EP-A1- 3 244 462
- WO-A1-2005/063684
- WO-A1-2011/131185
- WO-A1-2011/134458
- WO-A1-2013/083712
- JP-A- 2011 222 831
- JP-A- 2013 191 649
- JP-A- 2013 531 360
- US-A1- 2012 223 296

## Description

### Technical Field

The present invention relates to an organic electroluminescent device which is a preferred self-luminous device for various display devices. Specifically, this invention relates to organic electroluminescent devices (hereinafter referred to as organic EL devices) using specific arylamine compounds doped with an electron acceptor.

### Background Art

The organic EL device is a self-luminous device and has been actively studied for their brighter, superior visibility and the ability to display clearer images in comparison with liquid crystal devices.

In 1987, C. W. Tang and colleagues at Eastman Kodak developed a laminated structure device using materials assigned with different roles, realizing practical applications of an organic EL device with organic materials. These researchers laminated an electron-transporting phosphor and a hole-transporting organic substance, and injected both charges into a phosphor layer to cause emission in order to obtain a high luminance of 1,000 cd/m² or more at a voltage of 10 V or less (refer to Patent Documents 1 and 2, for example).

To date, various improvements have been made for practical applications of the organic EL device. Various roles of the laminated structure are further subdivided to provide an electroluminescence device that includes an anode, a hole injection layer, a hole transport layer, a light emitting layer, an electron transport layer, an electron injection layer, and a cathode successively formed on a substrate, and high efficiency and durability have been achieved by the electroluminescence device (refer to Non-Patent Document 1, for example).

Further, there have been attempts to use triplet excitons for further improvements of luminous efficiency, and the use of a phosphorescence-emitting compound has been examined (refer to Non-Patent Document 2, for example).

Devices that use light emission caused by thermally activated delayed fluorescence (TADF) have also been developed. In 2011, Adachi et al. at Kyushu University, National University Corporation realized 5.3% external quantum efficiency with a device using a thermally activated delayed fluorescent material (refer to Non-Patent Document 3, for example).

The light emitting layer can be also fabricated by doping a charge-transporting compound generally called a host material, with a fluorescent compound, a phosphorescence-emitting compound, or a delayed fluorescent-emitting material. As described in the Non-Patent Document, the selection of organic materials in an organic EL device greatly influences various device characteristics such as efficiency and durability (refer to Non-Patent Document 2, for example).

In an organic EL device, charges injected from both electrodes recombine in a light emitting layer to cause emission. What is important here is how efficiently the hole and electron charges are transferred to the light emitting layer in order to form a device having excellent carrier balance. The probability of hole-electron recombination can be improved by improving hole injectability and electron blocking performance of blocking injected electrons from the cathode, and high luminous efficiency can be obtained by confining excitons generated in the light emitting layer. The role of a hole transport material is therefore important, and there is a need for a hole transport material that has high hole injectability, high hole mobility, high electron blocking performance, and high durability to electrons.

Heat resistance and amorphousness of the materials are also important with respect to the lifetime of the device. The materials with low heat resistance cause thermal decomposition even at a low temperature by heat generated during the drive of the device, which leads to the deterioration of the materials. The materials with low amorphousness cause crystallization of a thin film even in a short time and lead to the deterioration of the device. The materials in use are therefore required to have characteristics of high heat resistance and satisfactory amorphousness.

N,N'-diphenyl-N,N'-di(α-naphthyl)benzidine (NPD) and various aromatic amine derivatives are known as the hole transport materials used for the organic EL device (refer to Patent Documents 1 and 2, for example). Although NPD has desirable hole transportability, its glass transition point (Tg), which is an index of heat resistance, is as low as 96°C, which causes the degradation of device characteristics by crystallization under a high-temperature condition (refer to Non-Patent Document 4, for example). The aromatic amine derivatives described in the Patent Documents include a compound known to have an excellent hole mobility of 10⁻³ cm²/Vs or higher (refer to Patent Documents 1 and 2, for example). However, since the compound is insufficient in terms of electron blocking performance, some of the electrons pass through the light emitting layer, and improvements in luminous efficiency cannot be expected. For such a reason, a material with higher electron blocking performance, a more stable thin-film state and higher heat resistance is needed for higher efficiency. Although an aromatic amine derivative having high durability is reported (refer to Patent Document 3, for example), the derivative is used as a charge transporting material used in an electrophotographic photoconductor, and there is no example of using the derivative in the organic EL device.

Arylamine compounds having a substituted carbazole structure are proposed as compounds improved in the characteristics such as heat resistance and hole injectability (refer to Patent Documents 4 and 5, for example). Further, it is proposed that hole injectability can be improved by p-doping materials such as trisbromophenylamine hexachloroantimony, radialene derivatives, and F4-TCNQ into a material commonly used for the hole injection layer or the hole transport layer (refer to Patent Document 6 and Non-Patent Document 5). Patent document WO 2013/083712 A1 discloses an organic electroluminescent device comprising hole transport layers including both an arylamine compound (N4,N4,N4",N4"-tetra([1,1'-biphenyl]4-yl)-[1,1':4',1" -terphenyl]-4,4"-diamine) and an electron acceptor (2,2,2"-(cyclopropane- 1,2,3-triylidene)tris(2-(p- cyanotetrafluorophenyl)acetonitrile). However, while the devices using these compounds for the hole injection layer or the hole transport layer have been improved in lower driving voltage and heat resistance, luminous efficiency and the like, the improvements are still insufficient. Further lower driving voltage and higher luminous efficiency are therefore needed.

In order to improve characteristics of the organic EL device and to improve the yield of the device production, it has been desired to develop a device having high luminous efficiency, low driving voltage and a long lifetime by using in combination the materials that excel in hole and electron injection/transport performances, stability as a thin film and durability, permitting holes and electrons to be highly efficiently recombined together.

Further, in order to improve characteristics of the organic EL device, it has been desired to develop a device that maintains carrier balance and has high efficiency, low driving voltage and a long lifetime by using in combination the materials that excel in hole and electron injection/transport performances, stability as a thin film and durability.

### Citation List

### Patent Literature

PTL 1: JP-A-8-048656
PTL 2: Japanese Patent No. 3194657
PTL 3: Japanese Patent No. 4943840
PTL 4: JP-A-2006-151979
PTL 5: WO2008/62636
PTL 6: WO2014/009310
PTL 7: WO2005/115970
PTL 8: JP-A-7-126615
PTL 9: JP-A-2005-108804
PTL 10: WO2011/059000
PTL 10: WO2003/060956
PTL 10: KR-A-2013-060157

### Non Patent Literature

NPL 1: The Japan Society of Applied Physics, 9th Lecture Preprints, pp. 55 to 61 (2001)
NPL 2: The Japan Society of Applied Physics, 9th Lecture Preprints, pp. 23 to 31 (2001)
NPL 3: Appl. Phys. Let., 98, 083302 (2011)
NPL 4: Organic EL Symposium, the 3rd Regular presentation Preprints, pp. 13 to 14 (2006)
NPL 5: Appl. Phys. Let., 89, 253506 (2006)

### Summary of Invention

### Technical Problem

An object of the invention is to provide an organic EL device that has a low driving voltage and a high luminous efficiency and also has a long lifetime, by combining various materials for an organic EL device that are excellent in injection and transport capabilities of holes and electrons, electron blocking capability, and stability and durability in a thin film state, as materials for an organic EL device having a high luminous efficiency and high durability, in such a manner that the characteristics of the materials each are effectively exhibited, and to provide an organic EL device that retains a low driving voltage or is effectively suppressed in the driving voltage rise, by controlling the doping concentration of the electron acceptor and/or the film thickness of the organic layer containing the electron acceptor.

Examples of the physical characteristics that the organic EL device provided by the invention should have include (1) a low light emission starting voltage, (2) a low practical driving voltage, (3) small rise of the driving voltage, (4) high luminous efficiency and high power efficiency, and (5) long lifetime.

### Solution to Problem

For achieving the aforementioned object, the present inventors have focused the fact that an arylamine compound doped with an electron acceptor is excellent in hole injection and transport capabilities and stability and durability of a thin film, have selected a particular arylamine compound (having a particular structure and a particular ionization potential), have doped a material for a hole injection layer with an electron acceptor to enable efficient injection and transport of holes from an anode, combining a particular arylamine compound (having a particular structure) that is not doped with an electron acceptor as a material for a hole transport layer, so as to produce various organic EL devices, and have earnestly evaluated the characteristics of the devices. Furthermore, the inventors have produced various organic EL devices having various doping concentrations of the electron acceptor in the hole injection layer and various thicknesses of the hole injection layer, and have earnestly evaluated the characteristics of the devices. As a result, the invention has been completed.

According to the present invention, an organic electroluminescent device according to claim 1 is provided.

The organic EL device according to the claimed invention is of the top emission type and has at least an anode, a hole injection layer, a hole transport layer, a light emitting layer, an electron transport layer, and a cathode, in this order, wherein the hole injection layer includes an arylamine compound of the following general formula (1) and an electron acceptor:

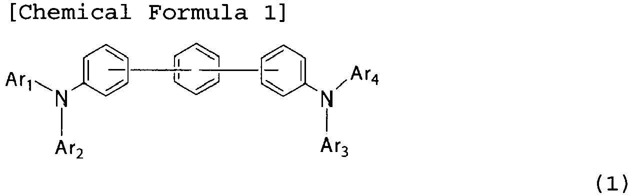

In the formula, Ar₁ to Ar₄ may be the same or different, and represent a substituted or unsubstituted aromatic hydrocarbon group, a substituted or unsubstituted aromatic heterocyclic group, or a substituted or unsubstituted condensed polycyclic aromatic group. Ar₁ to Ar₄ are as defined in claim 1. Moreover, in the organic EL device according to the claimed invention, the hole transport layer has a film thickness of 100 to 200 nm, said hole transport layer has the same arylamine compound as the hole injection layer, but no electron acceptor. Said electron acceptor is contained in an amount of 0.5 to 30% by weight based on the total hole injection layer and the hole injection layer has a film thickness of 5 to 150 nm.

In an embodiment, the layers that are adjacent to the light emitting layer do not include an electron acceptor.

In an embodiment, the electron acceptor is an electron acceptor selected from trisbromophenylamine hexachloroantimony, tetracyanoquinodimethane (TCNQ), 2,3,5,6-tetrafluoro-tetracyano-1,4-benzoquinodimethane (F4TCNQ), and a radialene derivative.

In an embodiment, the electron acceptor is a radialene derivative of the following general formula (2):

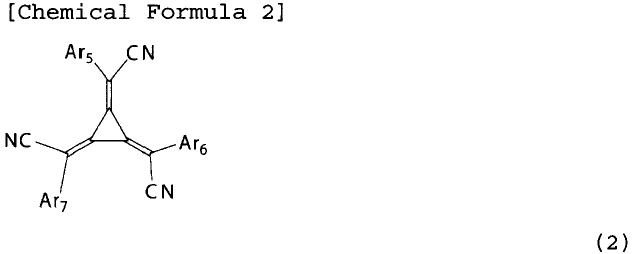

In the formula, Ar₅ to Ar₇ may be the same or different, and represent an aromatic hydrocarbon group, an aromatic heterocyclic group, or a condensed polycyclic aromatic group, having an electron acceptor group as a substituent.

5) The organic EL device of any one of 1) to 4), wherein the arylamine compound of the general formula (1) has an ionization potential of 5.4 to 5.8 eV.

In an embodiment, the hole transport layer includes an arylamine compound having a structure in which two to six triphenylamine structures are joined within a molecule via a single bond or a divalent group that does not contain a heteroatom.

In an embodiment, the arylamine compound having a structure in which two to six triphenylamine structures are joined within a molecule via a single bond or a divalent group that does not contain a heteroatom is an arylamine compound of the following general formula (3).

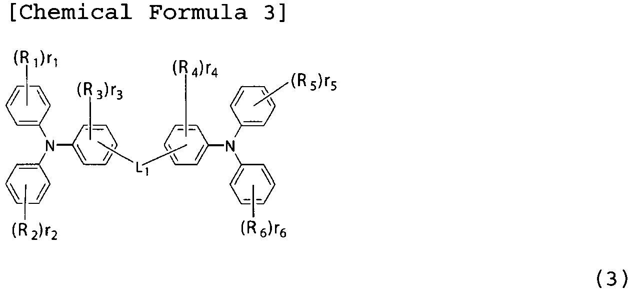

In the formula, R₁ to R₆ represent a deuterium atom, a fluorine atom, a chlorine atom, cyano, nitro, linear or branched alkyl of 1 to 6 carbon atoms that may have a substituent, cycloalkyl of 5 to 10 carbon atoms that may have a substituent, linear or branched alkenyl of 2 to 6 carbon atoms that may have a substituent, linear or branched alkyloxy of 1 to 6 carbon atoms that may have a substituent, cycloalkyloxy of 5 to 10 carbon atoms that may have a substituent, a substituted or unsubstituted aromatic hydrocarbon group, a substituted or unsubstituted aromatic heterocyclic group, a substituted or unsubstituted condensed polycyclic aromatic group, or substituted or unsubstituted aryloxy. r₁ to r₆ may be the same or different, r₁, r₂, r₅, and r₆ representing an integer of 0 to 5, and r₃ and r₄ representing an integer of 0 to 4. When r₁, r₂, r₅, and r₆ are an integer of 2 to 5, or when r₃ and r₄ are an integer of 2 to 4, R₁ to R₆, a plurality of which bind to the same benzene ring, may be the same or different and may bind to each other via a single bond, substituted or unsubstituted methylene, an oxygen atom, or a sulfur atom to form a ring. L₁ represents a divalent linking group or a single bond.

In an embodiment, the arylamine compound having a structure in which two to six triphenylamine structures are joined within a molecule via a single bond or a divalent group that does not contain a heteroatom is an arylamine compound of the following general formula (4).

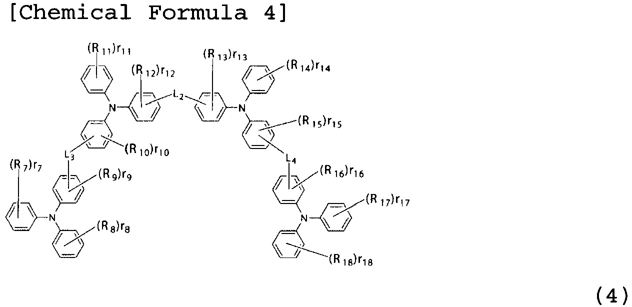

In the formula, R₇ to R₁₈ represent a deuterium atom, a fluorine atom, a chlorine atom, cyano, nitro, linear or branched alkyl of 1 to 6 carbon atoms that may have a substituent, cycloalkyl of 5 to 10 carbon atoms that may have a substituent, linear or branched alkenyl of 2 to 6 carbon atoms that may have a substituent, linear or branched alkyloxy of 1 to 6 carbon atoms that may have a substituent, cycloalkyloxy of 5 to 10 carbon atoms that may have a substituent, a substituted or unsubstituted aromatic hydrocarbon group, a substituted or unsubstituted aromatic heterocyclic group, a substituted or unsubstituted condensed polycyclic aromatic group, or substituted or unsubstituted aryloxy. R₇ to r₁₈ may be the same or different, r₇, r₈, r₁₁, r₁₄, r₁₇, and r₁₈ representing an integer of 0 to 5, and r₉, r₁₀, r₁₂, r₁₃, r₁₅, and r₁₆ representing an integer of 0 to 4. When r₇, r₈, r₁₁, r₁₄, r₁₇, and r₁₈ are an integer of 2 to 5, or when r₉, r₁₀, r₁₂, r₁₃, r₁₅, and r₁₆ are an integer of 2 to 4, R₇ to R₁₈, a plurality of which bind to the same benzene ring, may be the same or different and may bind to each other via a single bond, substituted or unsubstituted methylene, an oxygen atom, or a sulfur atom to form a ring. L₂, L₃, and L₄ may be the same or different, and represent a divalent linking group or a single bond.

In an embodiment, the electron transport layer includes a compound of the following general formula (5) having an anthracene ring structure.

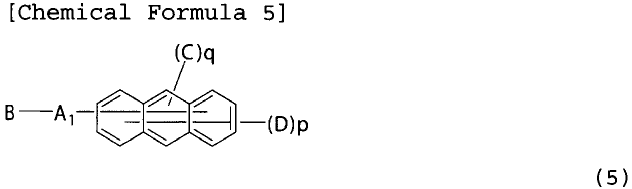

In the formula, A₁ represents a divalent group of a substituted or unsubstituted aromatic hydrocarbon, a divalent group of a substituted or unsubstituted aromatic heterocyclic ring, a divalent group of substituted or unsubstituted condensed polycyclic aromatics, or a single bond. B represents a substituted or unsubstituted aromatic heterocyclic group. C represents a substituted or unsubstituted aromatic hydrocarbon group, a substituted or unsubstituted aromatic heterocyclic group, or a substituted or unsubstituted condensed polycyclic aromatic group. D may be the same or different, and represents a hydrogen atom, a deuterium atom, a fluorine atom, a chlorine atom, cyano, trifluoromethyl, linear or branched alkyl of 1 to 6 carbon atoms, a substituted or unsubstituted aromatic hydrocarbon group, a substituted or unsubstituted aromatic heterocyclic group, or a substituted or unsubstituted condensed polycyclic aromatic group. p represents 7 or 8, and q represents 1 or 2 while maintaining a relationship that a sum of p and q is 9.

In an embodiment, the light emitting layer includes a blue light emitting dopant.

In an embodiment, the light emitting layer includes a pyrene derivative, which is a blue light emitting dopant.

In an embodiment, the light emitting layer includes an anthracene derivative.

In an embodiment, the light emitting layer includes a host material which is the anthracene derivative.

Specific examples of the "aromatic hydrocarbon group", the "aromatic heterocyclic group", or the "condensed polycyclic aromatic group" in the "substituted or unsubstituted aromatic hydrocarbon group", the "substituted or unsubstituted aromatic heterocyclic group", or the "substituted or unsubstituted condensed polycyclic aromatic group" represented by Ar₁ and Ar₂ in the general formula (1) include phenyl, biphenylyl, terphenylyl, anthracenyl, phenanthrenyl, fluorenyl, indenyl, pyrenyl, perylenyl, fluoranthenyl, triphenylenyl, pyridyl, pyrimidinyl, triazinyl, furyl, pyrrolyl, thienyl, quinolyl, isoquinolyl, benzofuranyl, benzothienyl, indolyl, carbazolyl, benzoxazolyl, benzothiazolyl, quinoxalinyl, benzoimidazolyl, pyrazolyl, dibenzofuranyl, dibenzothienyl, naphthyridinyl, phenanthrolinyl, acridinyl, and carbolinyl. It Specific examples of the "aromatic hydrocarbon group", the "aromatic heterocyclic group", or the "condensed polycyclic aromatic group" in the "substituted or unsubstituted aromatic hydrocarbon group", the "substituted or unsubstituted aromatic heterocyclic group", or the "substituted or unsubstituted condensed polycyclic aromatic group" represented by Ar₃ in the general formula (1) include phenyl, terphenylyl, naphthyl, anthracenyl, phenanthrenyl, fluorenyl, indenyl, pyrenyl, perylenyl, fluoranthenyl, triphenylenyl, pyridyl, pyrimidinyl, triazinyl, furyl, pyrrolyl, thienyl, quinolyl, isoquinolyl, benzofuranyl, benzothienyl, indolyl, carbazolyl, benzoxazolyl, benzothiazolyl, quinoxalinyl, benzoimidazolyl, pyrazolyl, dibenzofuranyl, dibenzothienyl, naphthyridinyl, phenanthrolinyl, acridinyl, and carbolinyl.

Specific examples of the "aromatic hydrocarbon group", the "aromatic heterocyclic group", or the "condensed polycyclic aromatic group" in the "substituted or unsubstituted aromatic hydrocarbon group", the "substituted or unsubstituted aromatic heterocyclic group", or the "substituted or unsubstituted condensed polycyclic aromatic group" represented by Ar₄ in the general formula (1) include phenyl, biphenylyl, terphenylyl, naphthyl, anthracenyl, phenanthrenyl, fluorenyl, indenyl, pyrenyl, perylenyl, fluoranthenyl, triphenylenyl, pyridyl, pyrimidinyl, triazinyl, furyl, pyrrolyl, thienyl, quinolyl, isoquinolyl, benzofuranyl, benzothienyl, indolyl, carbazolyl, benzoxazolyl, benzothiazolyl, quinoxalinyl, benzoimidazolyl, pyrazolyl, dibenzofuranyl, dibenzothienyl, naphthyridinyl, phenanthrolinyl, acridinyl, and carbolinyl.

Specific examples of the "substituent" in the "substituted aromatic hydrocarbon group", the "substituted aromatic heterocyclic group", or the "substituted condensed polycyclic aromatic group" represented by Ar₁ to Ar₄ in the general formula (1) include a deuterium atom; cyano; nitro; halogen atoms such as a fluorine atom, a chlorine atom, a bromine atom, and an iodine atom; linear or branched alkyls of 1 to 6 carbon atoms such as methyl, ethyl, n-propyl, isopropyl, n-butyl, isobutyl, tert-butyl, n-pentyl, isopentyl, neopentyl, and n-hexyl; linear or branched alkyloxys of 1 to 6 carbon atoms such as methyloxy, ethyloxy, and propyloxy; alkenyls such as allyl; aryloxys such as phenyloxy and tolyloxy; arylalkyloxys such as benzyloxy and phenethyloxy; aromatic hydrocarbon groups or condensed polycyclic aromatic groups such as phenyl, biphenylyl, terphenylyl, naphthyl, anthracenyl, phenanthrenyl, fluorenyl, indenyl, pyrenyl, perylenyl, fluoranthenyl, and triphenylenyl; aromatic heterocyclic groups such as pyridyl, pyrimidinyl, triazinyl, thienyl, furyl, pyrrolyl, quinolyl, isoquinolyl, benzofuranyl, benzothienyl, indolyl, carbazolyl, benzoxazolyl, benzothiazolyl, quinoxalinyl, benzoimidazolyl, pyrazolyl, dibenzofuranyl, dibenzothienyl, and carbolinyl; arylvinyls such as styryl and naphthylvinyl; acyls such as acetyl and benzoyl; and silyls, such as trimethylsilyl and triphenylsilyl. These substituents may be further substituted with the exemplified substituents above. These substituents may bind to each other via a single bond, substituted or unsubstituted methylene, an oxygen atom, or a sulfur atom to form a ring.

Specific examples of the "electron acceptor group" in the "aromatic hydrocarbon group, aromatic heterocyclic group, or condensed polycyclic aromatic ring having an electron acceptor group as a substituent" represented by Ar₅ to Ar₇ in the general formula (2) include a fluorine atom, a chlorine atom, a bromine atom, cyano, trimethylfluoro, and nitro.

Examples of the "aromatic hydrocarbon group", the "aromatic heterocyclic group", or the "condensed polycyclic aromatic group" in the "aromatic hydrocarbon group, aromatic heterocyclic group, or condensed polycyclic aromatic ring having an electron acceptor group as a substituent" represented by Ar₅ to Ar₇ in the general formula (2) include the same groups exemplified as the "aromatic hydrocarbon group", the "aromatic heterocyclic group", or the "condensed polycyclic aromatic group" in the "substituted or unsubstituted aromatic hydrocarbon group", the "substituted or unsubstituted aromatic heterocyclic group", or the "substituted or unsubstituted condensed polycyclic aromatic group" represented by Ar₁ to Ar₄ in the general formula (1).

These groups may have a substituent, in addition to the electron acceptor group, and specific examples of the substituent include a deuterium atom; aromatic hydrocarbon groups or condensed polycyclic aromatic groups such as phenyl, biphenylyl, terphenylyl, naphthyl, anthracenyl, phenanthrenyl, fluorenyl, indenyl, pyrenyl, perylenyl, fluoranthenyl, and triphenylenyl; and aromatic heterocyclic groups such as pyridyl, pyrimidinyl, triazinyl, thienyl, furyl, pyrrolyl, quinolyl, isoquinolyl, benzofuranyl, benzothienyl, indolyl, carbazolyl, benzoxazolyl, benzothiazolyl, quinoxalinyl, benzoimidazolyl, pyrazolyl, dibenzofuranyl, dibenzothienyl, and carbolinyl. These substituents may be further substituted with the exemplified substituents or electron acceptor groups above. These substituents may bind to each other via a single bond, substituted or unsubstituted methylene, an oxygen atom, or a sulfur atom to form a ring.

Specific examples of the "linear or branched alkyl of 1 to 6 carbon atoms", the "cycloalkyl of 5 to 10 carbon atoms", or the "linear or branched alkenyl of 2 to 6 carbon atoms" in the "linear or branched alkyl of 1 to 6 carbon atoms that may have a substituent", the "cycloalkyl of 5 to 10 carbon atoms that may have a substituent", or the "linear or branched alkenyl of 2 to 6 carbon atoms that may have a substituent" represented by R₁ to R₆ in the general formula (3) include methyl, ethyl, n-propyl, isopropyl, n-butyl, isobutyl, tert-butyl, n-pentyl, isopentyl, neopentyl, n-hexyl, cyclopentyl, cyclohexyl, 1-adamantyl, 2-adamantyl, vinyl, allyl, isopropenyl, and 2-butenyl. These groups may bind to each other via a single bond, substituted or unsubstituted methylene, an oxygen atom, or a sulfur atom to form a ring.

Specific examples of the "substituent" in the "linear or branched alkyl of 1 to 6 carbon atoms that has a substituent", the "cycloalkyl of 5 to 10 carbon atoms that has a substituent", or the "linear or branched alkenyl of 2 to 6 carbon atoms that has a substituent" represented by R₁ to R₆ in the general formula (3) include a deuterium atom; cyano; nitro; halogen atoms such as a fluorine atom, a chlorine atom, a bromine atom, and an iodine atom; linear or branched alkyloxys of 1 to 6 carbon atoms such as methyloxy, ethyloxy, and propyloxy; alkenyls such as allyl; aryloxys such as phenyloxy and tolyloxy; arylalkyloxys such as benzyloxy and phenethyloxy; aromatic hydrocarbon groups or condensed polycyclic aromatic groups such as phenyl, biphenylyl, terphenylyl, naphthyl, anthracenyl, phenanthrenyl, fluorenyl, indenyl, pyrenyl, perylenyl, fluoranthenyl, and triphenylenyl; and aromatic heterocyclic groups such as pyridyl, pyrimidinyl, triazinyl, thienyl, furyl, pyrrolyl, quinolyl, isoquinolyl, benzofuranyl, benzothienyl, indolyl, carbazolyl, benzoxazolyl, benzothiazolyl, quinoxalinyl, benzoimidazolyl, pyrazolyl, dibenzofuranyl, dibenzothienyl, and carbolinyl. These substituents may be further substituted with the exemplified substituents above. These substituents may bind to each other via a single bond, substituted or unsubstituted methylene, an oxygen atom, or a sulfur atom to form a ring.

Specific examples of the "linear or branched alkyloxy of 1 to 6 carbon atoms" or the "cycloalkyloxy of 5 to 10 carbon atoms" in the "linear or branched alkyloxy of 1 to 6 carbon atoms that may have a substituent" or the "cycloalkyloxy of 5 to 10 carbon atoms that may have a substituent" represented by R₁ to R₆ in the general formula (3) include methyloxy, ethyloxy, n-propyloxy, isopropyloxy, n-butyloxy, tert-butyloxy, n-pentyloxy, n-hexyloxy, cyclopentyloxy, cyclohexyloxy, cycloheptyloxy, cyclooctyloxy, 1-adamantyloxy, and 2-adamantyloxy. These groups may bind to each other via a single bond, substituted or unsubstituted methylene, an oxygen atom, or a sulfur atom to form a ring.

These groups may have a substituent. Examples of the substituent include the same groups exemplified as the "substituent" in the "linear or branched alkyl of 1 to 6 carbon atoms that has a substituent", the "cycloalkyl of 5 to 10 carbon atoms that has a substituent", or the "linear or branched alkenyl of 2 to 6 carbon atoms that has a substituent" represented by R₁ to R₆ in the general formula (3), and possible embodiments may also be the same embodiments as the exemplified embodiments.

Examples of the "aromatic hydrocarbon group", the "aromatic heterocyclic group", or the "condensed polycyclic aromatic group" in the "substituted or unsubstituted aromatic hydrocarbon group", the "substituted or unsubstituted aromatic heterocyclic group", or the "substituted or unsubstituted condensed polycyclic aromatic group" represented by R₁ to R₆ in the general formula (3) include the same groups exemplified as the groups for the "aromatic hydrocarbon group", the "aromatic heterocyclic group", or the "condensed polycyclic aromatic group" in the "substituted or unsubstituted aromatic hydrocarbon group", the "substituted or unsubstituted aromatic heterocyclic group", or the "substituted or unsubstituted condensed polycyclic aromatic group" represented by Ar₁ to Ar₄ in the general formula (1). These groups may bind to each other via a single bond, substituted or unsubstituted methylene, an oxygen atom, or a sulfur atom to form a ring.

These groups may have a substituent. Examples of the substituent include the same groups exemplified as the "substituent" in the "substituted aromatic hydrocarbon group", the "substituted aromatic heterocyclic group", or the "substituted condensed polycyclic aromatic group" represented by Ar₁ to Ar₄ in the general formula (1), and possible embodiments may also be the same embodiments as the exemplified embodiments.

Specific examples of the "aryloxy group" in the "substituted or unsubstituted aryloxy group" represented by R₁ to R₆ in the general formula (3) include phenyloxy, biphenylyloxy, terphenylyloxy, naphthyloxy, anthracenyloxy, phenanthrenyloxy, fluorenyloxy, indenyloxy, pyrenyloxy, and perylenyloxy. These groups may bind to each other via a single bond, substituted or unsubstituted methylene, an oxygen atom, or a sulfur atom to form a ring.

These groups may have a substituent. Examples of the substituent include the same groups exemplified as the "substituent" in the "substituted aromatic hydrocarbon group", the "substituted aromatic heterocyclic group", or the "substituted condensed polycyclic aromatic group" represented by Ar₁ to Ar₄ in the general formula (1), and possible embodiments may also be the same embodiments as the exemplified embodiments.

In the general formula (3), r₁ to r₆ may be the same or different, r₁, r₂, r₅, and r₆ representing an integer of 0 to 5, and r₃ and r₄ representing an integer of 0 to 4. When r₁, r₂, r₅, and r₆ are an integer of 2 to 5, or when r₃ and r₄ are an integer of 2 to 4, R₁ to R₆, a plurality of which bind to the same benzene ring, may be the same or different and may bind to each other via a single bond, substituted or unsubstituted methylene, an oxygen atom, or a sulfur atom to form a ring.

Examples of the "divalent linking group" represented by L₁ in the general formula (3) include "linear or branched alkylenes of 1 to 6 carbon atoms", such as methylene, ethylene, n-propylylene, isopropylylene, n-butylylene, isobutylylene, tert-butylylene, n-pentylylene, isopentylylene, neopentylylene, and n-hexylylene; "cycloalkylenes of 5 to 10 carbon atoms", such as cyclopentylylene, cyclohexylylene, and adamantylylene; "linear or branched alkenylenes of 2 to 6 carbon atoms", such as vinylene, arylene, isopropenylene, and butenylene; "divalent groups of aromatic hydrocarbons" that result from the removal of two hydrogen atoms from aromatic hydrocarbons, such as benzene, biphenyl, terphenyl, and tetrakisphenyl; and "divalent groups of condensed polycyclic aromatics" that result from the removal of two hydrogen atoms from condensed polycyclic aromatics, such as naphthalene, anthracene, acenaphthalene, fluorene, phenanthrene, indane, pyrene, and triphenylene.

These divalent groups may have a substituent. Examples of the substituent of the "linear or branched alkylene of 1 to 6 carbon atoms", the "cycloalkylene of 5 to 10 carbon atoms", or the "linear or branched alkenylene of 2 to 6 carbon atoms" include the same groups exemplified as the "substituent" in the "linear or branched alkyl of 1 to 6 carbon atoms that has a substituent", the "cycloalkyl of 5 to 10 carbon atoms that has a substituent", or the "linear or branched alkenyl of 2 to 6 carbon atoms that has a substituent" represented by R₁ to R₆ in the general formula (3), and examples of the substituent in the "divalent group of aromatic hydrocarbons" or the "divalent group of condensed polycyclic aromatics" include the same groups exemplified as the "substituent" in the "substituted aromatic hydrocarbon group", the "substituted aromatic heterocyclic group", or the "substituted condensed polycyclic aromatic group" represented by Ar₁ to Ar₄ in the general formula (1).

Examples of the "linear or branched alkyl of 1 to 6 carbon atoms", the "cycloalkyl of 5 to 10 carbon atoms", or the "linear or branched alkenyl of 2 to 6 carbon atoms" in the "linear or branched alkyl of 1 to 6 carbon atoms that may have a substituent", the "cycloalkyl of 5 to 10 carbon atoms that may have a substituent", or the "linear or branched alkenyl of 2 to 6 carbon atoms that may have a substituent" represented by R₇ to R₁₈ in the general formula (4) include the same groups exemplified as the groups for the "linear or branched alkyl of 1 to 6 carbon atoms", the "cycloalkyl of 5 to 10 carbon atoms", or the "linear or branched alkenyl of 2 to 6 carbon atoms" in the "linear or branched alkyl of 1 to 6 carbon atoms that may have a substituent", the "cycloalkyl of 5 to 10 carbon atoms that may have a substituent", or the "linear or branched alkenyl of 2 to 6 carbon atoms that may have a substituent" represented by R₁ to R₆ in the general formula (3), and possible embodiments may also be the same embodiments as the exemplified embodiments.

Examples of the "linear or branched alkyloxy of 1 to 6 carbon atoms" or the "cycloalkyloxy of 5 to 10 carbon atoms" in the "linear or branched alkyloxy of 1 to 6 carbon atoms that may have a substituent" or the "cycloalkyloxy of 5 to 10 carbon atoms that may have a substituent" represented by R₇ to R₁₈ in the general formula (4) include the same groups exemplified as the groups for the "linear or branched alkyloxy of 1 to 6 carbon atoms" or the "cycloalkyloxy of 5 to 10 carbon atoms" in the "linear or branched alkyloxy of 1 to 6 carbon atoms that may have a substituent" or the "cycloalkyloxy of 5 to 10 carbon atoms that may have a substituent" represented by R₁ to R₆ in the general formula (3), and possible embodiments may also be the same embodiments as the exemplified embodiments.

Examples of the "aromatic hydrocarbon group", the "aromatic heterocyclic group", or the "condensed polycyclic aromatic group" in the "substituted or unsubstituted aromatic hydrocarbon group", the "substituted or unsubstituted aromatic heterocyclic group", or the "substituted or unsubstituted condensed polycyclic aromatic group" represented by R₇ to R₁₈ in the general formula (4) include the same groups exemplified as the groups for the "aromatic hydrocarbon group", the "aromatic heterocyclic group", or the "condensed polycyclic aromatic group" in the "substituted or unsubstituted aromatic hydrocarbon group", the "substituted or unsubstituted aromatic heterocyclic group", or the "substituted or unsubstituted condensed polycyclic aromatic group" represented by Ar₁ to Ar₄ in the general formula (1). These groups may bind to each other via a single bond, substituted or unsubstituted methylene, an oxygen atom, or a sulfur atom to form a ring.

These groups may have a substituent, and examples of the substituent include the same substituents exemplified as the "substituent" in the "substituted aromatic hydrocarbon group", the "substituted aromatic heterocyclic group", or the "substituted condensed polycyclic aromatic group" represented by Ar₁ to Ar₄ in the general formula (1), and possible embodiments may also be the same embodiments as the exemplified embodiments.

Examples of the "aryloxy" in the "substituted or unsubstituted aryloxy" represented by R₇ to R₁₈ in the general formula (4) include the same groups exemplified as the groups for the "aryloxy" in the "substituted or unsubstituted aryloxy" represented by R₁ to R₆ in the general formula (3), and possible embodiments may also be the same embodiments as the exemplified embodiments.

In the general formula (4), r₇ to r₁₈ may be the same or different, r₇, r₈, r₁₁, r₁₄, r₁₇, and r₁₈ representing an integer of 0 to 5, and r₉, r₁₀, r₁₂, r₁₃, r₁₅ and r₁₆ representing an integer of 0 to 4. When r₇, r₈, r₁₁, r₁₄. r₁₇, and r₁₈ is an integer of 2 to 5, or r₉, r₁₀, r₁₂, r₁₃, r₁₅ and r₁₆ is an integer of 2 to 4, R₇ to R₁₈, a plurality of which bind to the same benzene ring, may be the same or different and may bind to each other via a single bond, substituted or unsubstituted methylene, an oxygen atom, or a sulfur atom to form a ring.

Examples of the "divalent linking group" represented by L₂, L₃, and L₄ in the general formula (4) include the same groups exemplified as the groups for the "divalent linking group" represented by L₁ in the general formula (3), and possible embodiments may also be the same embodiments as the exemplified embodiments.

Specific examples of the "aromatic hydrocarbon", the "aromatic heterocyclic ring", or the "condensed polycyclic aromatics" of the "substituted or unsubstituted aromatic hydrocarbon", the "substituted or unsubstituted aromatic heterocyclic ring", or the "substituted or unsubstituted condensed polycyclic aromatics" in the "divalent group of a substituted or unsubstituted aromatic hydrocarbon", the "divalent group of a substituted or unsubstituted aromatic heterocyclic ring", or the "divalent group of substituted or unsubstituted condensed polycyclic aromatics" represented by A₁ in the general formula (5) include benzene, biphenyl, terphenyl, tetrakisphenyl, styrene, naphthalene, anthracene, acenaphthalene, fluorene, phenanthrene, indane, pyrene, triphenylene, pyridine, pyrimidine, triazine, pyrrole, furan, thiophene, quinoline, isoquinoline, benzofuran, benzothiophene, indoline, carbazole, carboline, benzoxazole, benzothiazole, quinoxaline, benzimidazole, pyrazole, dibenzofuran, dibenzothiophene, naphthyridine, phenanthroline, and acridine.
The "divalent group of a substituted or unsubstituted aromatic hydrocarbon", the "divalent group of a substituted or unsubstituted aromatic heterocyclic ring", or the "divalent group of substituted or unsubstituted condensed polycyclic aromatics" represented by A₁ in the general formula (5) is a divalent group that results from the removal of two hydrogen atoms from the above "aromatic hydrocarbon", "aromatic heterocyclic ring", or "condensed polycyclic aromatics".

These divalent groups may have a substituent, and examples of the substituent include the same substituents exemplified as the "substituent" in the "substituted aromatic hydrocarbon group", the "substituted aromatic heterocyclic group", or the "substituted condensed polycyclic aromatic group" represented by Ar₁ to Ar₄ in the general formula (1), and possible embodiments may also be the same embodiments as the exemplified embodiments.

Specific examples of the "aromatic heterocyclic group" in the "substituted or unsubstituted aromatic heterocyclic group" represented by B in the general formula (5) include triazinyl, pyridyl, pyrimidinyl, furyl, pyrrolyl, thienyl, quinolyl, isoquinolyl, benzofuranyl, benzothienyl, indolyl, carbazolyl, benzoxazolyl, benzothiazolyl, quinoxalinyl, benzoimidazolyl, pyrazolyl, dibenzofuranyl, dibenzothienyl, naphthyridinyl, phenanthrolinyl, acridinyl, and carbolinyl.

Specific examples of the "substituent" in the "substituted aromatic heterocyclic group" represented by B in the general formula (5) include a deuterium atom; cyano; nitro; halogen atoms such as a fluorine atom, a chlorine atom, a bromine atom, and an iodine atom; linear or branched alkyls of 1 to 6 carbon atoms such as methyl, ethyl, n-propyl, isopropyl, n-butyl, isobutyl, tert-butyl, n-pentyl, isopentyl, neopentyl, and n-hexyl; cycloalkyls of 5 to 10 carbon atoms such as cyclopentyl, cyclohexyl, 1-adamantyl, and 2-adamantyl; linear or branched alkyloxys of 1 to 6 carbon atoms such as methyloxy, ethyloxy, and propyloxy; cycloalkyloxys of 5 to 10 carbon atoms such as cyclopentyloxy, cyclohexyloxy, 1-adamantyloxy, and 2-adamantyloxy; alkenyls such as allyl; aryloxys such as phenyloxy and tolyloxy; arylalkyloxys such as benzyloxy and phenethyloxy; aromatic hydrocarbon groups or condensed polycyclic aromatic groups such as phenyl, biphenylyl, terphenylyl, naphthyl, anthracenyl, phenanthrenyl, fluorenyl, indenyl, pyrenyl, perylenyl, fluoranthenyl, and triphenylenyl; aromatic heterocyclic groups such as pyridyl, pyrimidinyl, triazinyl, thienyl, furyl, pyrrolyl, quinolyl, isoquinolyl, benzofuranyl, benzothienyl, indolyl, carbazolyl, benzoxazolyl, benzothiazolyl, quinoxalinyl, benzoimidazolyl, pyrazolyl, dibenzofuranyl, dibenzothienyl, and carbolinyl; aryloxys such as phenyloxy, biphenylyloxy, naphthyloxy, anthracenyloxy, and phenanthrenyloxy; arylvinyls such as styryl and naphthylvinyl; and acyls such as acetyl and benzoyl. These substituents may be further substituted with the exemplified substituents above. These substituents may bind to each other via a single bond, substituted or unsubstituted methylene, an oxygen atom, or a sulfur atom to form a ring.

Examples of the "aromatic hydrocarbon group", the "aromatic heterocyclic group", or the "condensed polycyclic aromatic group" in the "substituted or unsubstituted aromatic hydrocarbon group", the "substituted or unsubstituted aromatic heterocyclic group", or the "substituted or unsubstituted condensed polycyclic aromatic group" represented by C in the general formula (5) include the same groups exemplified as the groups for the "aromatic hydrocarbon group", the "aromatic heterocyclic group", or the "condensed polycyclic aromatic group" in the "substituted or unsubstituted aromatic hydrocarbon group", the "substituted or unsubstituted aromatic heterocyclic group", or the "substituted or unsubstituted condensed polycyclic aromatic group" represented by Ar₁ to Ar₄ in the general formula (1). When a plurality of these groups binds to the same anthracene ring (when q is 2), these groups may be the same or different.

These groups may have a substituent, and examples of the substituent include the same substituents exemplified as the "substituent" in the "substituted aromatic hydrocarbon group", the "substituted aromatic heterocyclic group", or the "substituted condensed polycyclic aromatic group" represented by Ar₁ to Ar₄ in the general formula (1), and possible embodiments may also be the same embodiments as the exemplified embodiments.

Specific examples of the "linear or branched alkyl of 1 to 6 carbon atoms" represented by D in the general formula (5) include methyl, ethyl, n-propyl, isopropyl, n-butyl, isobutyl, tert-butyl, n-pentyl, isopentyl, neopentyl, and n-hexyl.

The plural groups represented by D may be the same or different, and may bind to each other via a single bond, substituted or unsubstituted methylene, an oxygen atom, or a sulfur atom to form a ring.

Examples of the "aromatic hydrocarbon group", the "aromatic heterocyclic group", or the "condensed polycyclic aromatic group" in the "substituted or unsubstituted aromatic hydrocarbon group", the "substituted or unsubstituted aromatic heterocyclic group", or the "substituted or unsubstituted condensed polycyclic aromatic group" represented by D in the general formula (5) include the same groups exemplified as the groups for the "aromatic hydrocarbon group", the "aromatic heterocyclic group", or the "condensed polycyclic aromatic group" in the "substituted or unsubstituted aromatic hydrocarbon group", the "substituted or unsubstituted aromatic heterocyclic group", or the "substituted or unsubstituted condensed polycyclic aromatic group" represented by Ar₁ to Ar₄ in the general formula (1). The plural groups represented by D may be the same or different, and may bind to each other via a single bond, substituted or unsubstituted methylene, an oxygen atom, or a sulfur atom to form a ring.

These groups may have a substituent, and examples of the substituent include the same substituents exemplified as the "substituent" in the "substituted aromatic hydrocarbon group", the "substituted aromatic heterocyclic group", or the "substituted condensed polycyclic aromatic group" represented by Ar₁ to Ar₄ in the general formula (1), and possible embodiments may also be the same embodiments as the exemplified embodiments.

Ar₁ to Ar₄ in the general formula (1) are preferably the "substituted or unsubstituted aromatic hydrocarbon group", the "substituted or unsubstituted oxygen-containing aromatic heterocyclic group", or the "substituted or unsubstituted condensed polycyclic aromatic group", and more preferably the "substituted or unsubstituted aromatic hydrocarbon group" or the "substituted or unsubstituted condensed polycyclic aromatic group". Specifically, for Ar₁ and Ar₂, phenyl, biphenylyl, terphenylyl, phenanthrenyl, fluorenyl, triphenylenyl, and dibenzofuranyl are preferred, and phenyl, biphenylyl, terphenylyl, phenanthrenyl, fluorenyl, and triphenylenyl are more preferred.

Specifically, for Ar₃, phenyl, terphenylyl, naphthyl, phenanthrenyl, fluorenyl, triphenylenyl, and dibenzofuranyl are preferred, and phenyl, terphenylyl, naphthyl, phenanthrenyl, fluorenyl, and triphenylenyl are more preferred. Specifically, for Ar₄, phenyl, biphenylyl, terphenylyl, naphthyl, phenanthrenyl, fluorenyl, triphenylenyl, and dibenzofuranyl are preferred, and phenyl, biphenylyl, terphenylyl, naphthyl, phenanthrenyl, fluorenyl, and triphenylenyl are more preferred.

The "substituent" in the "substituted aromatic hydrocarbon group", the "substituted aromatic heterocyclic group", or the "substituted condensed polycyclic aromatic group" represented by Ar₁ to Ar₄ is preferably a deuterium atom, the "linear or branched alkyl of 1 to 6 carbon atoms that may have a substituent", the "linear or branched alkenyl of 2 to 6 carbon atoms that may have a substituent", the "substituted or unsubstituted aromatic hydrocarbon group", the "substituted or unsubstituted aromatic heterocyclic group", the "substituted or unsubstituted condensed polycyclic aromatic group", or the "substituted or unsubstituted aryloxy group", and specifically is more preferably a deuterium atom, phenyl, biphenylyl, naphthyl, vinyl, methyl, indolyl, dibenzofuranyl, or phenyloxy. Embodiments where these groups bind to each other via a single bond to form a condensed aromatic ring are also preferred.

Examples of the electron acceptor, with which the arylamine compound represented by the general formula (1) is doped, in the hole injection layer of the organic EL device of the present invention include trisbromophenylamine hexachloroantimony, tetracyanoquinodimethane (TCNQ), 2,3,5,6-tetrafluoro-tetracyano-1,4-benzoquinodimethane (F4TCNQ), and a radialene derivative (see, for example, JP-A-2011-100621), and the radialene derivative of the general formula (2) is preferably used.

Ar₅ to Ar₇ in the general formula (2) are preferably the "aromatic hydrocarbon group", the "condensed polycyclic aromatic group", or pyridyl, and further preferably phenyl, biphenylyl, terphenylyl, naphthyl, phenanthryl, fluorenyl, or pyridyl, and the "electron acceptor group" therein is preferably a fluorine atom, a chlorine atom, cyano, or trifluoromethyl.

An embodiment is preferable that Ar₅ to Ar₇ in the general formula (2) are at least partially, preferably completely, substituted by the "electron acceptor group".

Ar₅ to Ar₇ in the general formula (2) are preferably phenyl that is completely substituted by a fluorine atom, a chlorine atom, cyano, or trifluoromethyl, such as tetrafluoropyridyl, tetrafluoro(trifluoromethyl)phenyl, cyanotetrafluorophenyl, dichlorodifluoro(trifluoromethyl)phenyl, or pentafluorophenyl, or pyridyl.

R₁ to R₆ in the general formula (3) are preferably a deuterium atom, the "linear or branched alkyl of 1 to 6 carbon atoms that may have a substituent", the "linear or branched alkenyl of 2 to 6 carbon atoms that may have a substituent", the "substituted or unsubstituted aromatic hydrocarbon group", or the "substituted or unsubstituted condensed polycyclic aromatic group", and further preferably, a deuterium atom, phenyl, biphenylyl, naphthyl, or vinyl. It is also preferable that these groups bind to each other via a single bond to form a condensed aromatic ring. A deuterium atom, phenyl, and biphenylyl are particularly preferable.

r₁ to r₆ in the general formula (3) are preferably an integer of 0 to 3, and further preferably an integer of 0 to 2.

The "divalent linking group" represented by L₁ in the general formula (3) is preferably methylene, the "cycloalkyl of 5 to 10 carbon atoms", the "divalent group of an aromatic hydrocarbon", or the "divalent group of condensed polycyclic aromatics", or a single bond, further preferably divalent groups represented by the following structural formulae (A) to (F), or a single bond, and particularly preferably a divalent group represented by the following structural formula (A).

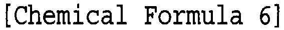

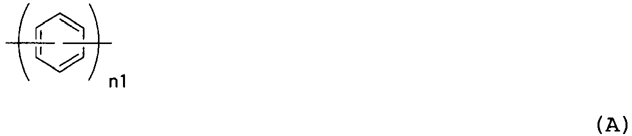

In the formula, n1 represents an integer of 1 to 3.

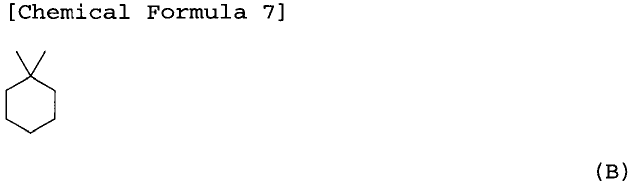

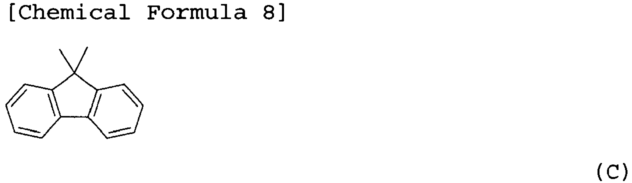

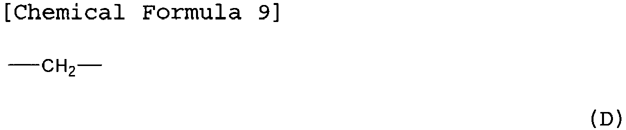

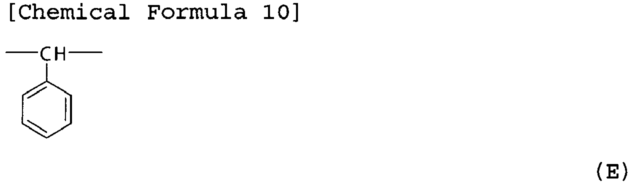

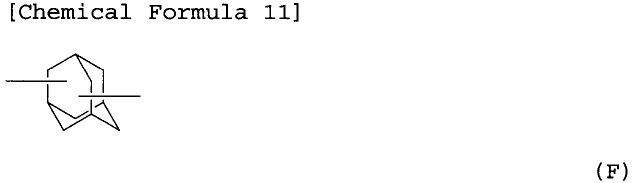

R₇ to R₁₈ in the general formula (4) are preferably a deuterium atom, the "linear or branched alkyl of 1 to 6 carbon atoms that may have a substituent", the "linear or branched alkenyl of 2 to 6 carbon atoms that may have a substituent", the "substituted or unsubstituted aromatic hydrocarbon group", or the "substituted or unsubstituted condensed polycyclic aromatic group", and further preferably, a deuterium atom, phenyl, biphenylyl, naphthyl, or vinyl. It is also preferable that these groups bind to each other via a single bond to form a condensed aromatic ring. A deuterium atom, phenyl, and biphenylyl are particularly preferable.

r₇ to r₁₈ in the general formula (4) are preferably an integer of 0 to 3, and further preferably an integer of 0 to 2.

The "divalent linking groups" represented by L₂ to L₄ in the general formula (4) are preferably methylene, the "cycloalkyl of 5 to 10 carbon atoms", the "divalent group of an aromatic hydrocarbon", or the "divalent group of condensed polycyclic aromatics", or a single bond, and further preferably divalent groups represented by the structural formulae (A) to (F), or a single bond.

The "aromatic heterocyclic group" in the "substituted or unsubstituted aromatic heterocyclic group" represented by B in the general formula (5) is preferably a nitrogen-containing aromatic heterocyclic group, such as pyridyl, pyrimidinyl, pyrrolyl, quinolyl, isoquinolyl, indolyl, carbazolyl, benzoxazolyl, benzothiazolyl, quinoxalinyl, benzoimidazolyl, pyrazolyl, or carbolinyl, and further preferably pyridyl, pyrimidinyl, quinolyl, isoquinolyl, indolyl, pyrazolyl, benzoimidazolyl, or carbolinyl.

For p and q in the general formula (5), p represents 7 or 8, and q represents 1 or 2, while maintaining the relationship, in which the sum of p and q (p+q) is 9.

A₁ in the general formula (5) is preferably the "divalent group of a substituted or unsubstituted aromatic hydrocarbon" or the "divalent group of substituted or unsubstituted condensed polycyclic aromatics", and further preferably divalent groups that result from the removal of two hydrogen atoms from benzene, biphenyl, naphthalene, or phenanthrene.

The compound having an anthracene ring structure of the general formula (5) is preferably a compound having an anthracene ring structure of the following general formula (5a), the following general formula (5b), or the following general formula (5c).

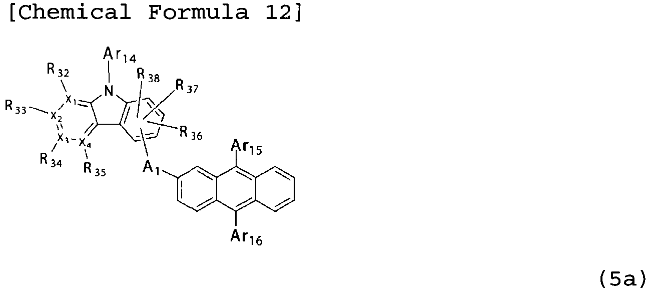

In the formula, A₁ represents a divalent group of a substituted or unsubstituted aromatic hydrocarbon, a divalent group of a substituted or unsubstituted aromatic heterocyclic ring, a divalent group of substituted or unsubstituted condensed polycyclic aromatics, or a single bond. Ar₁₄, Ar₁₅, and Ar₁₆ may be the same or different, and represent a substituted or unsubstituted aromatic hydrocarbon group, a substituted or unsubstituted aromatic heterocyclic group, or a substituted or unsubstituted condensed polycyclic aromatic group. R₃₂ to R₃₈ may be the same or different, and represent a hydrogen atom, a deuterium atom, a fluorine atom, a chlorine atom, cyano, nitro, linear or branched alkyl of 1 to 6 carbon atoms that may have a substituent, cycloalkyl of 5 to 10 carbon atoms that may have a substituent, linear or branched alkenyl of 2 to 6 carbon atoms that may have a substituent, linear or branched alkyloxy of 1 to 6 carbon atoms that may have a substituent, cycloalkyloxy of 5 to 10 carbon atoms that may have a substituent, a substituted or unsubstituted aromatic hydrocarbon group, a substituted or unsubstituted aromatic heterocyclic group, a substituted or unsubstituted condensed polycyclic aromatic group, or substituted or unsubstituted aryloxy, where these groups may bind to each other via a single bond, substituted or unsubstituted methylene, an oxygen atom, or a sulfur atom to form a ring. X₁, X₂, X₃, and X₄ represent a carbon atom or a nitrogen atom, and only one of X₁, X₂, X₃, and X₄ is a nitrogen atom. In this case, the nitrogen atom does not have the hydrogen atom or substituent for R₃₂ to R₃₅.

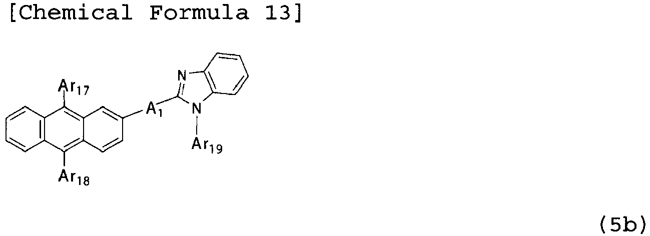

In the formula, A₁ represents a divalent group of a substituted or unsubstituted aromatic hydrocarbon, a divalent group of a substituted or unsubstituted aromatic heterocyclic ring, a divalent group of substituted or unsubstituted condensed polycyclic aromatics, or a single bond. Ar₁₇, Ar₁₈, and Ar₁₉ may be the same or different, and represent a substituted or unsubstituted aromatic hydrocarbon group, a substituted or unsubstituted aromatic heterocyclic group, or a substituted or unsubstituted condensed polycyclic aromatic group.

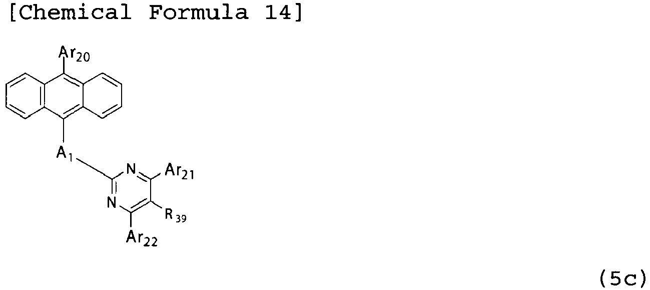

In the formula, A₁ represents a divalent group of a substituted or unsubstituted aromatic hydrocarbon, a divalent group of a substituted or unsubstituted aromatic heterocyclic ring, a divalent group of substituted or unsubstituted condensed polycyclic aromatics, or a single bond. Ar₂₀, Ar₂₁, and Ar₂₂ may be the same or different, and represent a substituted or unsubstituted aromatic hydrocarbon group, a substituted or unsubstituted aromatic heterocyclic group, or a substituted or unsubstituted condensed polycyclic aromatic group. R₃₉ represents a hydrogen atom, a deuterium atom, a fluorine atom, a chlorine atom, cyano, nitro, linear or branched alkyl of 1 to 6 carbon atoms that may have a substituent, cycloalkyl of 5 to 10 carbon atoms that may have a substituent, linear or branched alkenyl of 2 to 6 carbon atoms that may have a substituent, linear or branched alkyloxy of 1 to 6 carbon atoms that may have a substituent, cycloalkyloxy of 5 to 10 carbon atoms that may have a substituent, a substituted or unsubstituted aromatic hydrocarbon group, a substituted or unsubstituted aromatic heterocyclic group, a substituted or unsubstituted condensed polycyclic aromatic group, or substituted or unsubstituted aryloxy.

Examples of the "aromatic hydrocarbon group", the "aromatic heterocyclic group", or the "condensed polycyclic aromatic group" in the "substituted or unsubstituted aromatic hydrocarbon group", the "substituted or unsubstituted aromatic heterocyclic group", or the "substituted or unsubstituted condensed polycyclic aromatic group" represented by Ar₁₄, Ar₁₅, and Ar₁₆ in the general formula (5a) include the same groups exemplified as the groups for the "aromatic hydrocarbon group", the "aromatic heterocyclic group", or the "condensed polycyclic aromatic group" in the "substituted or unsubstituted aromatic hydrocarbon group", the "substituted or unsubstituted aromatic heterocyclic group", or the "substituted or unsubstituted condensed polycyclic aromatic group" represented by Ar₁ to Ar₄ in the general formula (1).

These groups may have a substituent. Examples of the substituent include the same groups exemplified as the "substituent" in the "substituted aromatic hydrocarbon group", the "substituted aromatic heterocyclic group", or the "substituted condensed polycyclic aromatic group" represented by Ar₁ to Ar₄ in the general formula (1), and possible embodiments may also be the same embodiments as the exemplified embodiments.

Specific examples of the "linear or branched alkyl of 1 to 6 carbon atoms", the "cycloalkyl of 5 to 10 carbon atoms", or the "linear or branched alkenyl of 2 to 6 carbon atoms" in the "linear or branched alkyl of 1 to 6 carbon atoms that may have a substituent", the "cycloalkyl of 5 to 10 carbon atoms that may have a substituent", or the "linear or branched alkenyl of 2 to 6 carbon atoms that may have a substituent" represented by R₃₂ to R₃₈ in the general formula (5a) include methyl, ethyl, n-propyl, isopropyl, n-butyl, isobutyl, tert-butyl, n-pentyl, isopentyl, neopentyl, n-hexyl, cyclopentyl, cyclohexyl, 1-adamantyl, 2-adamantyl, vinyl, allyl, isopropenyl, and 2-butenyl. These groups may bind to each other via a single bond, substituted or unsubstituted methylene, an oxygen atom, or a sulfur atom to form a ring.

Specific examples of the "substituent" in the "linear or branched alkyl of 1 to 6 carbon atoms that has a substituent", the "cycloalkyl of 5 to 10 carbon atoms that has a substituent", or the "linear or branched alkenyl of 2 to 6 carbon atoms that has a substituent" represented by R₃₂ to R₃₈ in the general formula (5a) include a deuterium atom; cyano; nitro; halogen atoms such as a fluorine atom, a chlorine atom, a bromine atom, and an iodine atom; linear or branched alkyloxys of 1 to 6 carbon atoms such as methyloxy, ethyloxy, and propyloxy; alkenyls such as allyl; aryloxys such as phenyloxy and tolyloxy; arylalkyloxys such as benzyloxy and phenethyloxy; aromatic hydrocarbon groups or condensed polycyclic aromatic groups such as phenyl, biphenylyl, terphenylyl, naphthyl, anthracenyl, phenanthrenyl, fluorenyl, indenyl, pyrenyl, perylenyl, fluoranthenyl, and triphenylenyl; and aromatic heterocyclic groups such as pyridyl, pyrimidinyl, triazinyl, thienyl, furyl, pyrrolyl, quinolyl, isoquinolyl, benzofuranyl, benzothienyl, indolyl, carbazolyl, benzoxazolyl, benzothiazolyl, quinoxalinyl, benzoimidazolyl, pyrazolyl, dibenzofuranyl, dibenzothienyl, and carbolinyl. These substituents may be further substituted with the exemplified substituents above. These substituents may bind to each other via a single bond, substituted or unsubstituted methylene, an oxygen atom, or a sulfur atom to form a ring.

Specific examples of the "linear or branched alkyloxy of 1 to 6 carbon atoms" or the "cycloalkyloxy of 5 to 10 carbon atoms" in the "linear or branched alkyloxy of 1 to 6 carbon atoms that may have a substituent" or the "cycloalkyloxy of 5 to 10 carbon atoms that may have a substituent" represented by R₃₂ to R₃₈ in the general formula (5a) include methyloxy, ethyloxy, n-propyloxy, isopropyloxy, n-butyloxy, tert-butyloxy, n-pentyloxy, n-hexyloxy, cyclopentyloxy, cyclohexyloxy, cycloheptyloxy, cyclooctyloxy, 1-adamantyloxy, and 2-adamantyloxy. These groups may bind to each other via a single bond, substituted or unsubstituted methylene, an oxygen atom, or a sulfur atom to form a ring.

These groups may have a substituent. Examples of the substituent include the same groups exemplified as the "substituent" in the "linear or branched alkyl of 1 to 6 carbon atoms that has a substituent", the "cycloalkyl of 5 to 10 carbon atoms that has a substituent", or the "linear or branched alkenyl of 2 to 6 carbon atoms that has a substituent" represented by R₃₂ to R₃₈ in the general formula (5a), and possible embodiments may also be the same embodiments as the exemplified embodiments.

Examples of the "aromatic hydrocarbon group", the "aromatic heterocyclic group", or the "condensed polycyclic aromatic group" in the "substituted or unsubstituted aromatic hydrocarbon group", the "substituted or unsubstituted aromatic heterocyclic group", or the "substituted or unsubstituted condensed polycyclic aromatic group" represented by R₃₂ to R₃₈ in the general formula (5a) include the same groups exemplified as the groups for the "aromatic hydrocarbon group", the "aromatic heterocyclic group", or the "condensed polycyclic aromatic group" in the "substituted or unsubstituted aromatic hydrocarbon group", the "substituted or unsubstituted aromatic heterocyclic group", or the "substituted or unsubstituted condensed polycyclic aromatic group" represented by Ar₃ to Ar₄ in the general formula (1). These groups may bind to each other via a single bond, substituted or unsubstituted methylene, an oxygen atom, or a sulfur atom to form a ring.

These groups may have a substituent. Examples of the substituent include the same groups exemplified as the "substituent" in the "substituted aromatic hydrocarbon group", the "substituted aromatic heterocyclic group", or the "substituted condensed polycyclic aromatic group" represented by Ar₁ to Ar₄ in the general formula (1), and possible embodiments may also be the same embodiments as the exemplified embodiments.

Specific examples of the "aryloxy group" in the "substituted or unsubstituted aryloxy group" represented by R₃₂ to R₃₈ in the general formula (5a) include phenyloxy, biphenylyloxy, terphenylyloxy, naphthyloxy, anthracenyloxy, phenanthrenyloxy, fluorenyloxy, indenyloxy, pyrenyloxy, and perylenyloxy. These groups may bind to each other via a single bond, substituted or unsubstituted methylene, an oxygen atom, or a sulfur atom to form a ring.

These groups may have a substituent. Examples of the substituent include the same groups exemplified as the "substituent" in the "substituted aromatic hydrocarbon group", the "substituted aromatic heterocyclic group", or the "substituted condensed polycyclic aromatic group" represented by Ar₁ to Ar₄ in the general formula (1), and possible embodiments may also be the same embodiments as the exemplified embodiments.

In the general formula (5a), X₁, X₂, X₃, and X₄ represent a carbon atom or a nitrogen atom, and only one of X₁, X₂, X₃, and X₄ is a nitrogen atom (and the others are carbon atoms). In this case, the nitrogen atom does not have the hydrogen atom or substituent for R₃₂ to R₃₅. That is, R₃₂ does not exist when X₁ is a nitrogen atom, R₃₃ does not exist when X₂ is a nitrogen atom, R₃₄ does not exist when X₃ is a nitrogen atom, and R₃₅ does not exist when X₄ is a nitrogen atom.

In the general formula (5a), it is preferable that X₃ is a nitrogen atom (and X₁, X₂, and X₄ are carbon atoms), and in this case, a hydrogen atom or substituent for R₃₄ does not exist.

The binding position of the linking group A₁ is preferably the position corresponding to the para-position of the nitrogen atom of the pyridoindole ring.

Examples of the "aromatic hydrocarbon group", the "aromatic heterocyclic group", or the "condensed polycyclic aromatic group" in the "substituted or unsubstituted aromatic hydrocarbon group", the "substituted or unsubstituted aromatic heterocyclic group", or the "substituted or unsubstituted condensed polycyclic aromatic group" represented by Ar₁₇, Ar₁₈, and Ar₁₉ in the general formula (5b) include the same groups exemplified as the groups for the "aromatic hydrocarbon group", the "aromatic heterocyclic group", or the "condensed polycyclic aromatic group" in the "substituted or unsubstituted aromatic hydrocarbon group", the "substituted or unsubstituted aromatic heterocyclic group", or the "substituted or unsubstituted condensed polycyclic aromatic group" represented by Ar₁ to Ar₄ in the general formula (1).

These groups may have a substituent. Examples of the substituent include the same groups exemplified as the "substituent" in the "substituted aromatic hydrocarbon group", the "substituted aromatic heterocyclic group", or the "substituted condensed polycyclic aromatic group" represented by Ar₁ to Ar₄ in the general formula (1), and possible embodiments may also be the same embodiments as the exemplified embodiments.

Examples of the "aromatic hydrocarbon group", the "aromatic heterocyclic group", or the "condensed polycyclic aromatic group" in the "substituted or unsubstituted aromatic hydrocarbon group", the "substituted or unsubstituted aromatic heterocyclic group", or the "substituted or unsubstituted condensed polycyclic aromatic group" represented by Ar₂₀, Ar₂₁, and Ar₂₂ in the general formula (5c) include the same groups exemplified as the groups for the "aromatic hydrocarbon group", the "aromatic heterocyclic group", or the "condensed polycyclic aromatic group" in the "substituted or unsubstituted aromatic hydrocarbon group", the "substituted or unsubstituted aromatic heterocyclic group", or the "substituted or unsubstituted condensed polycyclic aromatic group" represented by Ar₁ to Ar₄ in the general formula (1).

These groups may have a substituent. Examples of the substituent include the same groups exemplified as the "substituent" in the "substituted aromatic hydrocarbon group", the "substituted aromatic heterocyclic group", or the "substituted condensed polycyclic aromatic group" represented by Ar₁ to Ar₄ in the general formula (1), and possible embodiments may also be the same embodiments as the exemplified embodiments.

Examples of the "linear or branched alkyl of 1 to 6 carbon atoms", the "cycloalkyl of 5 to 10 carbon atoms", or the "linear or branched alkenyl of 2 to 6 carbon atoms" in the "linear or branched alkyl of 1 to 6 carbon atoms that may have a substituent", the "cycloalkyl of 5 to 10 carbon atoms that may have a substituent", or the "linear or branched alkenyl of 2 to 6 carbon atoms that may have a substituent" represented by R₃₉ in the general formula (5c) include the same groups exemplified as the groups for the "linear or branched alkyl of 1 to 6 carbon atoms", the "cycloalkyl of 5 to 10 carbon atoms", or the "linear or branched alkenyl of 2 to 6 carbon atoms" in the "linear or branched alkyl of 1 to 6 carbon atoms that may have a substituent", the "cycloalkyl of 5 to 10 carbon atoms that may have a substituent", or the "linear or branched alkenyl of 2 to 6 carbon atoms that may have a substituent" represented by R₃₂ to R₃₈ in the general formula (5a).

These groups may have a substituent. Examples of the substituent include the same groups exemplified as the "substituent" in the "linear or branched alkyl of 1 to 6 carbon atoms that may have a substituent", the "cycloalkyl of 5 to 10 carbon atoms that may have a substituent", or the "linear or branched alkenyl of 2 to 6 carbon atoms that may have a substituent" represented by R₃₂ to R₃₈ in the general formula (5a), and possible embodiments may also be the same embodiments as the exemplified embodiments.

Examples of the "linear or branched alkyloxy of 1 to 6 carbon atoms" or the "cycloalkyloxy of 5 to 10 carbon atoms" in the "linear or branched alkyloxy of 1 to 6 carbon atoms that may have a substituent" or the "cycloalkyloxy of 5 to 10 carbon atoms that may have a substituent" represented by R₃₉ in the general formula (5c) include the same groups exemplified as the "substituent" in the "linear or branched alkyloxy of 1 to 6 carbon atoms" or the "cycloalkyloxy of 5 to 10 carbon atoms" in the "linear or branched alkyloxy of 1 to 6 carbon atoms that may have a substituent" or the "cycloalkyloxy of 5 to 10 carbon atoms that may have a substituent" represented by R₃₂ to R₃₈ in the general formula (5a).

These groups may have a substituent. Examples of the substituent include the same groups exemplified as the "substituent" in the "linear or branched alkyl of 1 to 6 carbon atoms that may have a substituent", the "cycloalkyl of 5 to 10 carbon atoms that may have a substituent", or the "linear or branched alkenyl of 2 to 6 carbon atoms that may have a substituent" represented by R₃₂ to R₃₈ in the general formula (5a), and possible embodiments may also be the same embodiments as the exemplified embodiments.

Examples of the "aromatic hydrocarbon group", the "aromatic heterocyclic group", or the "condensed polycyclic aromatic group" in the "substituted or unsubstituted aromatic hydrocarbon group", the "substituted or unsubstituted aromatic heterocyclic group", or the "substituted or unsubstituted condensed polycyclic aromatic group" represented by R₃₉ in the general formula (5c) include the same groups exemplified as the groups for the "aromatic hydrocarbon group", the "aromatic heterocyclic group", or the "condensed polycyclic aromatic group" in the "substituted or unsubstituted aromatic hydrocarbon group", the "substituted or unsubstituted aromatic heterocyclic group", or the "substituted or unsubstituted condensed polycyclic aromatic group" represented by Ar₁ to Ar₄ in the general formula (1).

These groups may have a substituent. Examples of the substituent include the same groups exemplified as the "substituent" in the "substituted aromatic hydrocarbon group", the "substituted aromatic heterocyclic group", or the "substituted condensed polycyclic aromatic group" represented by Ar₁ to Ar₄ in the general formula (1), and possible embodiments may also be the same embodiments as the exemplified embodiments.

Examples of the "aryloxy group" in the "substituted or unsubstituted aryloxy group" represented by R₃₉ in the general formula (5c) include the same groups exemplified as the groups for the "aryloxy group" in the "substituted or unsubstituted aryloxy group" represented by R₃₂ to R₃₈ in the general formula (5a).

These groups may have a substituent. Examples of the substituent include the same groups exemplified as the "substituent" in the "substituted aromatic hydrocarbon group", the "substituted aromatic heterocyclic group", or the "substituted condensed polycyclic aromatic group" represented by Ar₁ to Ar₄ in the general formula (1), and possible embodiments may also be the same embodiments as the exemplified embodiments.

The arylamine compound of the general formula (1) preferably used in the organic EL device of the present invention can be used as a constitutive material of a hole injection layer or a hole transport layer of an organic EL device. The compound has high hole mobility and is a preferred compound as a material of a hole injection layer or a hole transport layer.

The radialene derivative of the general formula (2) preferably used in the organic EL device of the present invention is a preferred compound as a p-type doping material for a material generally used in a hole injection layer or a hole transport layer of an organic EL device.

The arylamine compound of general formula (3) having two triphenylamine structures in the molecule and the arylamine compound of general formula (4) having four triphenylamine structures in the molecule preferably used in the organic EL device of the present invention are a preferred compound as a constitutive material of a hole injection layer or a hole transport layer of an organic EL device.

The compound of the general formula (5) having an anthracene ring structure preferably used in the organic EL device of the present invention is a preferred compound as a constitutive material of an electron transport layer of an organic EL device.

The organic EL device of the invention uses materials for an organic EL device that are excellent in hole injection and transport capabilities and stability and durability of a thin film, and are combined in consideration of the carrier balance, and thus is enhanced in hole transport efficiency from the anode to the hole transport layer, whereby (and further using the particular arylamine compound (having the particular structure)) the luminous efficiency of the organic EL device can be enhanced while achieving a low driving voltage thereof, and the durability thereof can be enhanced.

Accordingly, an organic EL device that has a low driving voltage, an enhanced luminous efficiency, and a long lifetime can be achieved.

Furthermore, the doping concentration of the electron acceptor and/or the film thickness of the organic layer containing the electron acceptor are controlled, and thereby the low driving voltage can be retained, or the driving voltage rise can be effectively suppressed.

### Advantageous Effects of Invention

In the organic EL device of the invention, the particular arylamine compound (having the particular structure and ionization potential) capable of effectively exhibiting the hole injection and transport function is selected as a material of a hole injection layer and doped with an electron acceptor, the particular arylamine compound (having the particular structure) that is not doped with an electron acceptor is combined as a material of a hole transport layer therewith, and further the doping concentration of the electron acceptor in the hole injection layer and the film thickness of the hole injection layer are controlled, thereby achieving an organic EL device that is enhanced in luminous efficiency at a low driving voltage and improved in durability, and further retains a low driving voltage or is effectively suppressed in the driving voltage rise.

### Brief Description of Drawings

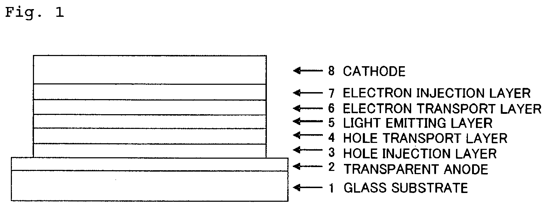

FIG. 1 is a diagram illustrating the configuration of the EL devices of Examples 38 to 47 (Examples 40, 41 and 45-47 not forming part of the claimed invention) and Comparative

### Examples 1 and 2.

### Description of Embodiments

The following presents specific examples of preferred compounds among the arylamine compounds of the general formula (1) preferably used in the organic EL device of the present invention. Compounds 1-2 , 1-4, 1-6, 1-7, 1-20, 1-22, 1-24, 1-29, 1-54, 1-55, 1-57, 1-59, 1-65, 1-66, 1-67, 1-70, 1-76 and 1-90 are not forming part of the claimed invention. The present invention, however, is not restricted to these compounds.

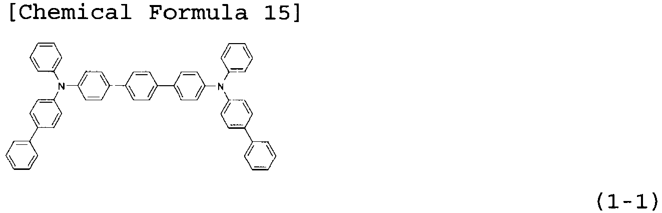

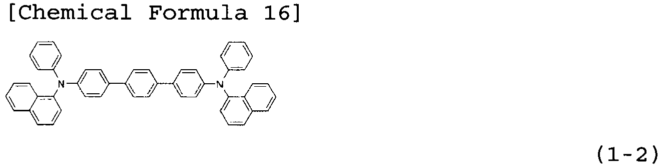

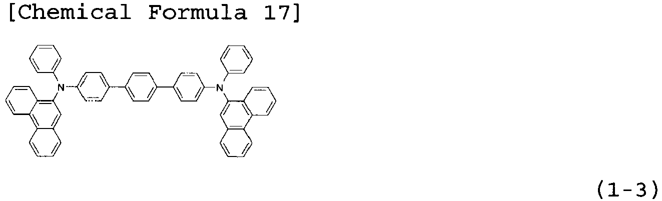

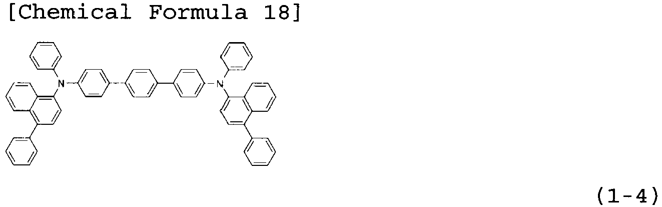

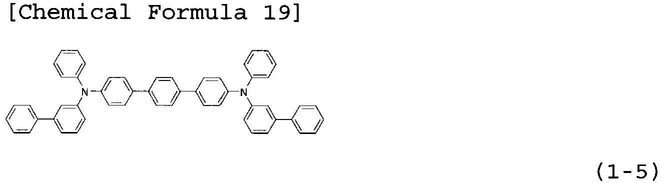

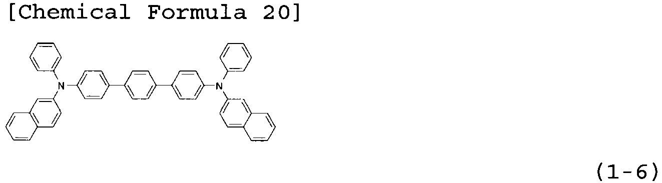

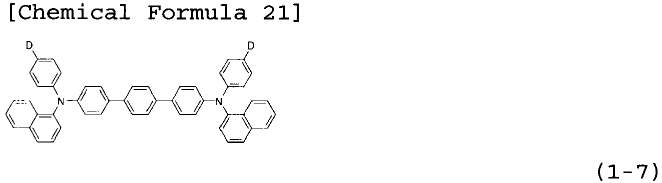

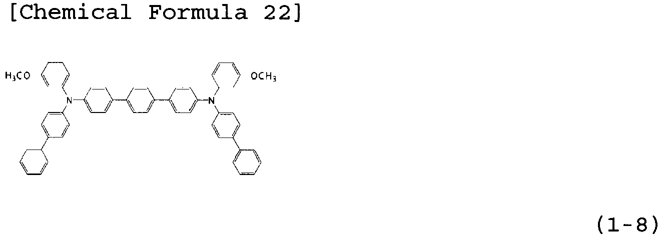

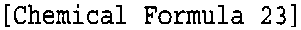

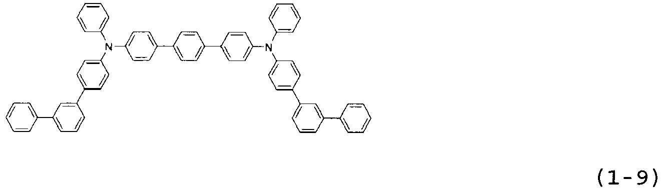

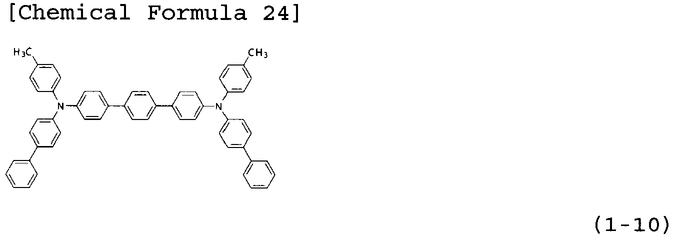

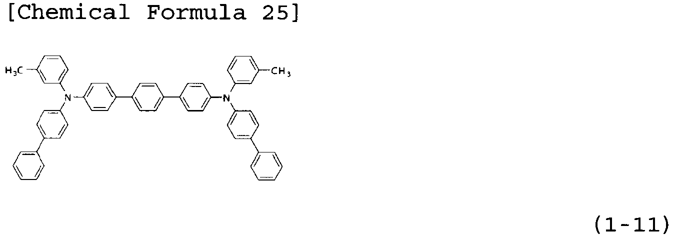

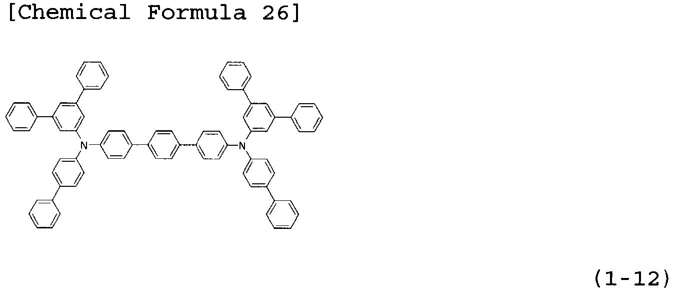

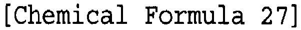

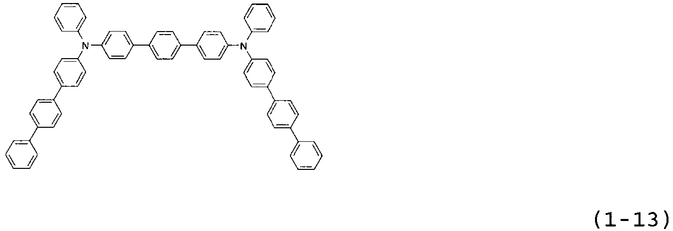

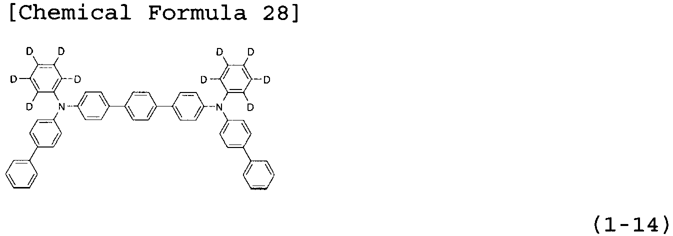

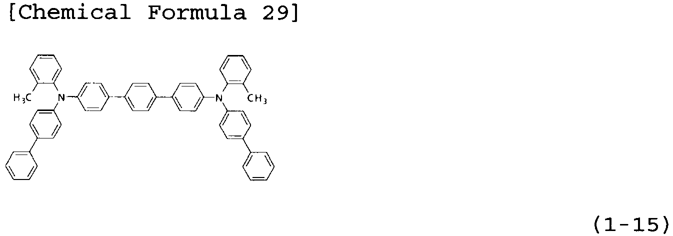

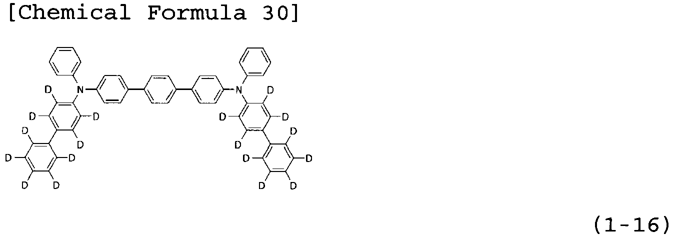

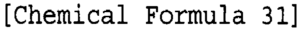

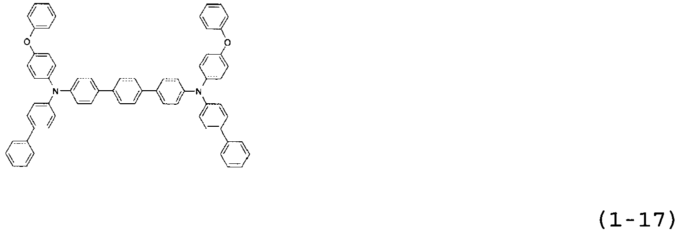

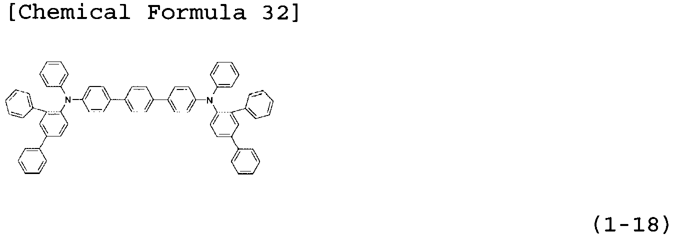

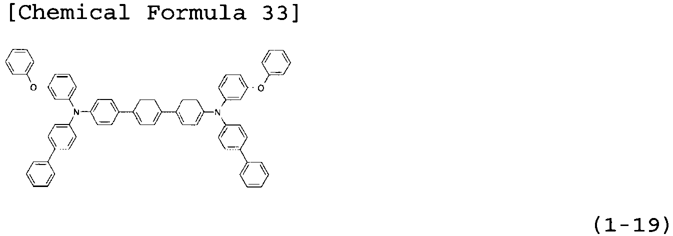

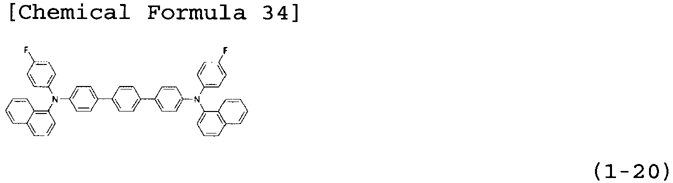

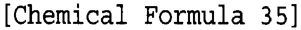

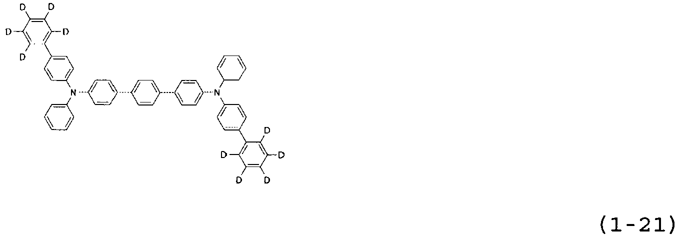

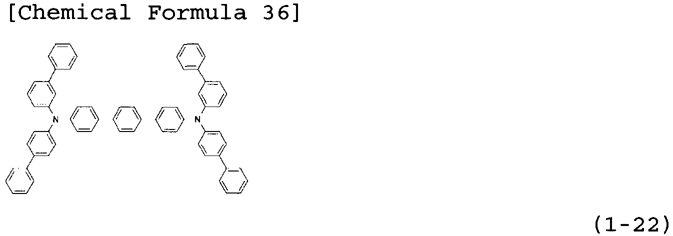

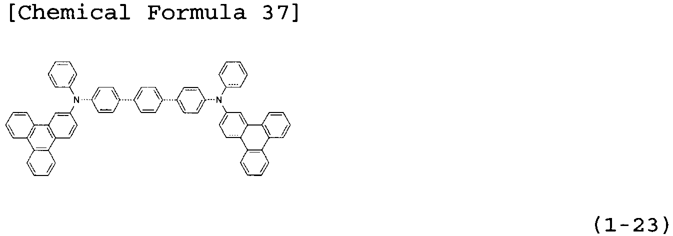

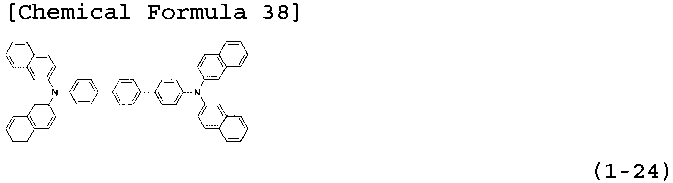

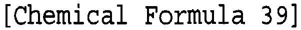

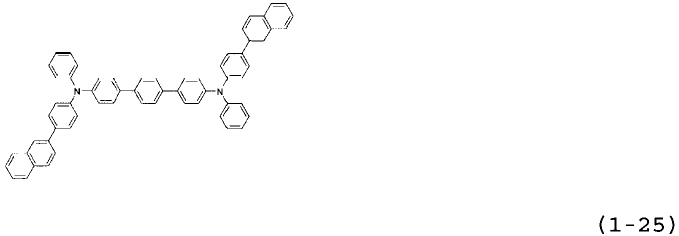

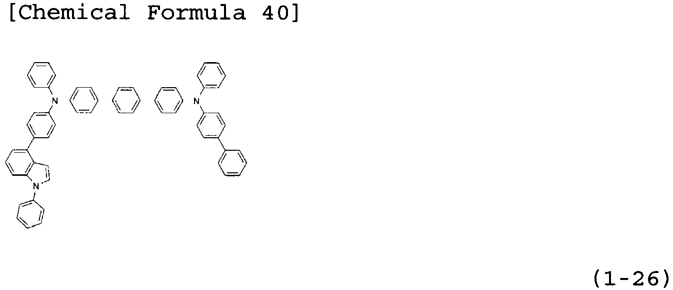

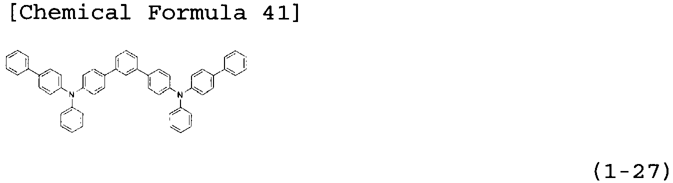

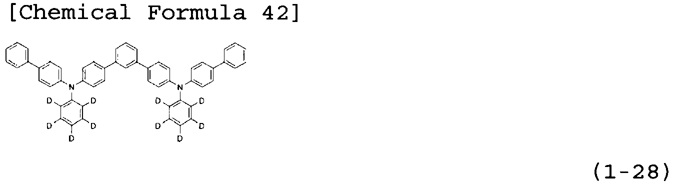

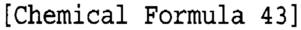

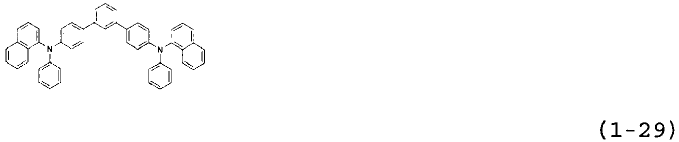

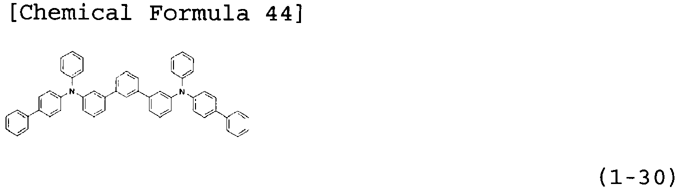

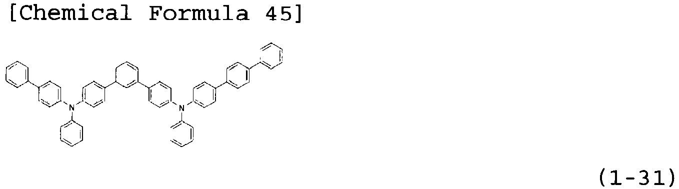

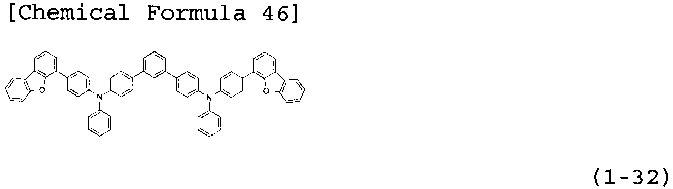

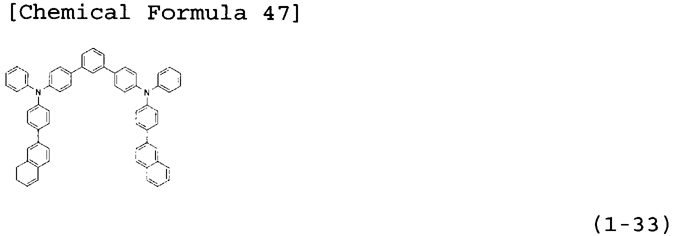

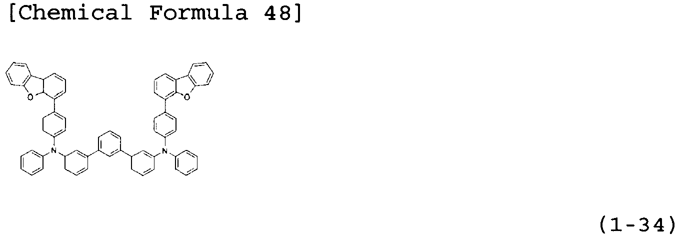

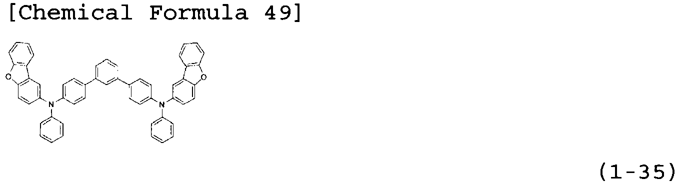

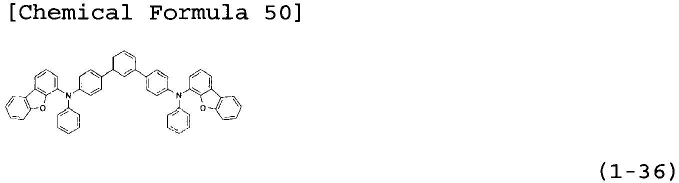

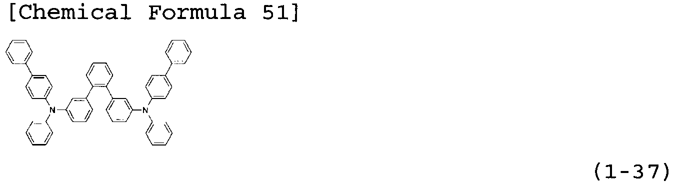

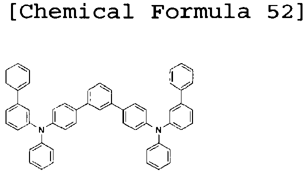

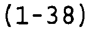

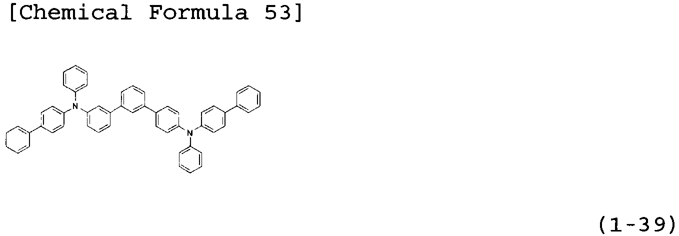

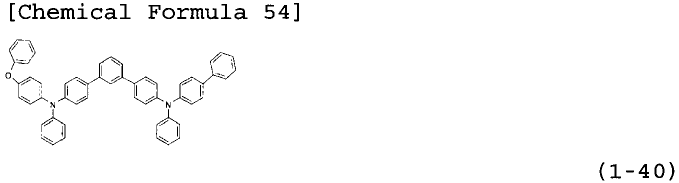

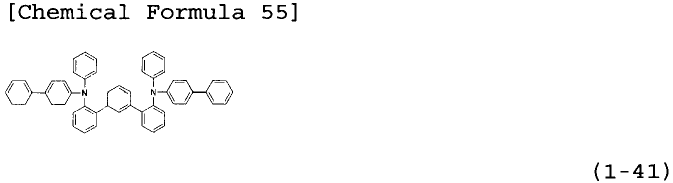

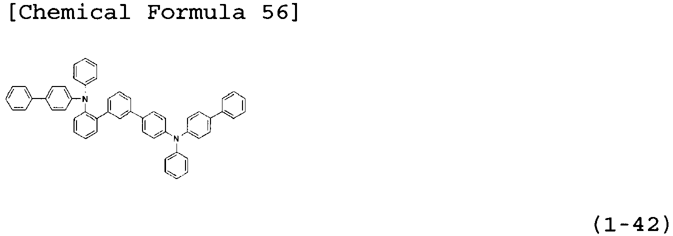

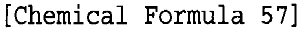

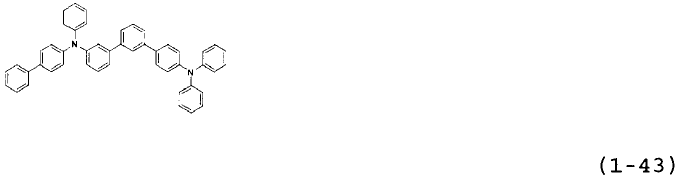

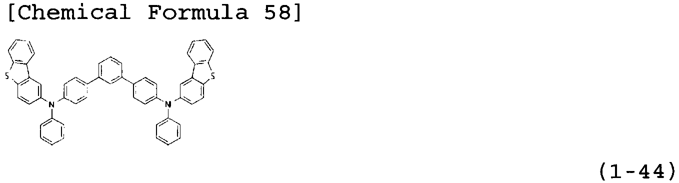

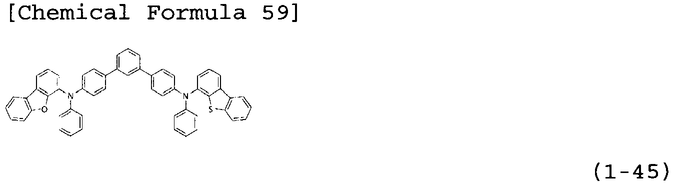

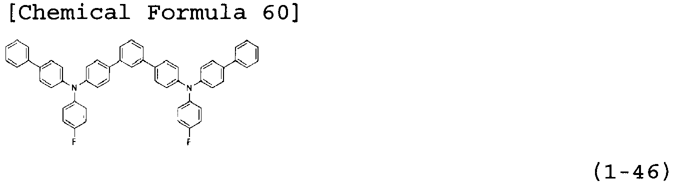

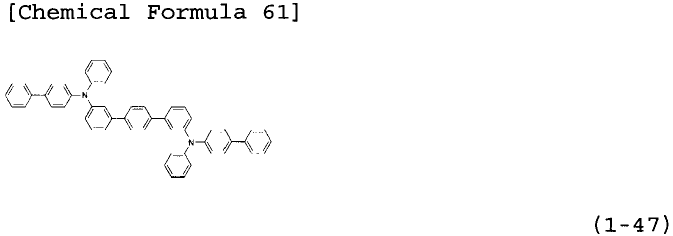

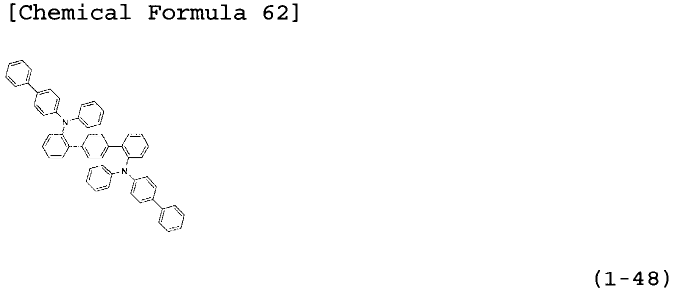

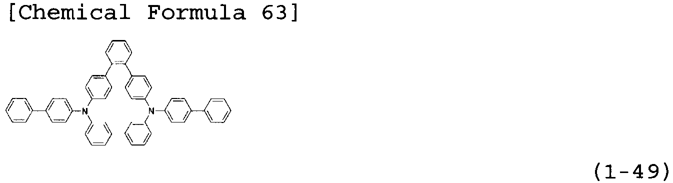

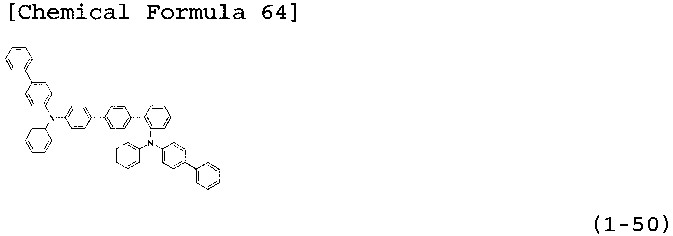

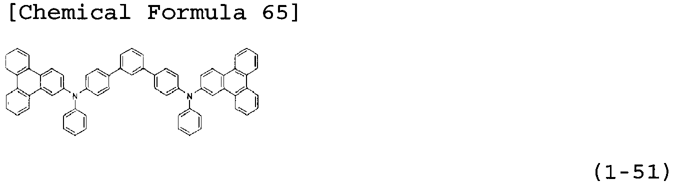

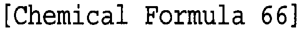

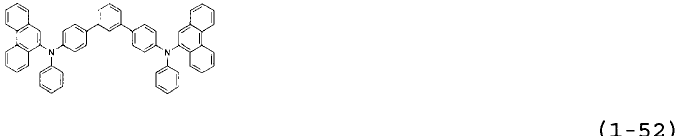

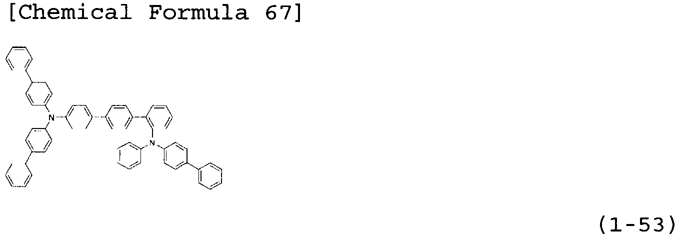

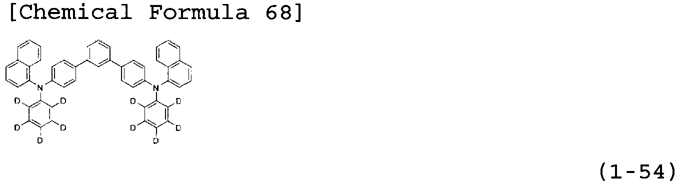

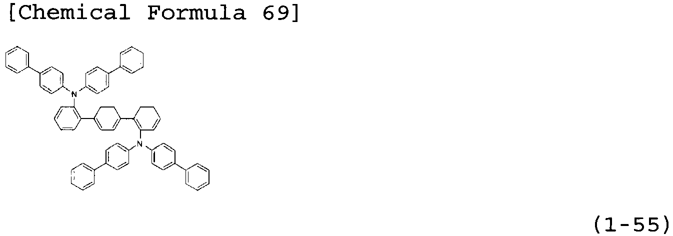

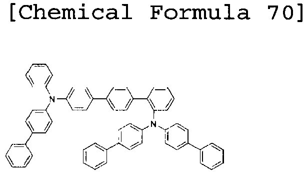

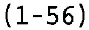

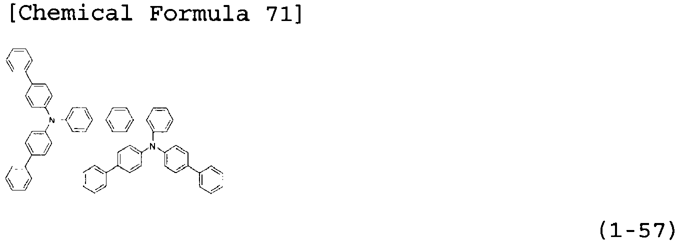

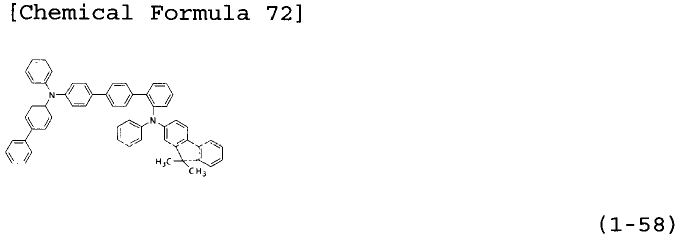

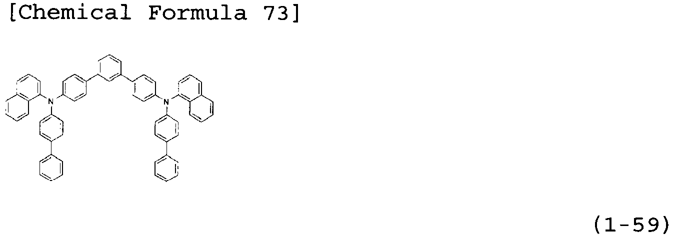

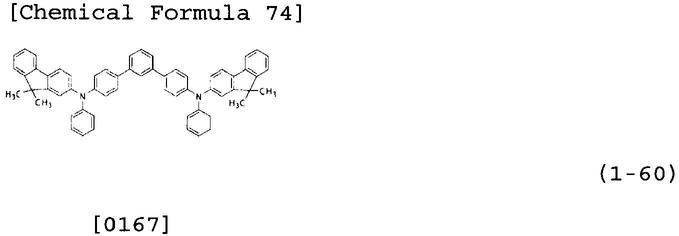

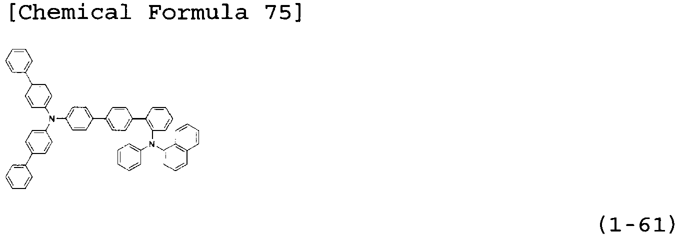

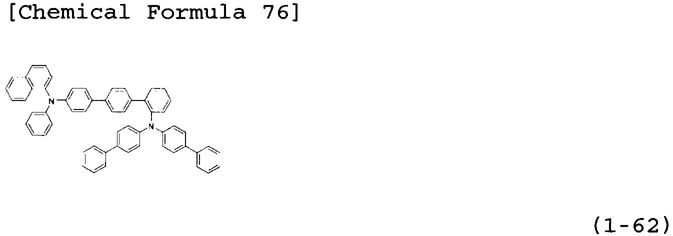

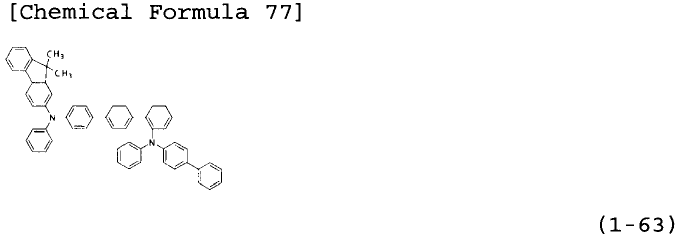

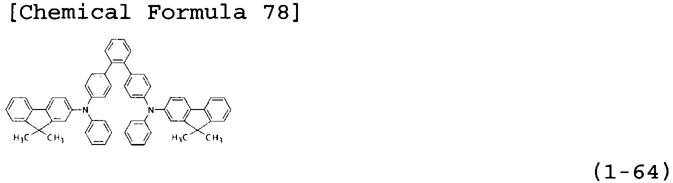

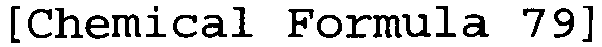

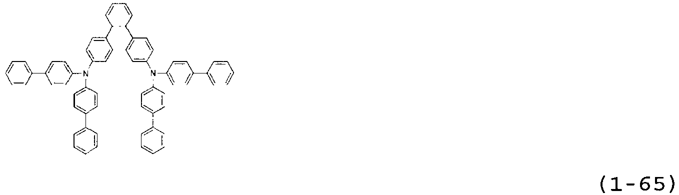

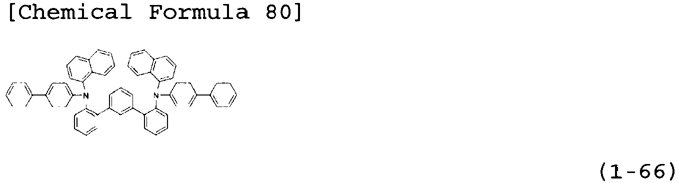

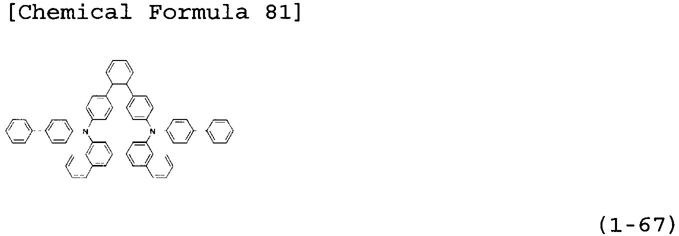

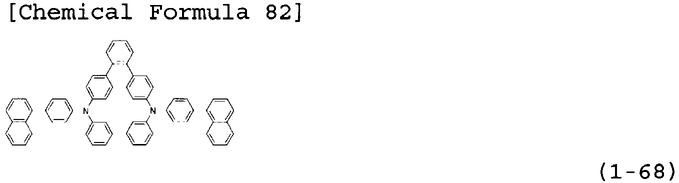

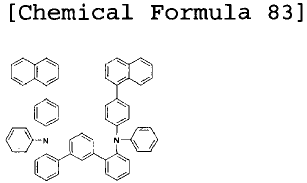

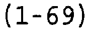

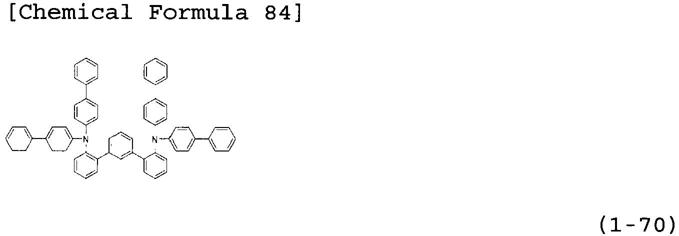

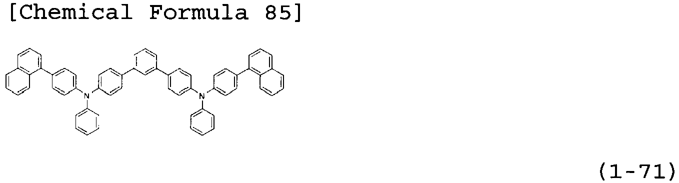

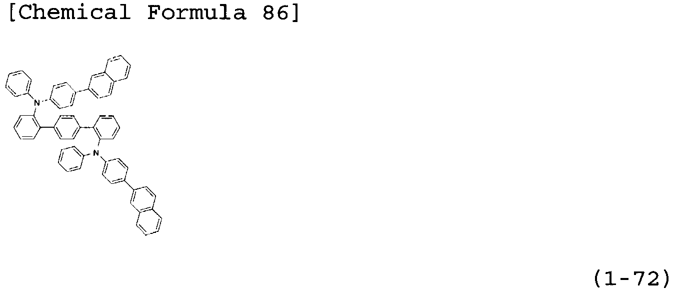

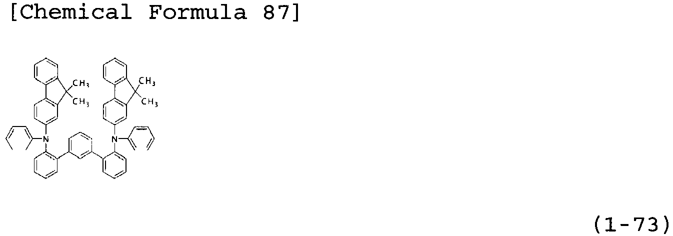

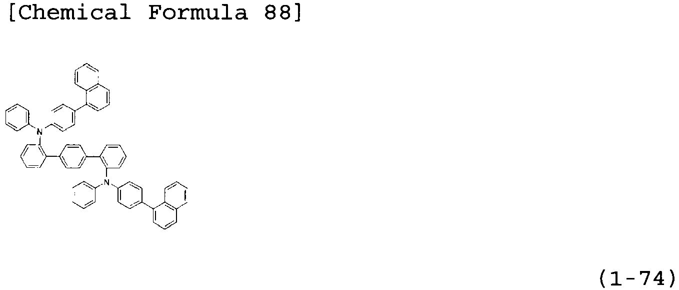

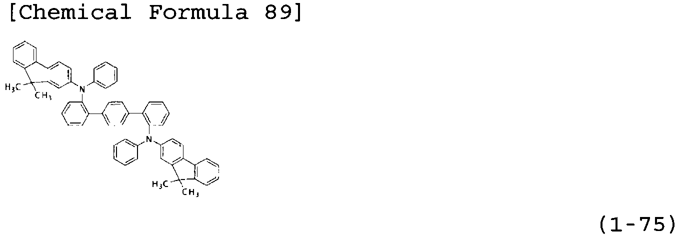

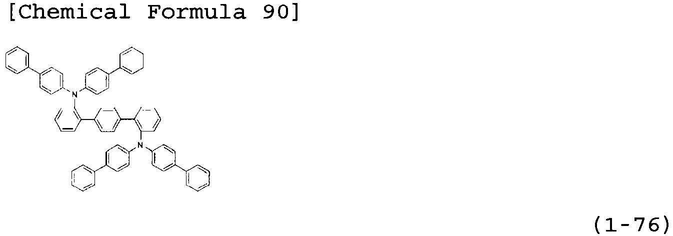

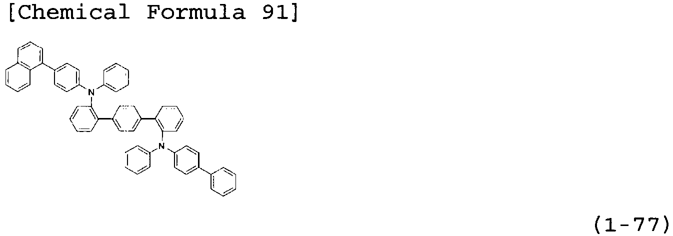

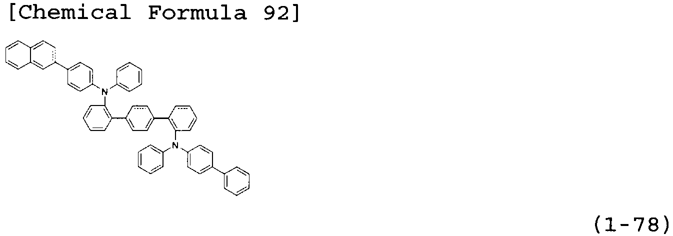

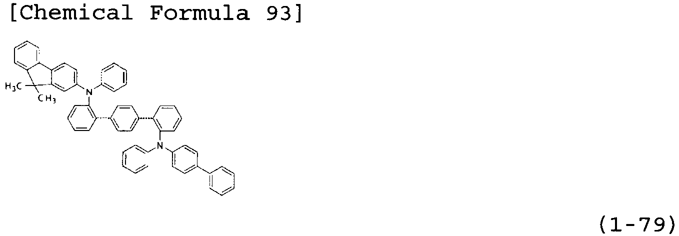

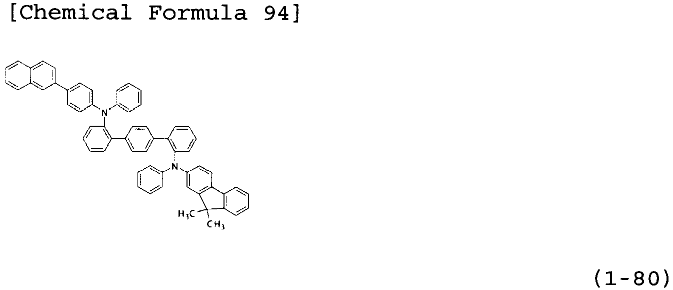

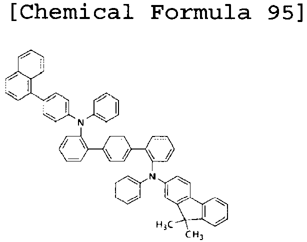

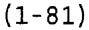

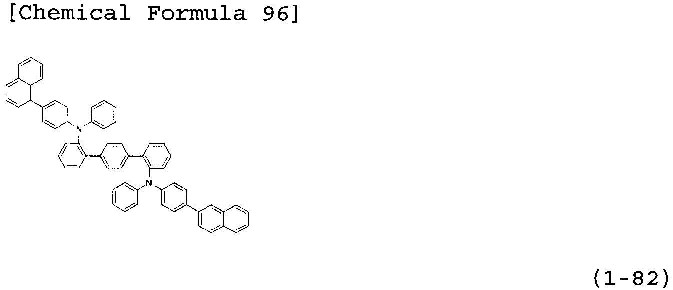

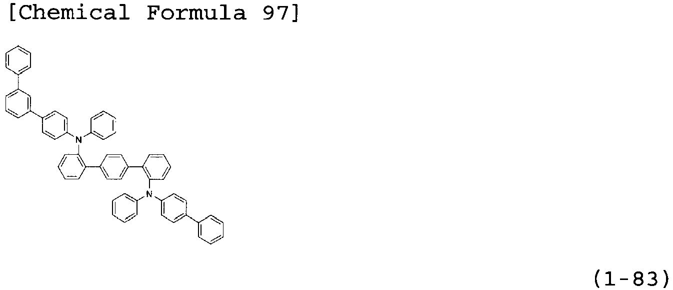

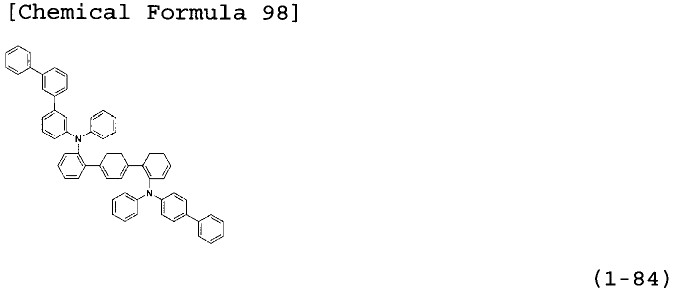

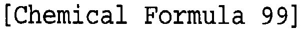

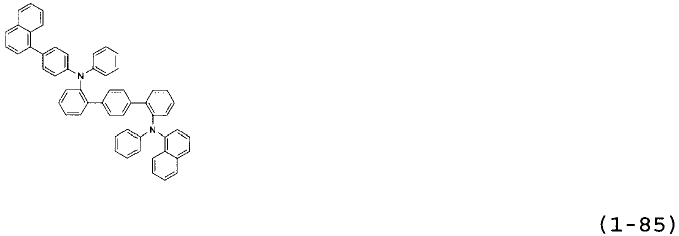

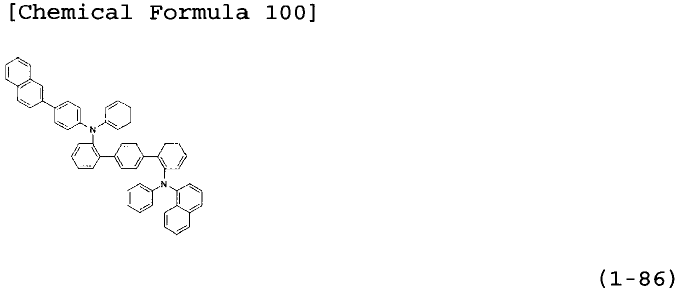

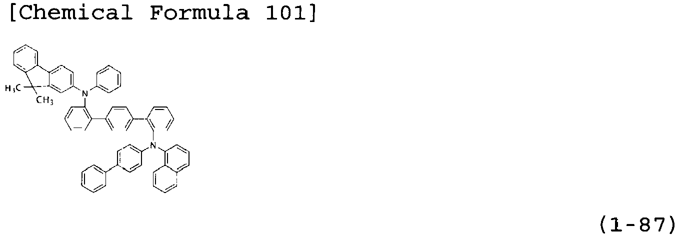

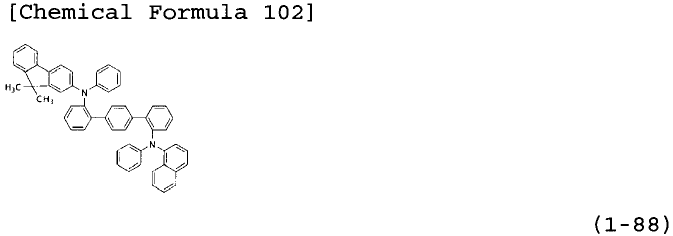

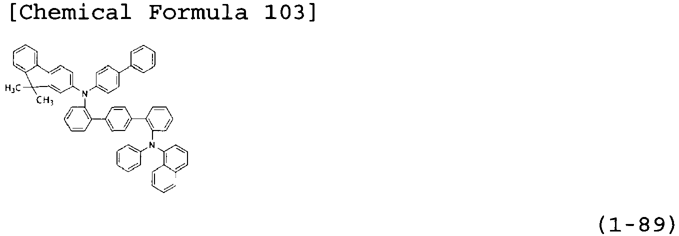

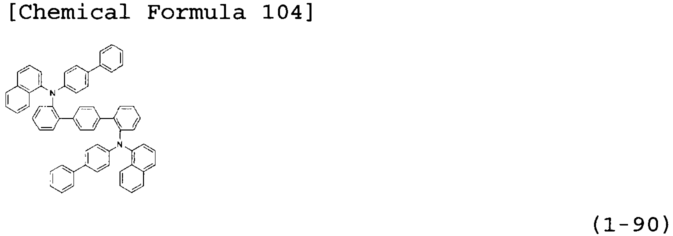

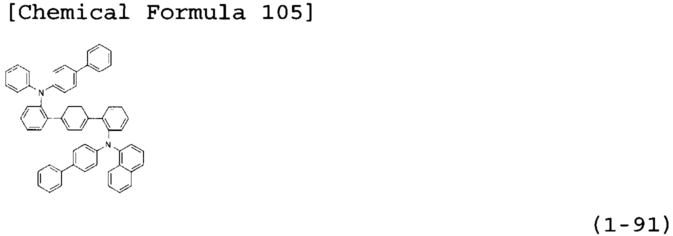

The arylamine compounds described above can be synthesized according to the known methods (refer to Patent Document 7, for example).

The following presents specific examples of preferred compounds among the arylamine compounds of the general formula (3) preferably used in the organic EL device of the present invention. The present invention, however, is not restricted to these compounds.

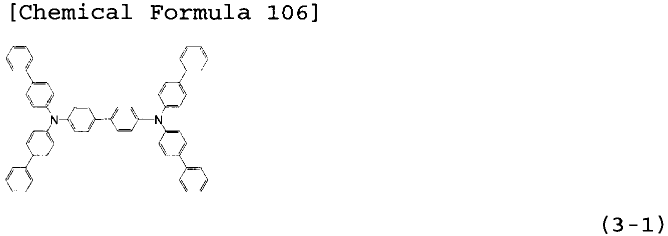

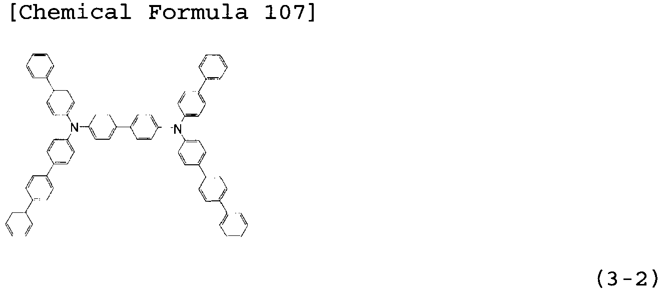

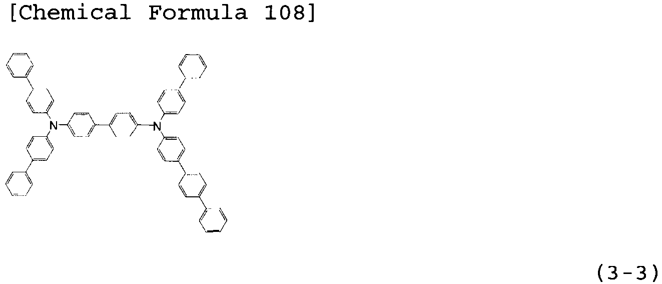

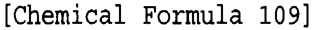

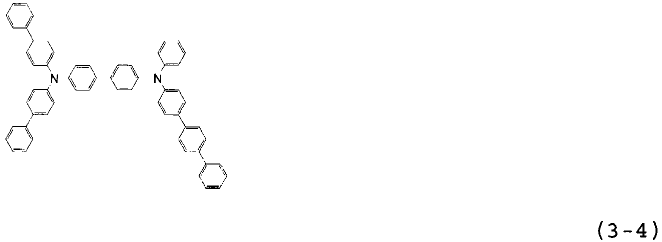

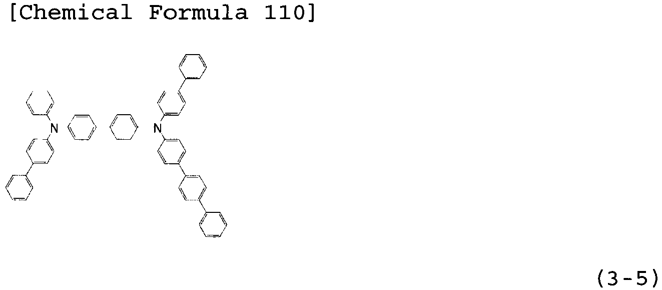

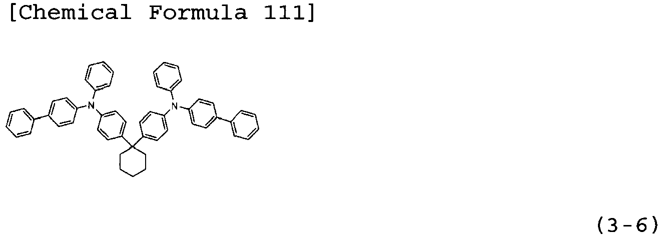

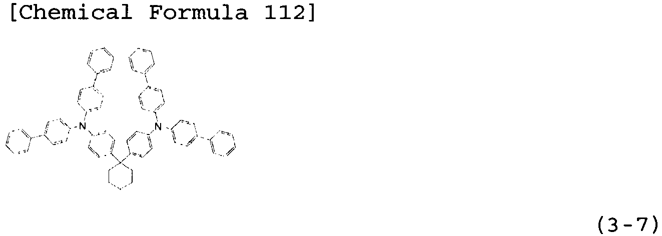

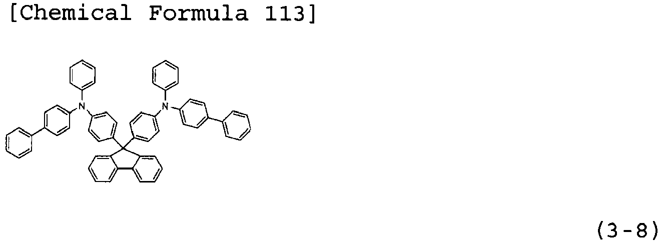

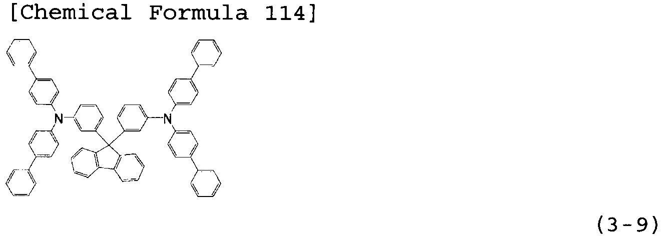

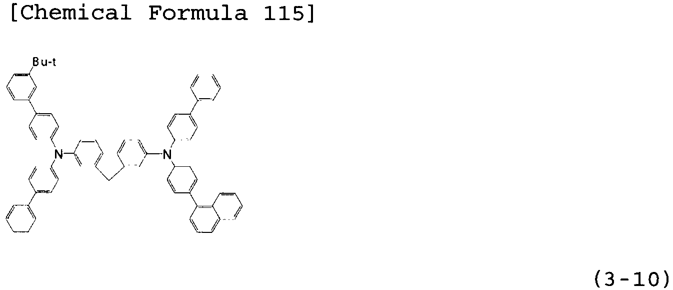

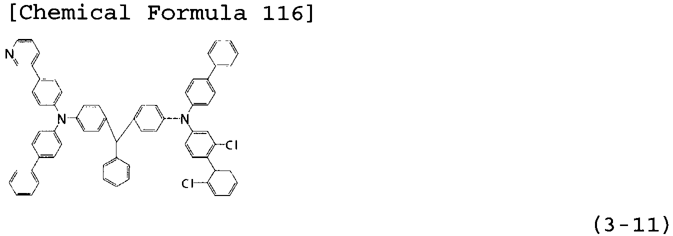

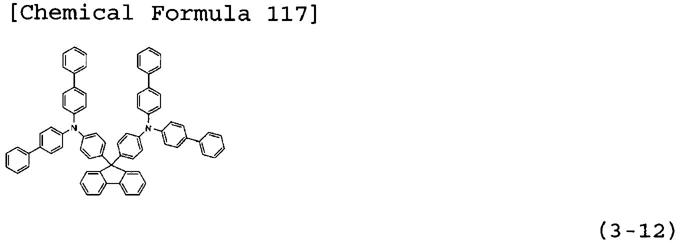

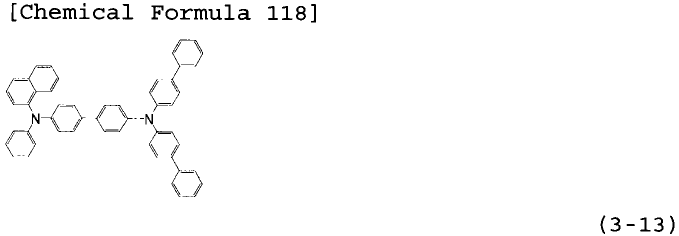

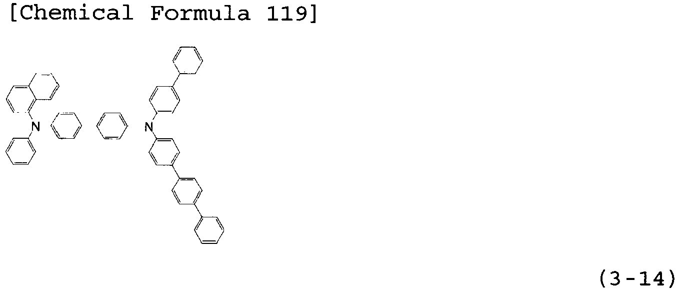

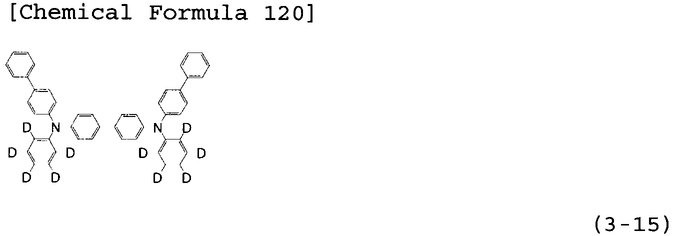

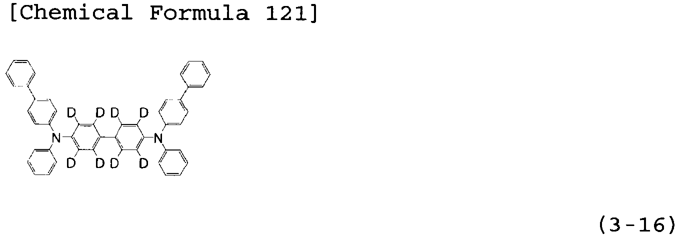

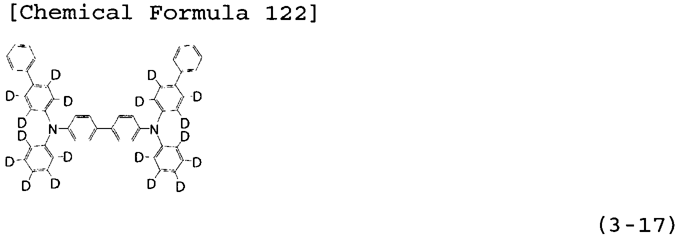

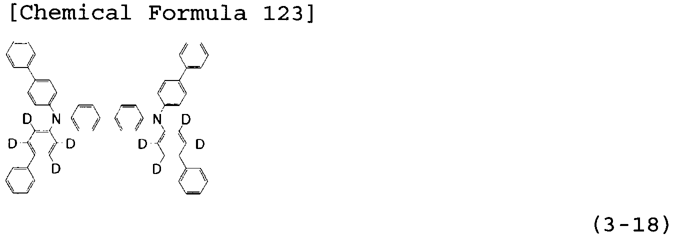

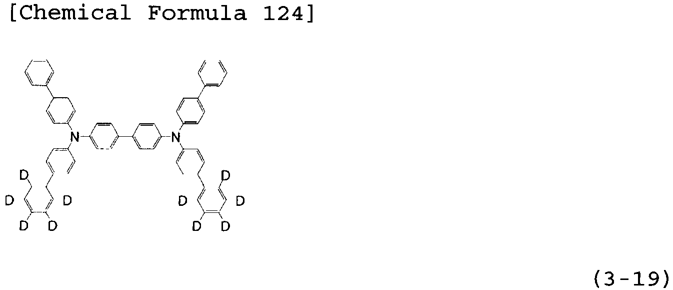

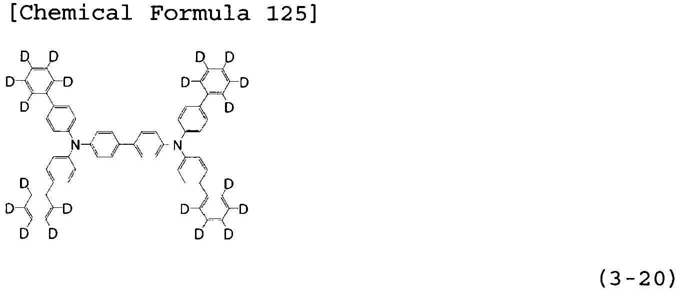

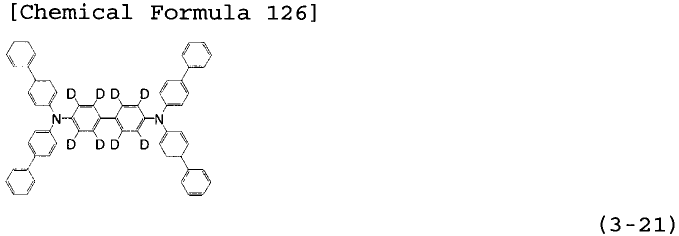

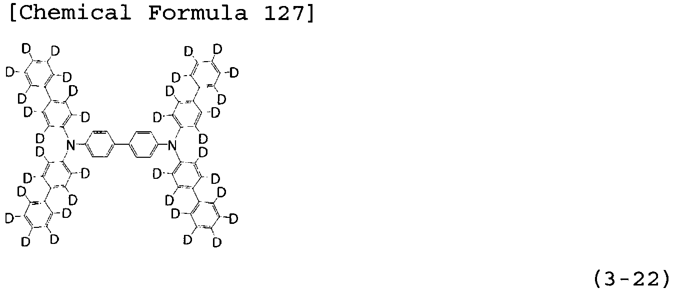

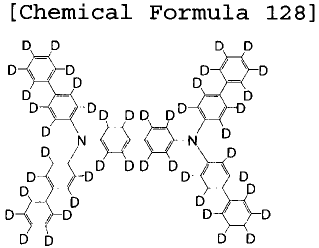

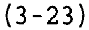

The following presents specific examples of preferred compounds of the arylamine compounds having two triphenylamine structures in the molecule among the triphenylamine compounds having a structure in which two to six triphenylamine structures in the molecule bind via a single bond or a divalent group that does not contain a heteroatom preferably used in the organic EL device of the present invention, in addition to the arylamine compounds of general formula (3). The present invention, however, is not restricted to these compounds.

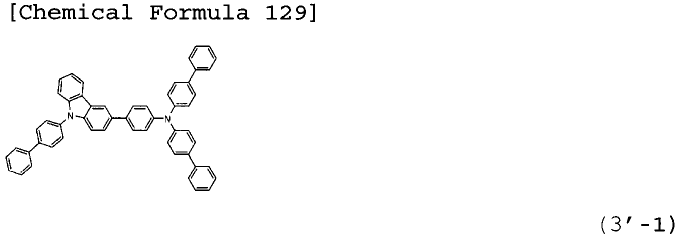

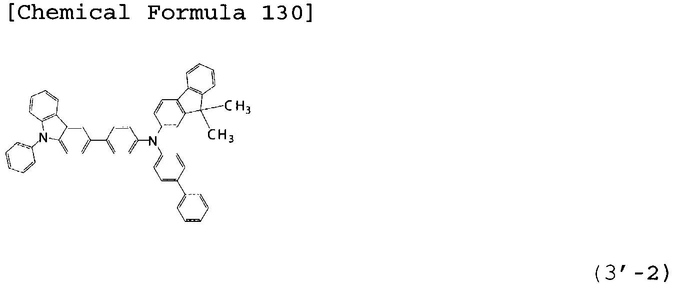

The following presents specific examples of preferred compounds among the arylamine compounds of the general formula (4) preferably used in the organic EL device of the present invention. The present invention, however, is not restricted to these compounds.

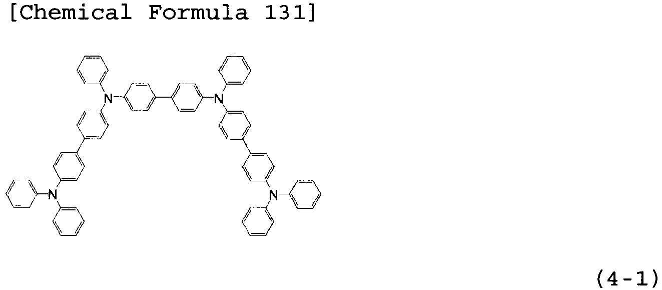

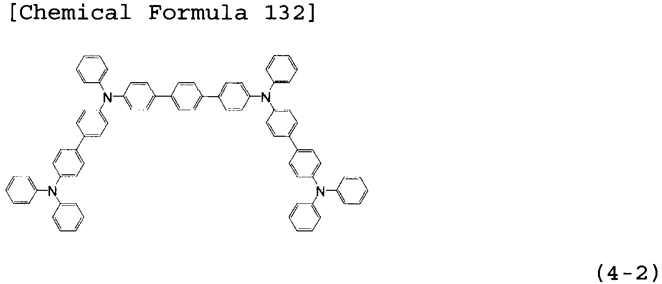

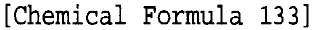

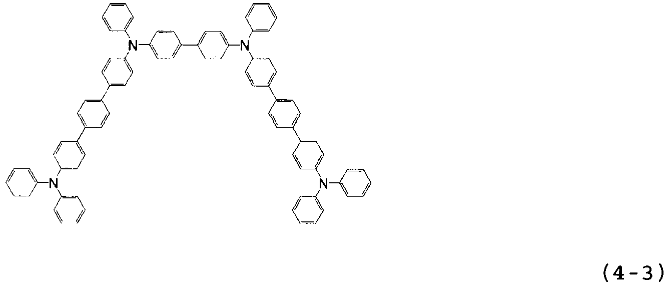

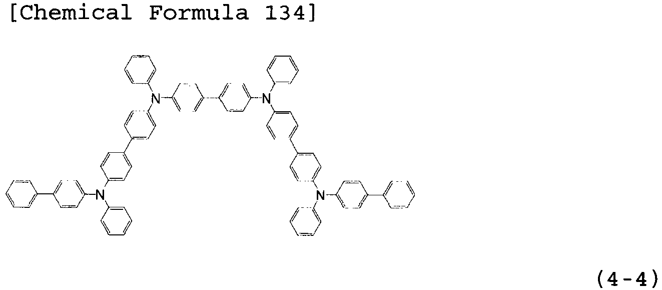

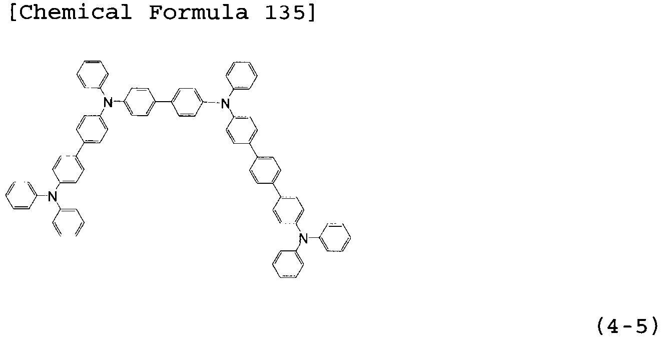

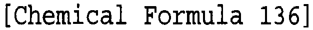

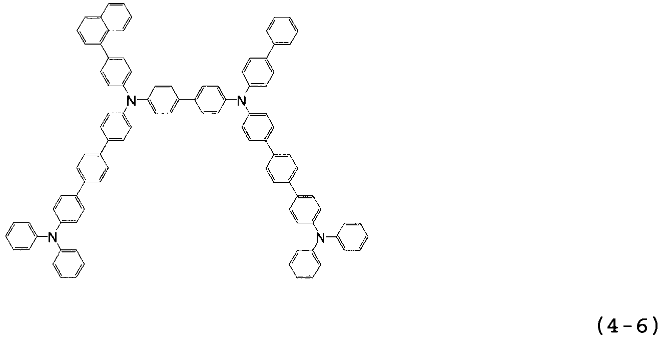

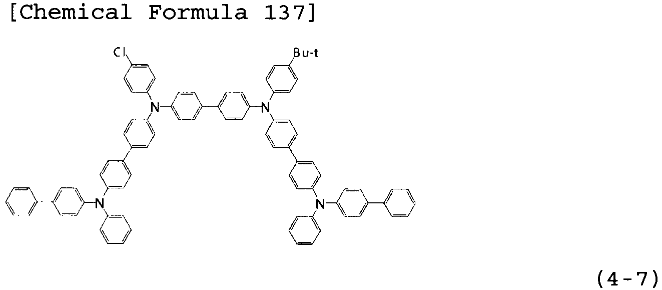

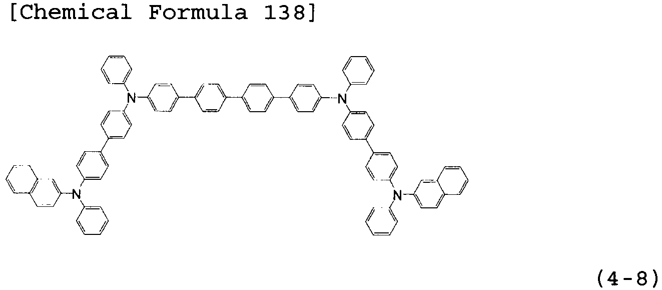

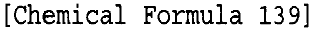

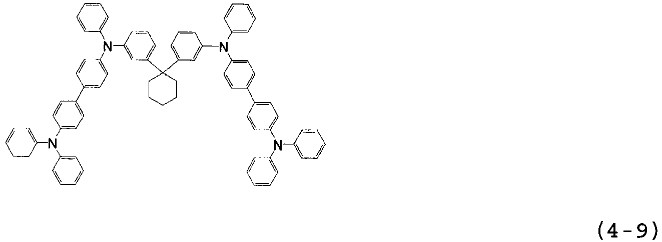

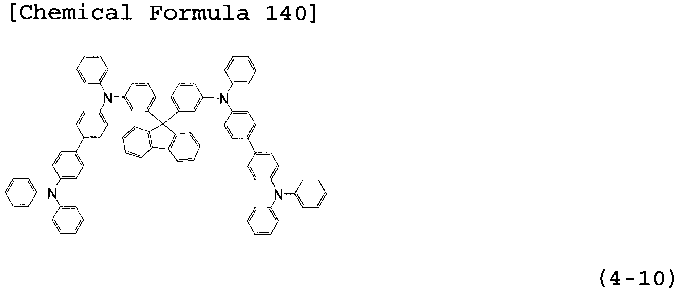

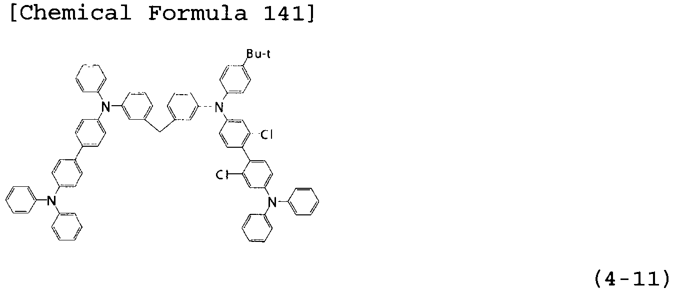

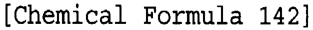

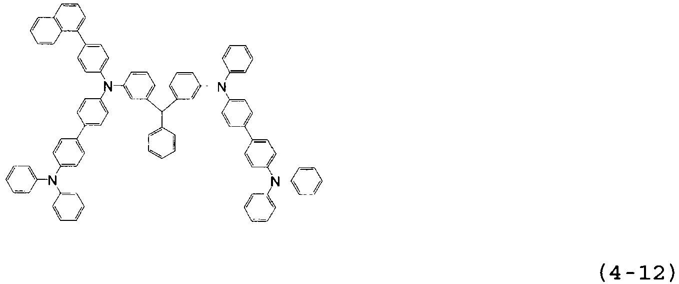

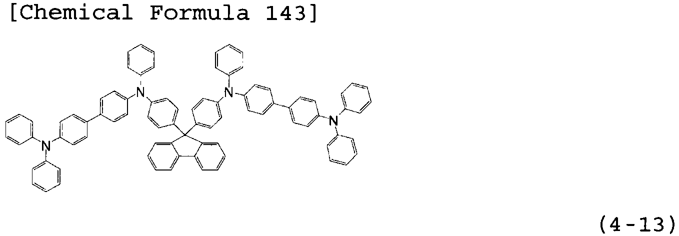

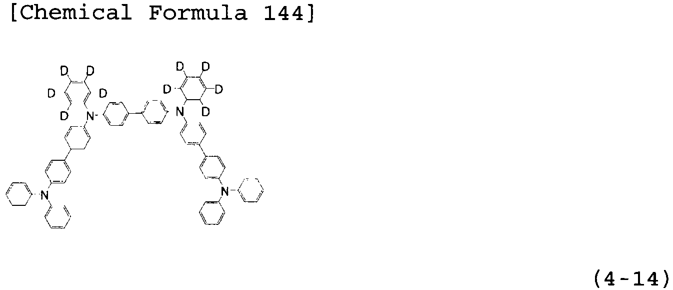

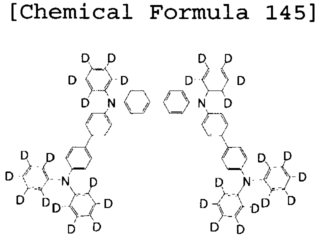

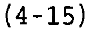

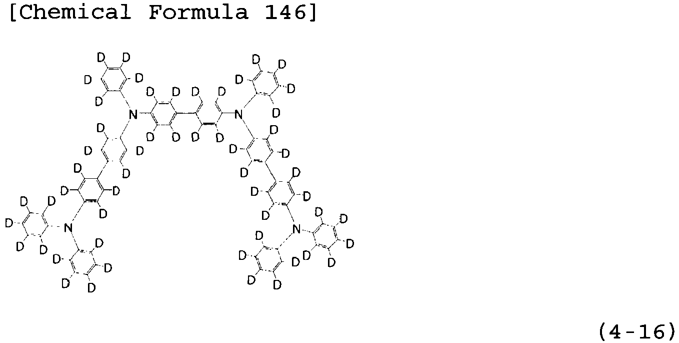

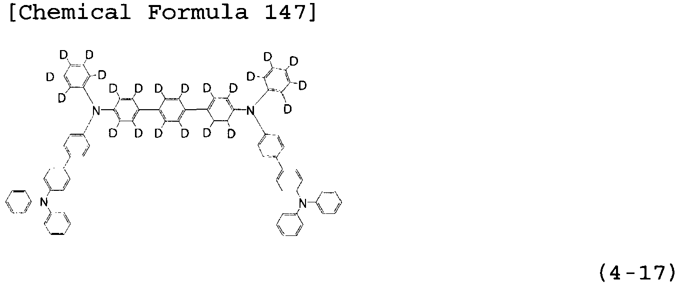

The arylamine compounds of the general formula (3) and the arylamine compounds of the general formula (4) can be synthesized by a known method (refer to Patent Documents 1, 8, 9, for example).

The following presents specific examples of preferred compounds among the compounds of the general formula (5a) preferably used in the organic EL device of the present invention and having an anthracene ring structure. The present invention, however, is not restricted to these compounds.

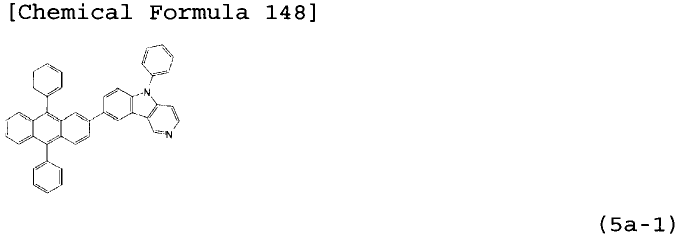

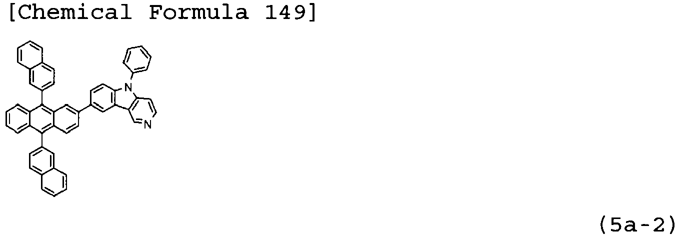

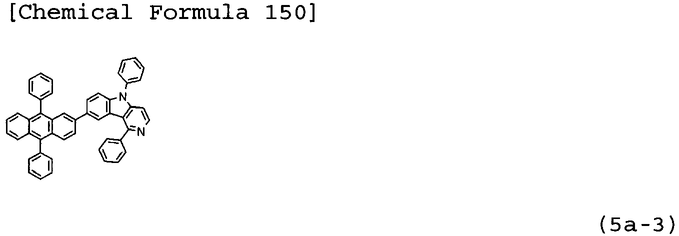

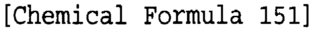

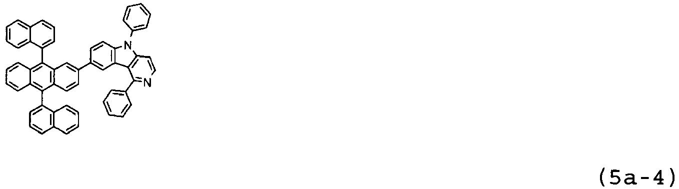

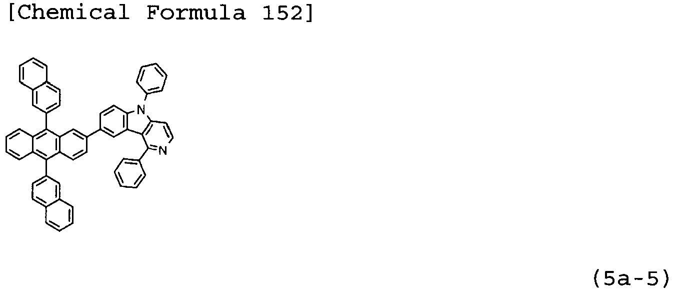

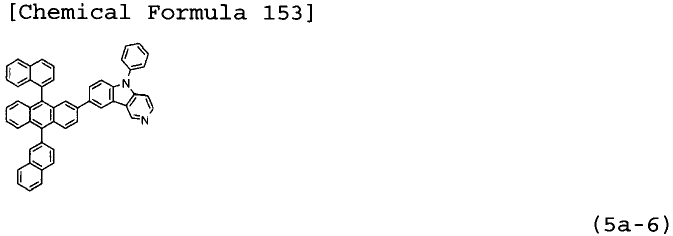

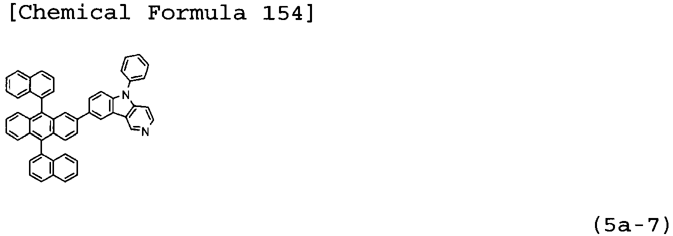

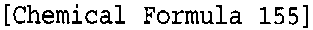

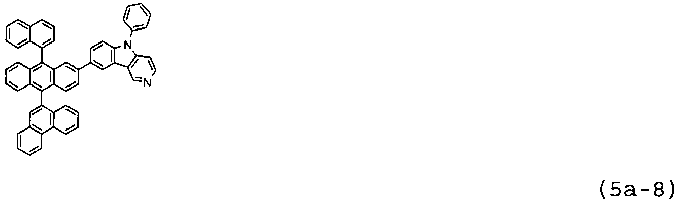

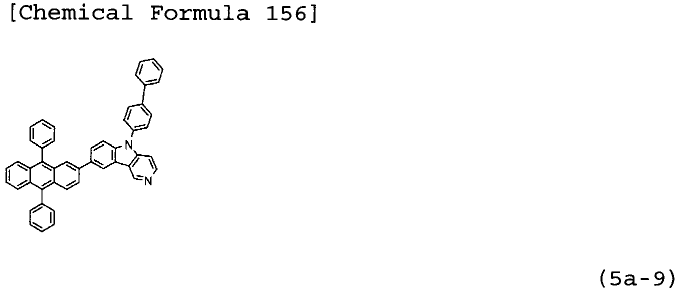

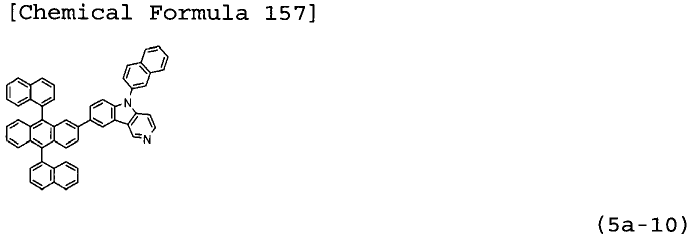

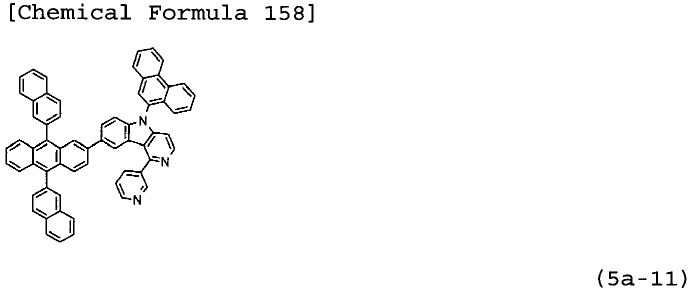

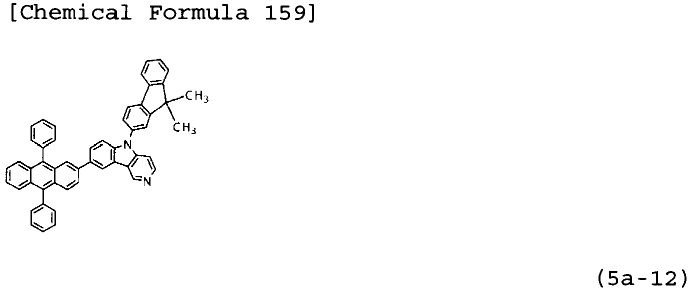

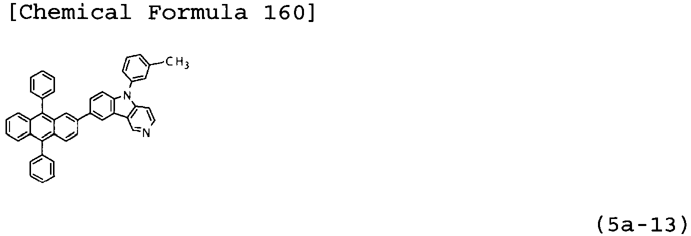

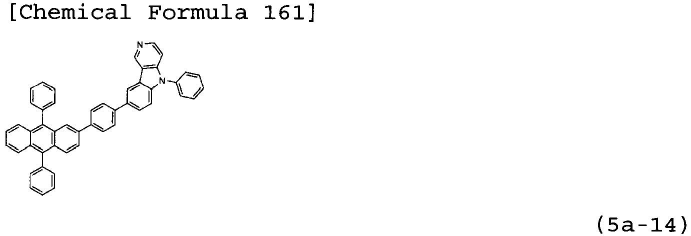

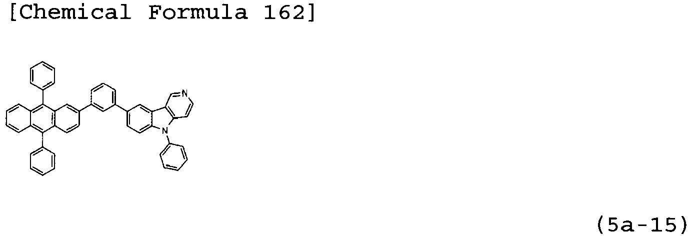

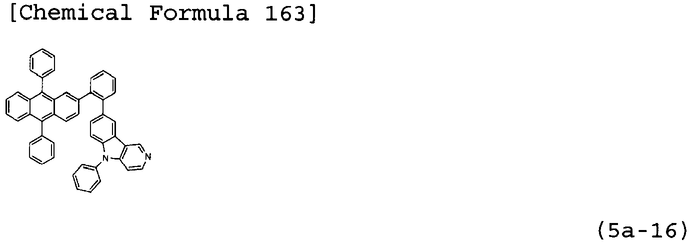

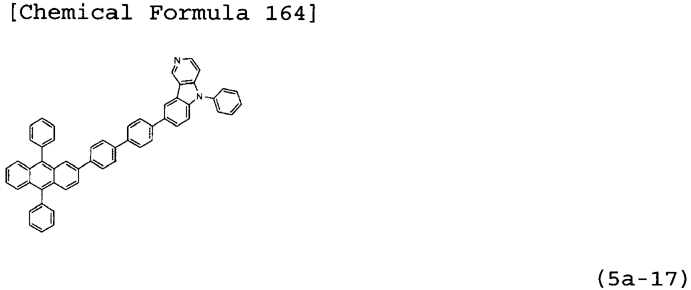

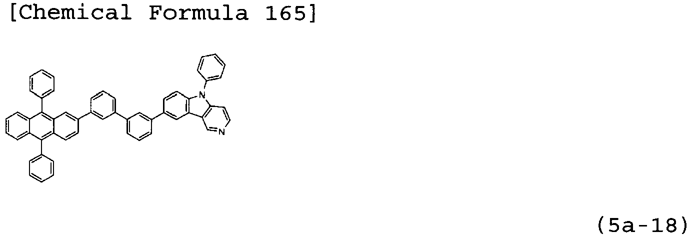

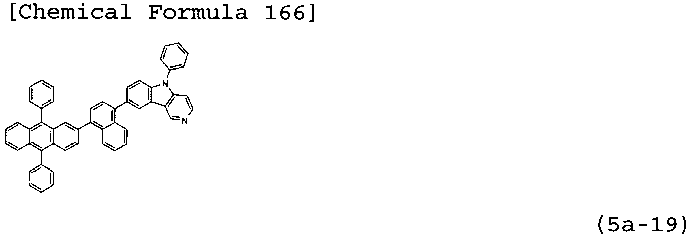

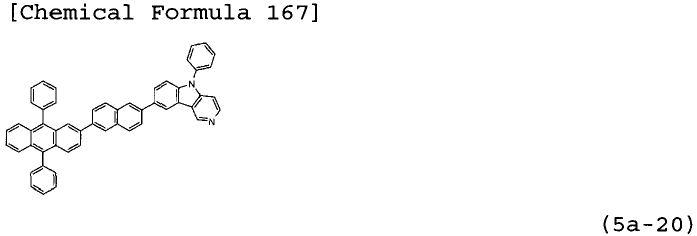

The following presents specific examples of preferred compounds among the compounds of the general formula (5b) preferably used in the organic EL device of the present invention and having an anthracene ring structure. The present invention, however, is not restricted to these compounds.

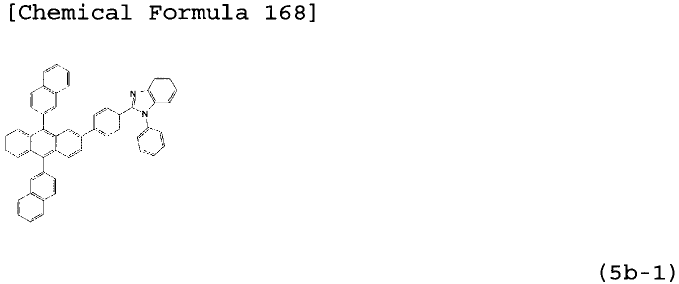

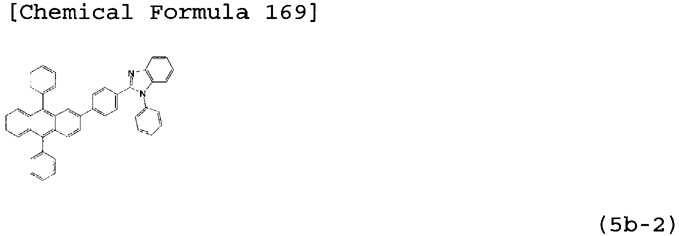

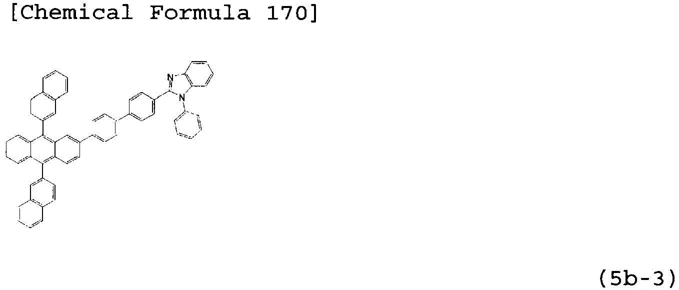

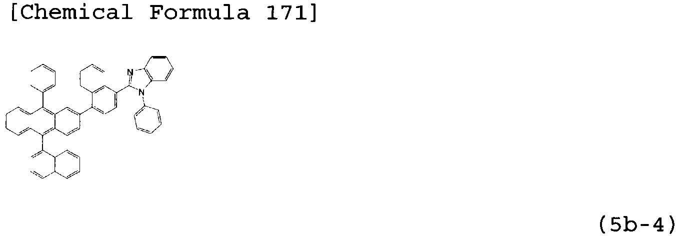

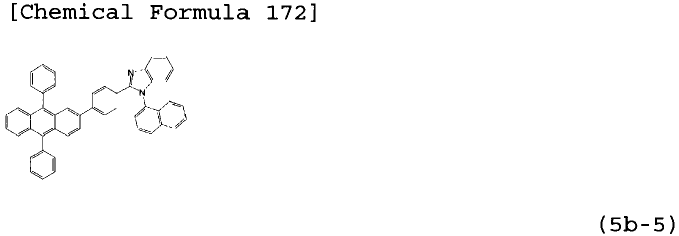

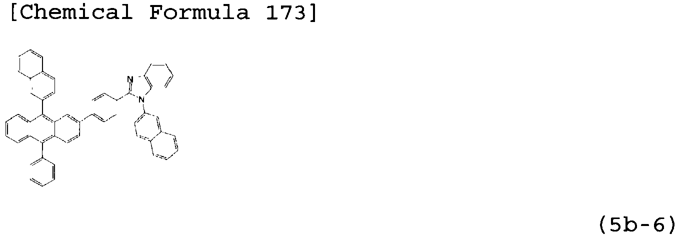

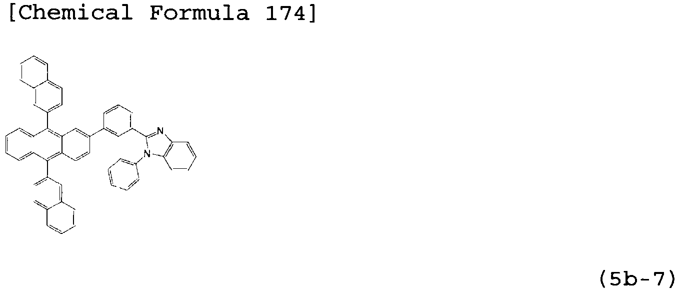

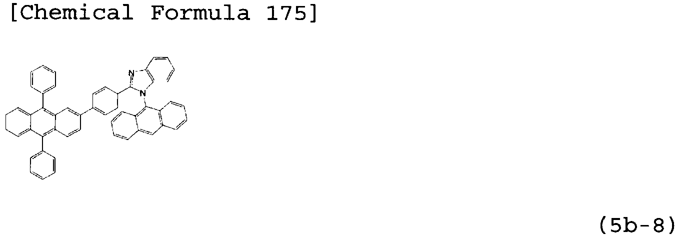

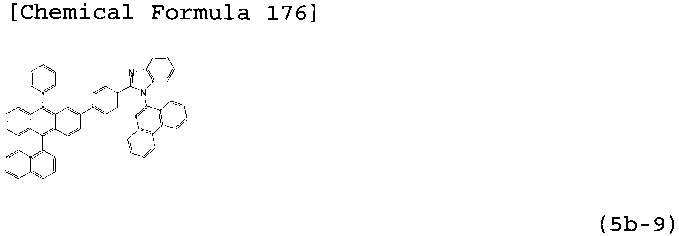

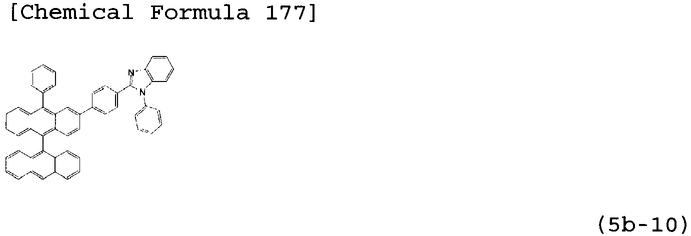

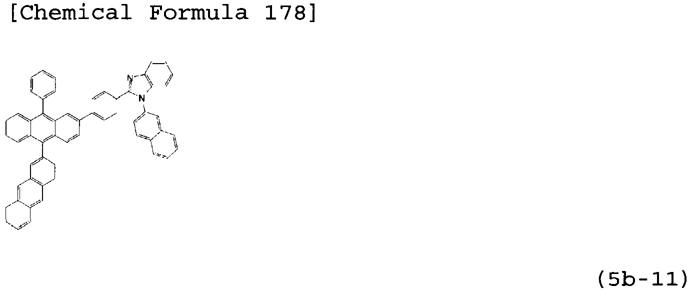

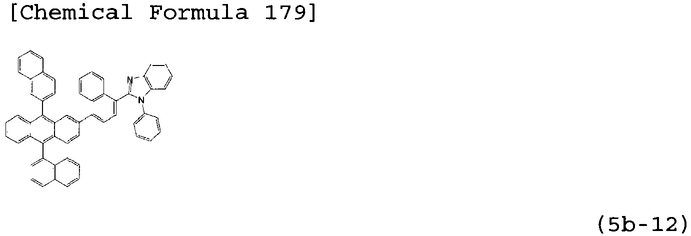

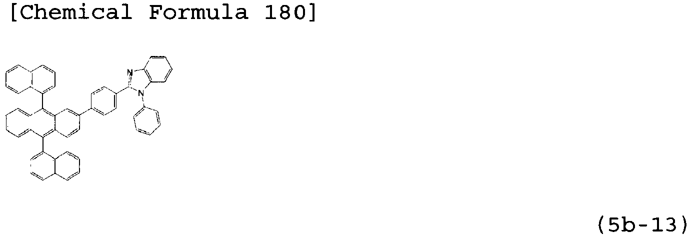

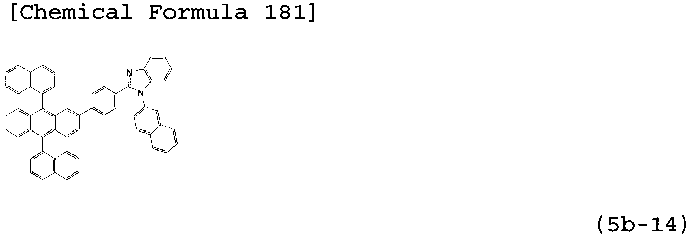

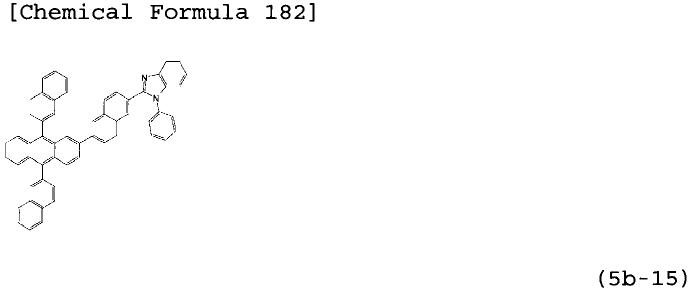

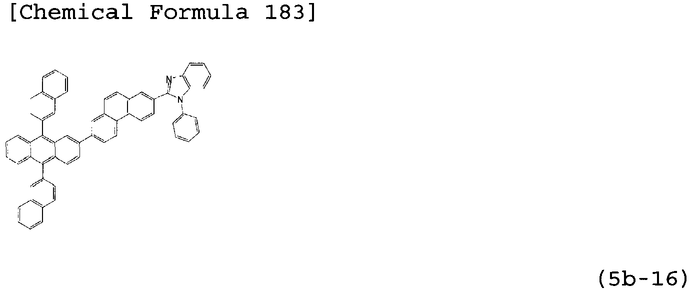

The following presents specific examples of preferred compounds among the compounds of the general formula (5c) preferably used in the organic EL device of the present invention and having an anthracene ring structure. The present invention, however, is not restricted to these compounds.

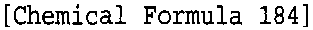

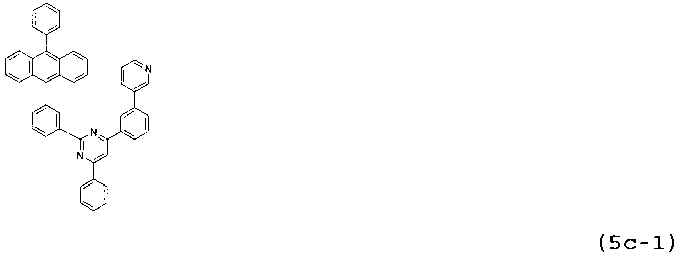

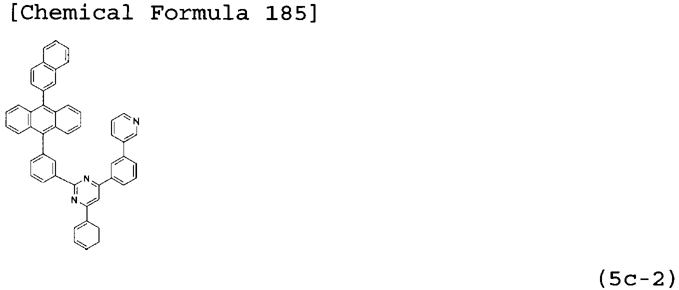

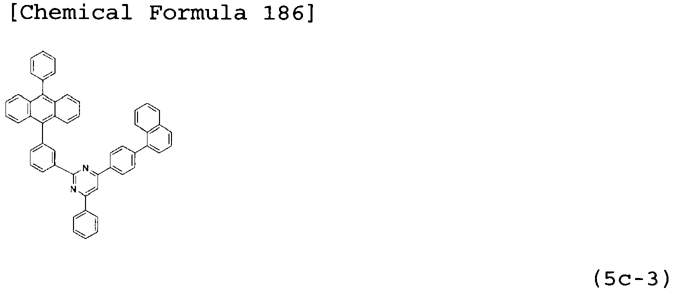

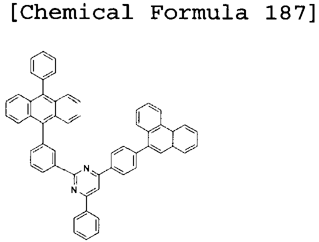

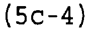

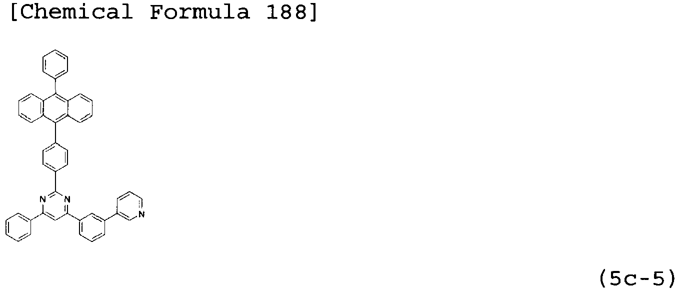

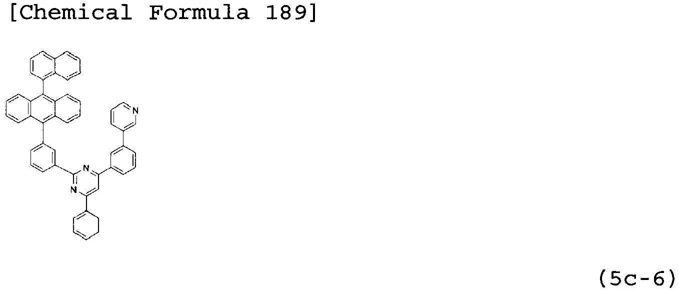

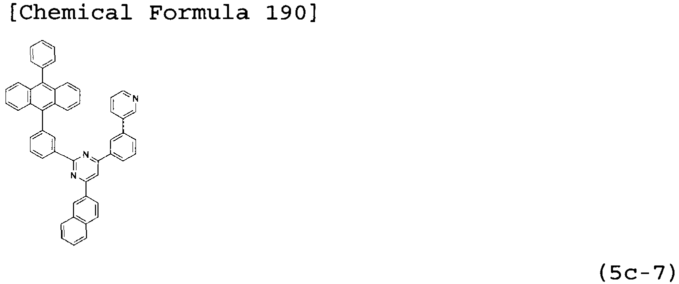

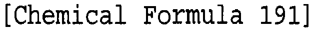

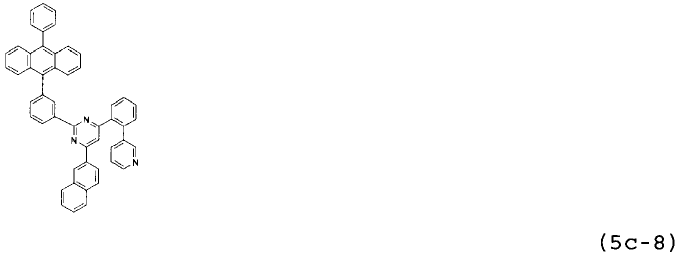

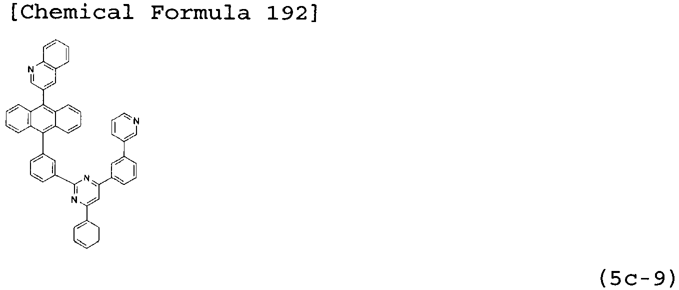

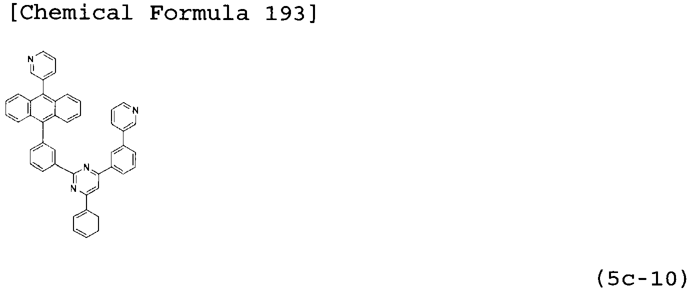

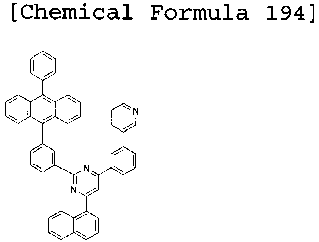

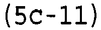

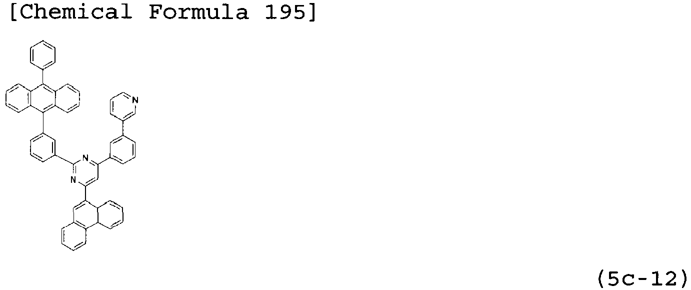

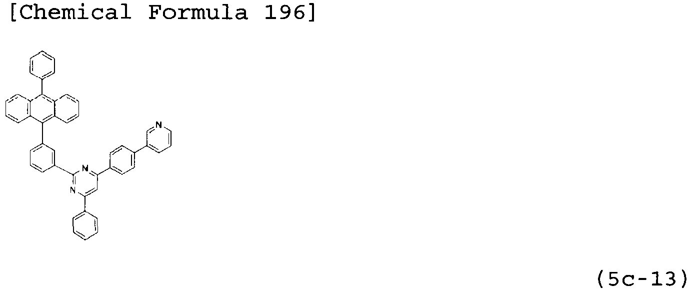

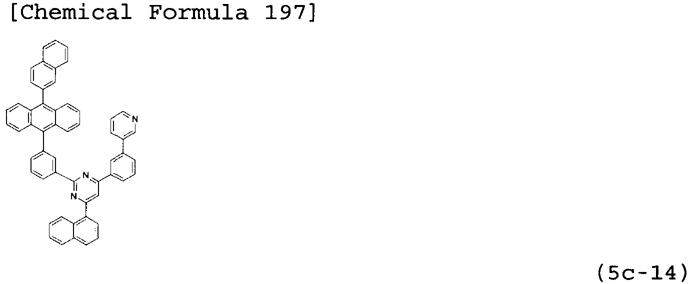

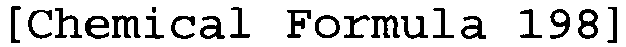

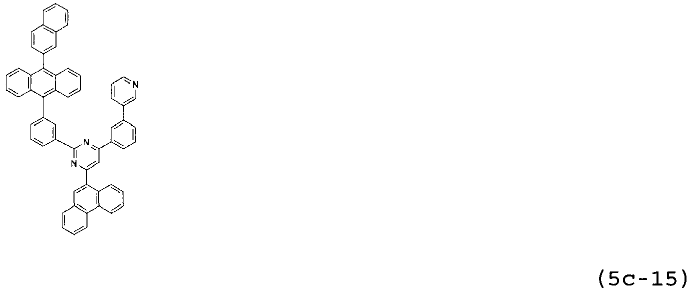

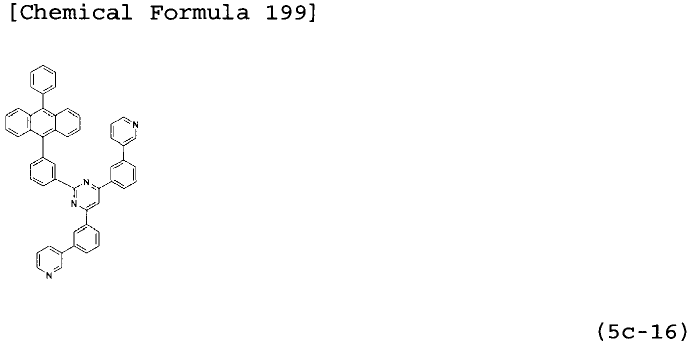

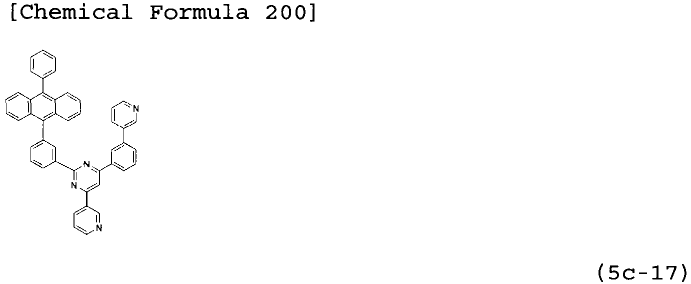

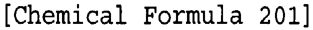

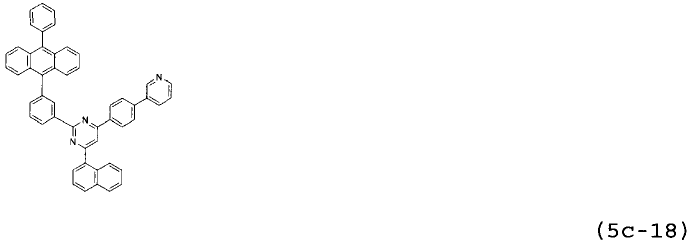

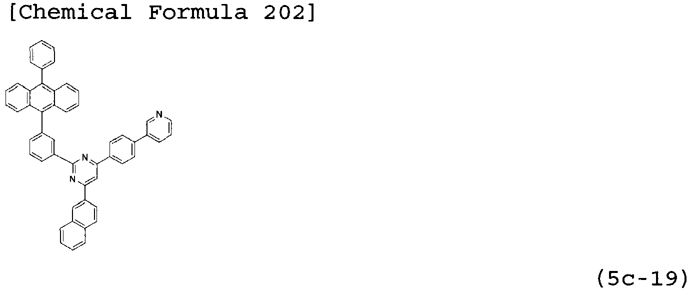

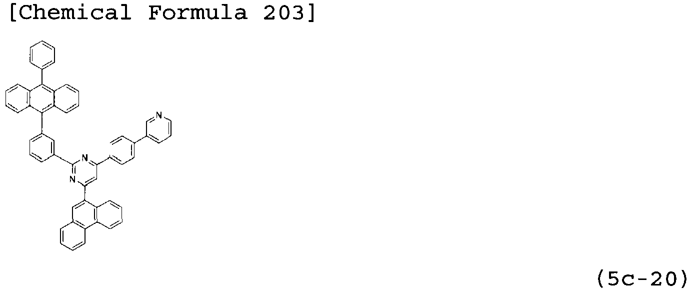

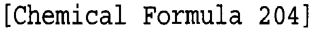

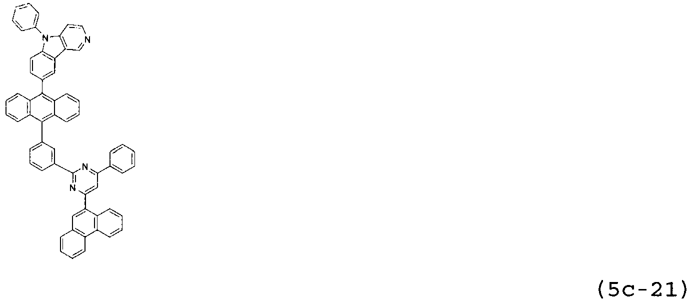

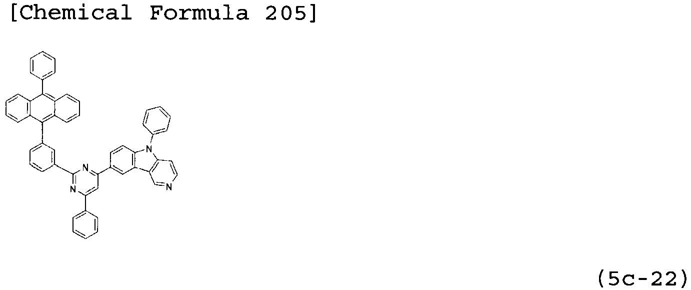

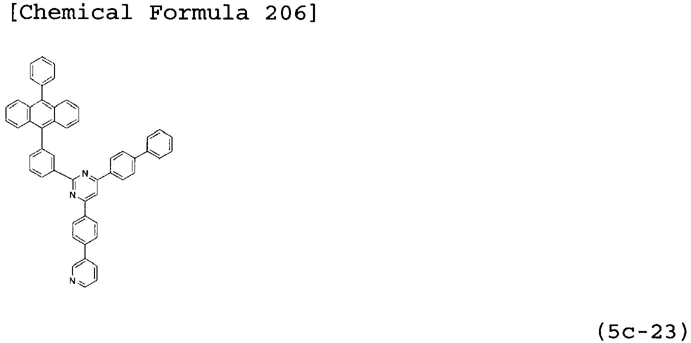

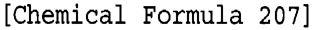

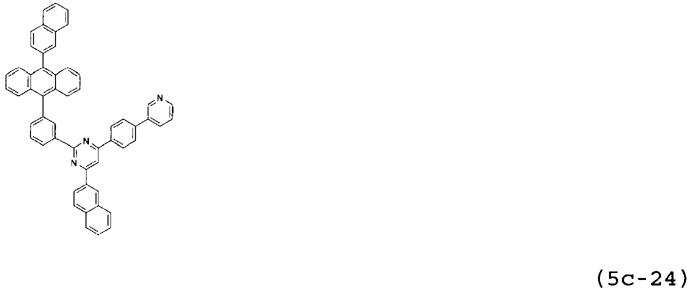

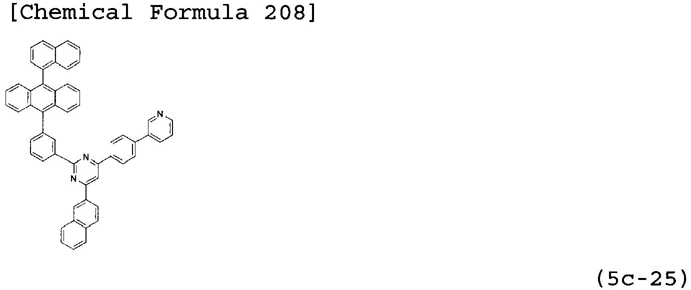

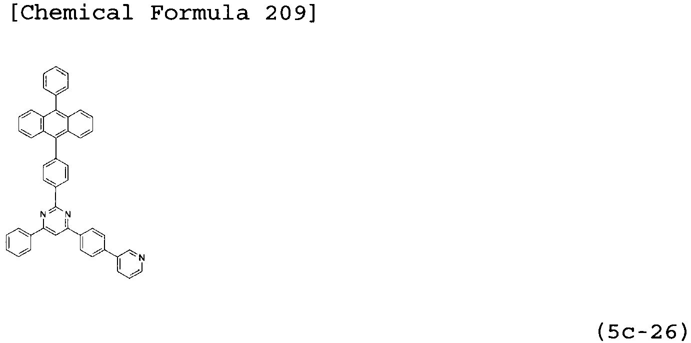

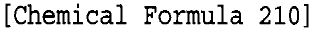

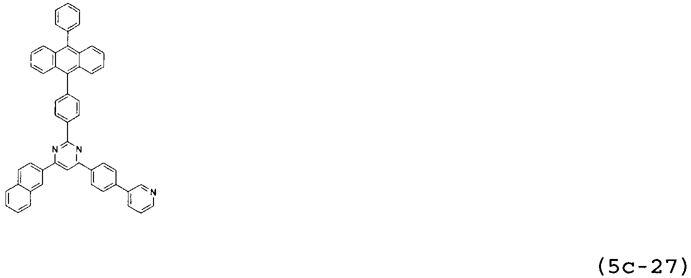

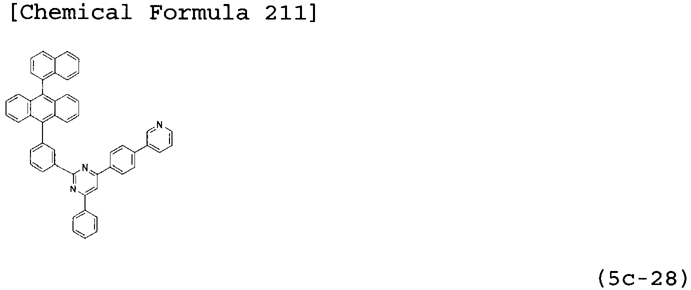

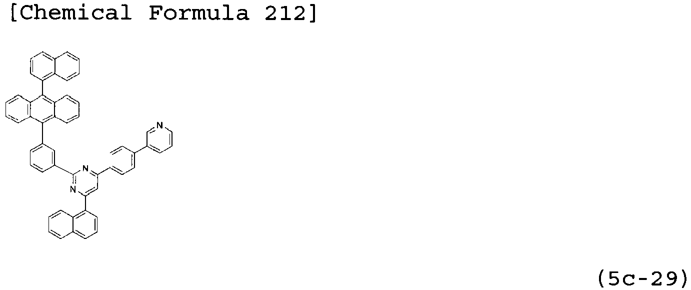

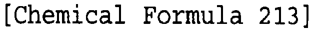

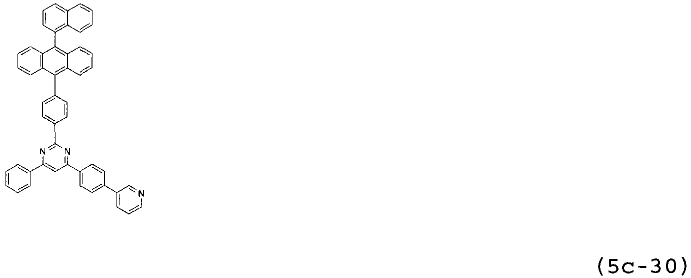

The compounds described above having an anthracene ring structure can be synthesized by a known method (refer to Patent Documents 10 to 12, for example).

The arylamine compounds of the general formula (1) were purified by methods such as column chromatography, adsorption using, for example, a silica gel, activated carbon, or activated clay, recrystallization or crystallization using a solvent, and a sublimation purification method. The compounds were identified by an NMR analysis. A melting point, a glass transition point (Tg), and a work function were measured as material property values. The melting point can be used as an index of vapor deposition, the glass transition point (Tg) as an index of stability in a thin-film state, and the work function as an index of hole transportability and hole blocking performance.

Other compounds used for the organic EL device of the present invention were purified by methods such as column chromatography, adsorption using, for example, a silica gel, activated carbon, or activated clay, and recrystallization or crystallization using a solvent, and finally purified by sublimation.

The melting point and the glass transition point (Tg) were measured by a high-sensitive differential scanning calorimeter (DSC3100SA produced by Bruker AXS) using powder.

For the measurement of the work function, a 100 nm-thick thin film was fabricated on an ITO substrate, and an ionization potential measuring device (PYS-202 produced by Sumitomo Heavy Industries, Ltd.) was used.

The organic EL device of the present invention may have a structure including an anode, a hole injection layer, a hole transport layer, a light emitting layer, an electron transport layer, an electron injection layer, and a cathode successively formed on a substrate, optionally with an electron blocking layer between the hole transport layer and the light emitting layer, and a hole blocking layer between the light emitting layer and the electron transport layer. Some of the organic layers in the multilayer structure may be omitted, or may serve more than one function. For example, a single organic layer may serve as the electron injection layer and the electron transport layer.

Further, the organic layers having a same function may have a laminate structure of two or more layers, for example, the hole transport layers may have a laminate structure of two or more layers, the light emitting layers may have a laminate structure of two or more layers, or the electron transport layers may have a laminate structure of two or more layers.

Electrode materials with high work functions such as ITO and gold are used as the anode of the organic EL device of the present invention.

As the hole injection layer of the organic EL device of the present invention, the arylamine compound of the general formula (1) subjected to p-type doping with an electron acceptor is preferably used. As a hole inj ection/transport material that can be mixed with or can be used simultaneously with the arylamine compound of the general formula (1), material such as starburst-type triphenylamine derivatives and various triphenylamine tetramers; porphyrin compounds as represented by copper phthalocyanine; accepting heterocyclic compounds such as hexacyanoazatriphenylene; coating-type polymer materials, and the like can be used. These materials may be formed into a thin film by a vapor deposition method or other known methods such as a spin coating method and an inkjet method.

The film thickness of the hole injection layer is 5 to 150 nm, preferably 5 to 100 nm, and particularly preferably 5 to 30 nm.

In the invention, for the doping concentration in the case where the electron acceptor is subjected to p-type doping, the doping is performed by co-deposition in a range of 0.5 to 30% by weight, preferably in a range of 1 to 20% by weight, more preferably in a range of 2 to 10% by weight, and particularly preferably in a range of 2 to 5% by weight, based on the total organic layer subjected to p-type doping.

In the hole transport layer of the organic EL device of the invention, the same arylamine compound as in the hole injection layer, but no electron acceptor is used. The compounds that are not subjected to p-type doping may be individually formed into a film, may be used as a single layer formed with another hole transport material mixed, or may be formed as a laminated structure of the individually deposited layers, a laminated structure of the mixed layers, or a laminated structure of the individually deposited layer and the mixed layer. These materials may be formed into a thin-film by a vapor deposition method or other known methods such as a spin coating method and an inkjet method.

As the electron blocking layer of the organic EL device of the present invention, the arylamine compound of the general formula (1) is preferably used, and in addition, compounds having an electron blocking effect can be used, for example, an arylamine compound having a structure in which four triphenylamine structures in the molecule are joined within a molecule via a single bond or a divalent group that does not contain a heteroatom, an arylamine compound having a structure in which two triphenylamine structures in the molecule are joined within a molecule via a single bond or a divalent group that does not contain a heteroatom, carbazole derivatives such as 4,4',4"-tri(N-carbazolyl)triphenylamine (TCTA), 9,9-bis[4-(carbazol-9-yl)phenyl]fluorene, 1,3-bis(carbazol-9-yl)benzene (mCP), and 2,2-bis(4-carbazol-9-ylphenyl)adamantane (Ad-Cz); and compounds having a triphenylsilyl group and a triarylamine structure, as represented by 9-[4-(carbazol-9-yl)phenyl]-9-[4-(triphenylsilyl)phenyl]-9H-fluorene. These may be individually formed into a film, may be used as a single layer formed with another hole transport material mixed, or may be formed as a laminated structure of the individually deposited layers, a laminated structure of the mixed layers, or a laminated structure of the individually deposited layer and the mixed layer. These materials may be formed into a thin-film by a vapor deposition method or other known methods such as a spin coating method and an inkjet method.

The compounds that are not subjected to p-type doping are preferably used.

In the organic EL device of the present invention, it is preferable that the electron acceptor in the layer adjacent to the light emitting layer (for example, the hole transport layer and the electron blocking layer) is not subjected to p-type doping.

In these layers, the arylamine compound of the general formula (3) or the arylamine compound of the general formula (4) is preferably used, and the arylamine compound of the general formula (1) is particularly preferably used.

The film thicknesses of these layers are not particularly limited as far as the film thicknesses are those having been ordinarily used, and for example, may be 20 to 300 nm for the hole transport layer (preferably 20 to 100 nm for the bottom emission type, and 100 to 200 nm for the top emission type), and may be 5 to 30 nm for the electron blocking layer.

When the film thicknesses are too large, the driving voltage tends to rise, and thus the film thicknesses are preferably appropriately determined.

Examples of material used for the light emitting layer of the organic EL device of the present invention can be various metal complexes, anthracene derivatives, bis(styryl)benzene derivatives, pyrene derivatives, oxazole derivatives, and polyparaphenylene vinylene derivatives, in addition to quinolinol derivative metal complexes such as Alq₃. Further, the light emitting layer may be made of a host material and a dopant material. Examples of the host material can be preferably anthracene derivatives. Other examples of the host material can be thiazole derivatives, benzimidazole derivatives, and polydialkyl fluorene derivatives, in addition to the above light-emitting materials. Examples of the dopant material can be preferably pyrene derivatives, amine derivatives having a condensed ring structure. Other examples of the dopant material can be quinacridone, coumarin, rubrene, perylene, derivatives thereof, benzopyran derivatives, indenophenanthrene derivatives, rhodamine derivatives, and aminostyryl derivatives. These may be individually deposited for film forming, may be used as a single layer deposited mixed with other materials, or may be formed as a laminate of individually deposited layers, a laminate of mixedly deposited layers, or a laminate of the individually deposited layer and the mixedly deposited layer.

Further, the light-emitting material may be a phosphorescent material. Phosphorescent materials as metal complexes of metals such as iridium and platinum may be used. Examples of the phosphorescent materials include green phosphorescent materials such as Ir(ppy)₃, blue phosphorescent materials such as FIrpic and FIr6, and red phosphorescent materials such as Btp₂Ir(acac). Here, carbazole derivatives such as 4,4'-di(N-carbazolyl)biphenyl (CBP), TCTA, and mCP may be used as the hole injecting and transporting host material. Compounds such as p-bis(triphenylsilyl)benzene (UGH2) and 2,2',2''-(1,3,5-phenylene)-tris(1-phenyl-1H-benzimidazole) (TPBI) may be used as the electron transporting host material. In this way, a high-performance organic EL device can be produced.

In order to avoid concentration quenching, the doping of the host material with the phosphorescent light-emitting material should preferably be made by co-evaporation in a range of 1 to 30 weight percent with respect to the whole light emitting layer.

Further, Examples of the light-emitting material may be delayed fluorescent-emitting material such as a CDCB derivative of PIC-TRZ, CC2TA, PXZ-TRZ, 4CzIPN or the like (refer to Non-Patent Document 3, for example).

These materials may be formed into a thin film by a vapor deposition method or other known methods such as a spin coating method and an inkjet method.

The hole blocking layer of the organic EL device of the present invention may be formed by using hole blocking compounds such as various rare earth complexes, triazole derivatives, triazine derivatives, and oxadiazole derivatives, in addition to the metal complexes of phenanthroline derivatives such as bathocuproin (BCP), and the metal complexes of quinolinol derivatives such as aluminum(III) bis(2-methyl-8-quinolinate)-4-phenylphenolate (BAlq). These materials may also serve as the material of the electron transport layer. These may be individually deposited for film forming, may be used as a single layer deposited mixed with other materials, or may be formed as a laminate of individually deposited layers, a laminate of mixedly deposited layers, or a laminate of the individually deposited layer and the mixedly deposited layer. These materials may be formed into a thin-film by using a vapor deposition method or other known methods such as a spin coating method and an inkjet method.

In the electron transport layer of the organic EL device of the invention, the compound having an anthracene ring structure of the general formula (5) is preferably used, and in addition, a metal complex of a quinolinol derivative, such as Alq₃ and BAlq, various metal complexes, a triazole derivative, a triazine derivative, an oxadiazole derivative, a pyridine derivative, a pyrimidine derivative, a benzimidazole derivative, a thiadiazole derivative, an anthracene derivative, a carbodiimide derivative, a quinoxaline derivative, a pyridoindole derivative, a phenanthroline derivative, a silole derivative, and the like may also be used. These may be individually formed into a film, may be used as a single layer formed with another material mixed, or may be formed as a laminated structure of the individually deposited layers, a laminated structure of the mixed layers, or a laminated structure of the individually deposited layer and the mixed layer. These materials may be formed into a thin film by a vapor deposition method or other known methods such as a spin coating method and an inkjet method.

Examples of material used for the electron injection layer of the organic EL device of the present invention can be alkali metal salts such as lithium fluoride and cesium fluoride; alkaline earth metal salts such as magnesium fluoride; and metal oxides such as aluminum oxide. However, the electron injection layer may be omitted in the preferred selection of the electron transport layer and the cathode.

The cathode of the organic EL device of the present invention may be made of an electrode material with a low work function such as aluminum, or an alloy of an electrode material with an even lower work function such as a magnesium-silver alloy, a magnesium-indium alloy, or an aluminum-magnesium alloy.

The following describes an embodiment of the present invention in more detail based on Examples. The present invention, however, is not restricted to the following Examples.

### Example 1

### <Synthesis of 4,"-bis{(biphenyl-4-yl)-phenylamino}-1,1':4',1"-terphenyl (Compound 1-1)>

(Biphenyl-4-yl)-phenylamine (39.5 g), 4,"-diiodo-1,1':4',1"-terphenyl (32.4 g), a copper powder (0.42 g), potassium carbonate (27.8 g), 3,5-di-tert-butylsalicylic acid (1.69 g), sodium bisulfite (2.09 g), dodecylbenzene (32 ml), and toluene (50 ml) were added into a reaction vessel and heated up to 210°C while removing the toluene by distillation. After the obtained product was stirred for 30 hours, the product was cooled, and toluene (50 ml) and methanol (100 ml) were added. A precipitated solid was collected by filtration and washed with a methanol/water (5/1, v/v) mixed solution (500 ml). The solid was heated after adding 1,2-dichlorobenzene (350 ml), and insoluble matter was removed by filtration. After the filtrate was left to cool, methanol (400 ml) was added, and a precipitated crude product was collected by filtration. The crude product was washed under reflux with methanol (500 ml) to obtain a gray powder of 4,4"-bis{(biphenyl-4-yl)-phenylaminol-1,1':4',1"-terphenyl (Compound 1-1; 45.8 g; yield 91%).

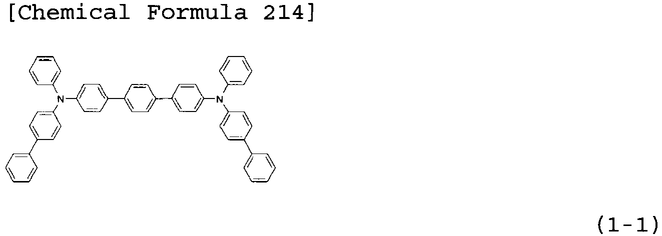

The structure of the obtained gray powder was identified by NMR.

¹H-NMR (CDCl₃) detected 40 hydrogen signals, as follows.

δ (ppm) = 7.68-7.63 (4H), 7.62-7.48 (12H), 7.45 (4H), 7.38-7.10 (20H).

### Example 2

### <Synthesis of 4,"-bis((biphenyl-4-yl)-4-tolylamino}-1,1':4',1'-terphenyl (Compound 1-10)>

(Biphenyl-4-yl)-4-tolylamine (16.7 g), 4,4"-diiodo-1,1':4',1"-terphenyl (12.9 g), a copper powder (0.17 g), potassium carbonate (11.2 g), 3,5-di-tert-butylsalicylic acid (0.71 g), sodium bisulfite (0.89 g), dodecylbenzene (20 ml), and toluene (20 ml) were added into a reaction vessel and heated up to 210°C while removing the toluene by distillation. The obtained product was stirred for 28 hours, and after the product was cooled, toluene (150 ml) was added, and insoluble matter was removed by filtration. Methanol (100 ml) was added, and a precipitated crude product was collected by filtration. Recrystallization of the crude product using a toluene/methanol mixed solvent was repeated three times to obtain a yellowish white powder of 4,4"-bis{(biphenyl-4-yl)-4-tolylamino}-1,1':4',1"-terphenyl (Compound 1-10; 12.3 g; yield 61%).

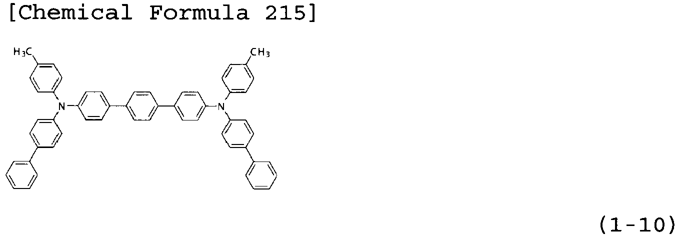

The structure of the obtained a pale yellowish white powder was identified by NMR.

¹H-NMR (CDCl₃) detected 44 hydrogen signals, as follows.

δ (ppm) = 7.68-7.62 (4H), 7.61-7.41 (16H), 7.38-7.08 (18H), 2.38 (6H).

### Example 3

### <Synthesis of 4,4"-bis{(biphenyl-4-yl)-(phenyl-d₅)amino}-1,1':4',1"-terphenyl (Compound 1-14)>

(Biphenyl-4-yl)-(phenyl-d₅)amine (25.3 g), 4,4"-diiodo-1,1':4',1"-terphenyl (20.3 g), a copper powder (0.30 g), potassium carbonate (17.5 g), 3,5-di-tert-butylsalicylic acid (1.05 g), sodium bisulfite (1.31 g), dodecylbenzene (20 ml), and toluene (30 ml) were added into a reaction vessel and heated up to 210°C while removing the toluene by distillation. After the obtained product was stirred for 23 hours, the product was cooled, and toluene (30 ml) and methanol (60 ml) were added. A precipitated solid was collected by filtration and washed with a methanol/water (1/5, v/v) mixed solution (180 ml) followed by washing with methanol (90 ml). An obtained gray powder was heated after adding 1,2-dichlorobenzene (210 ml), and insoluble matter was removed by filtration. After the filtrate was left to cool, methanol (210 ml) was added, and a precipitated crude product was collected by filtration. The crude product was washed under reflux with methanol (210 ml) to obtain a gray powder of 4,4"-bis{(biphenyl-4-yl)-(phenyl-d₅)amino}-1,1':4',1"-terphenyl (Compound 1-14; 29.3 g; yield 96%).

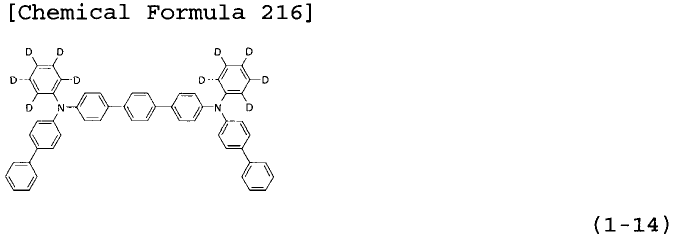

The structure of the obtained gray powder was identified by NMR.

¹H-NMR (THF-d₈) detected 30 hydrogen signals, as follows.

δ (ppm) = 7.69 (4H), 7.65-7.52 (12H), 7.39 (4H), 7.28 (2H), 7.20-7.14 (8H).

### Example 4

### <Synthesis of 4,"-bis{(naphthalen-1-yl)-phenylamino}-1,1':4',1"-terphenyl (Compound 1-2)> (not forming part of the claimed invention)

(Naphthalen-1-yl)-phenylamine (40.0 g), 4,"-diiodo-1,1':4',1"-terphenyl (43.7 g), a copper powder (0.53 g), potassium carbonate (34.4 g), 3,5-di-tert-butylsalicylic acid (2.08 g), sodium bisulfite (2.60 g), dodecylbenzene (40 ml), and xylene (40 ml) were added into a reaction vessel and heated up to 210°C while removing the xylene by distillation. After the obtained product was stirred for 35 hours, the product was cooled. Toluene (100 ml) was added, and a precipitated solid was collected by filtration. 1,2-dichlorobenzene (210 ml) was added to the obtained solid, and the solid was dissolved under heat, and after silica gel (30 g) was added, insoluble matter was removed by filtration. After the filtrate was left to cool, a precipitated crude product was collected by filtration. The crude product was washed under reflux with methanol to obtain a pale yellow powder of 4,4"-bis{(naphthalen-1-yl)-phenylamino}-1,1':4',1"-terphenyl (Compound 1-2; 21.9 g; yield 40%).

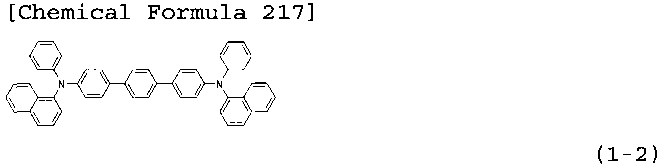

The structure of the obtained pale yellow powder was identified by NMR.

¹H-NMR (THF-d₈) detected 36 hydrogen signals, as follows.

δ (ppm) = 7.98-7.88 (4H), 7.80 (2H), 7.60 (4H), 7.52-7.40 (8H), 7.36 (4H), 7.18 (4H), 7.08-7.01 (8H), 6.93 (2H).

### Example 5

### <Synthesis of 4,"-bis{(naphthalen-2-yl)-phenylamino}-1,1':4',1"-terphenyl (Compound 1-6)> (not forming part of the claimed invention)

(Naphthalen-2-yl)-phenylamine (50.0 g), 4,"-diiodo-1,1':4',1"-terphenyl (50.0 g), tert-butoxy sodium (23.9 g), and xylene (500 ml) were added into a reaction vessel and aerated with nitrogen gas for 1 hour under ultrasonic irradiation. Palladium acetate (0.47 g) and a toluene solution (2.96 ml) containing 50% (w/v) tri-tert-butylphosphine were added, and the mixture was heated up to 120°C and stirred for 15 hours. After the mixture was left to cool, the mixture was concentrated under reduced pressure, and methanol (300 ml) was added. A precipitated solid was collected by filtration and dissolved under heat after adding 1,2-dichlorobenzene (300 ml). After silica gel (140 g) was added, insoluble matter was removed by filtration. The filtrate was concentrated under reduced pressure, and after the product was purified by recrystallization with 1,2-dichlorobenzene (250 ml), the purified product was washed under reflux with methanol to obtain a white powder of 4,4'-bis{(naphthalen-2-yl)-phenylamino}-1,1':4',1"-terphenyl (Compound 1-6; 51.0 g; yield 74%).

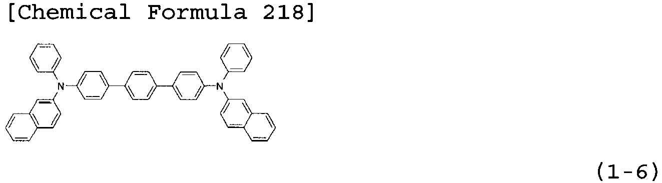

The structure of the obtained white powder was identified by NMR.

¹H-NMR (THF-d₈) detected 36 hydrogen signals, as follows.

δ (ppm) = 7.77 (4H), 7.70 (4H), 7.64-7.58 (6H), 7.48 (2H), 7.40-7.21 (10H), 7.21-7.12 (8H), 7.04 (2H).

### Example 6

### <Synthesis of 4,4"-bis[{(biphenyl-2',3',4',5',6'-d₅)-4-yl}-phenylamino]-1,1':4',1"-terphenyl (Compound 1-21)>

{(Biphenyl-2',3',4',5',6'-d₅)-4-yl}-phenylamine (24.8 g), 4,4"-diiodo-1,1':4',1"-terphenyl (19.9 g), a copper powder (0.26 g), potassium carbonate (17.2 g), 3,5-di-tert-butylsalicylic acid (2.06 g), sodium bisulfite (1.30 g), and dodecylbenzene (20 ml) were added into a reaction vessel and heated up to 215°C. After the obtained product was stirred for 21 hours, the product was cooled, and toluene (30 ml) and methanol (60 ml) were added. A precipitated solid was collected by filtration and washed with a methanol/water (1/5, v/v) mixed solution. After adding 1,2-dichlorobenzene (300 ml) to the obtained solid, the solid was heated, and insoluble matter was removed by filtration. After the filtrate was left to cool, methanol (300 ml) was added, and a precipitate was collected by filtration to obtain a yellow powder of 4,4"-bis[{(biphenyl-2',3',4',5',6'-d₅)-4-yl}-phenylamino]-1,1':4',1"-terphenyl (Compound 1-21; 25.5 g; yield 85%).

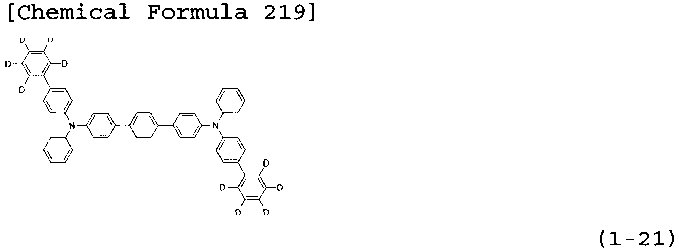

The structure of the obtained yellow powder was identified by NMR.

¹H-NMR (THF-d₈) detected 30 hydrogen signals, as follows.

δ (ppm) = 7.69 (4H), 7.65-7.52 (8H), 7.28 (4H), 7.20-7.12 (10H), 7.03 (4H).

### Example 7

### <Synthesis of 4,"-bis{(biphenyl-3-yl)- (biphenyl-4-yl)amino}-1,1':4',1"-terphenyl (Compound 1-22)> (not forming part of the claimed invention)

(Biphenyl-3-yl)-(biphenyl-4-yl)amine (16.1 g), 4,4"-diiodo-1,1':4',1"-terphenyl (11.0 g), a copper powder (0.29 g), potassium carbonate (9.46 g), 3,5-di-tert-butylsalicylic acid (1.14 g), sodium bisulfite (0.71 g), and dodecylbenzene (22 ml) were added into a reaction vessel and heated up to 220°C. After the obtained product was stirred for 34 hours, the product was cooled, and toluene and heptane were added. A precipitated solid was collected by filtration and dissolved under heat after adding 1,2-dichlorobenzene (200 ml). After silica gel (50 g) was added, insoluble matter was removed by filtration. After the filtrate was concentrated under reduced pressure, toluene and acetone were added. A precipitated solid was collected by filtration, and the precipitated solid was crystallized with 1,2-dichloromethane followed by crystallization with acetone, and further crystallized with 1,2-dichloromethane followed by crystallization with methanol to obtain a pale yellow powder of 4,4"-bis{(biphenyl-3-yl)- (biphenyl-4-yl)amino}-1,1':4',1"-terphenyl (Compound 1-22; 25.5 g; yield 77%).

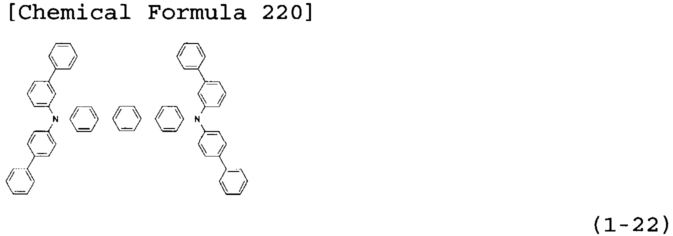

The structure of the obtained pale yellow powder was identified by NMR.

¹H-NMR (THF-d₈) detected 48 hydrogen signals, as follows.

δ (ppm) = 7.71 (4H), 7.67-7.50 (16H), 7.47 (4H), 7.43-7.20 (20H), 7.12 (4H).

### Example 8

### <Synthesis of 4,4"-bis{(phenanthren-9-yl)-phenylamino}-1,1':4',1"-terphenyl (Compound 1-3)>

(Phenanthren-9-yl)-phenylamine (16.9 g), 4,4"-diiodo-1,1':4',1"-terphenyl (12.6 g), a copper powder (0.16 g), potassium carbonate (10.9 g), 3,5-di-tert-butylsalicylic acid (0.65 g), sodium bisulfite (0.83 g), and dodecylbenzene (13 ml) were added into a reaction vessel and heated up to 210°C. After the obtained product was stirred for 23 hours, the product was cooled, and toluene (26 ml) and methanol (26 ml) were added. A precipitated solid was collected by filtration and washed with a methanol/water (1/5, v/v) mixed solution (120 ml). The precipitated solid was crystallized with 1,2-dichlorobenzene followed by crystallization with methanol to obtain a white powder of 4,4"-bis{(phenanthren-9-yl)-phenylamino)-1,1':4',1"-terphenyl (Compound 1-3; 9.38 g; yield 47%).

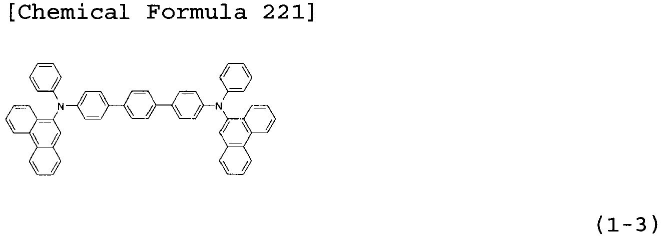

The structure of the obtained yellow powder was identified by NMR.

¹H-NMR (THF-d₈) detected 40 hydrogen signals, as follows.

δ (ppm) = 8.88-8.73 (4H), 8.09 (2H), 7.71 (2H), 7.68-7.41 (18H), 7.21-7.10 (12H), 6.92 (2H).

### Example 9

### <Synthesis of 4,4"-bis{(biphenyl-3-yl)-phenylamino}-1,1':4',1"-terphenyl (Compound 1-5)>

(Biphenyl-3-yl)-phenylamine (12.7 g), 4,4"-diiodo-1,1':4',1"-terphenyl (11.3 g), a copper powder (0.30 g), potassium carbonate (9.72 g), 3,5-di-tert-butylsalicylic acid (1.17 g), sodium bisulfite (0.73 g), and dodecylbenzene (23 ml) were added into a reaction vessel and heated up to 220°C. After the obtained product was stirred for 21 hours, the product was cooled, and after 1,2-dichlorobenzene (250 ml) and silica (30 g) were added, insoluble matter was removed by filtration. After the filtrate was concentrated under reduced pressure, heptane was added. A precipitated solid was collected by filtration, and the precipitated solid was crystallized with a 1,2-dichlorobenzene/heptane mixed solvent and further crystallized with a 1,2-dichlorobenzene/methanol mixed solvent to obtain a pale brown powder of 4,4"-bis{(biphenyl-3-yl)-phenylamino}-1,1':4',1"-terphenyl (Compound 1-5; 10.8 g; yield 64%).

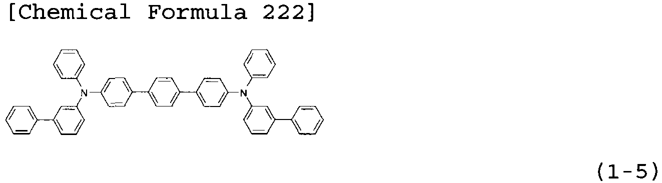

The structure of the obtained pale brown powder was identified by NMR.

¹H-NMR (THF-d₈) detected 40 hydrogen signals, as follows.

δ (ppm) = 7.69 (4H), 7.60 (4H), 7.52 (4H), 7.42-7.21 (16H), 7.20-7.13 (8H), 7.10-7.00 (4H).

### Example 10

### <Synthesis of 4,4"-bis{(triphenylen-2-yl)-phenylamino}-1,1':4',1"-terphenyl (Compound 1-23)>

(Triphenylen-2-yl)-phenylamine (11.9 g), 4,4"-diiodo-1,1':4',1"-terphenyl (8.55 g), tert-butoxy sodium (4.09 g), and xylene (86 ml) were added into a reaction vessel and aerated with nitrogen gas for 40 minutes under ultrasonic irradiation. Palladium acetate (0.08 g) and a toluene solution (0.55 ml) containing 50% (w/v) tri-tert-butylphosphine were added, and the mixture was heated up to 100°C. After the mixture was stirred for 7 hours, the mixture was cooled. Methanol (80 ml) was added, and a precipitated solid was collected by filtration. 1,2-dichlorobenzene (300 ml) was added to the obtained solid, and the solid was heated, and after silica gel (45 g) was added, insoluble matter was removed by filtration. The filtrate was concentrated under reduced pressure, and after purified by recrystallization with 1,2-dichlorobenzene, the purified product was washed under reflux with methanol to obtain a pale yellowish green powder of 4,4"-bis{(triphenylen-2-yl)-phenylamino)-1,1':4',1"-terphenyl (Compound 1-23; 11.4 g; yield 74%).

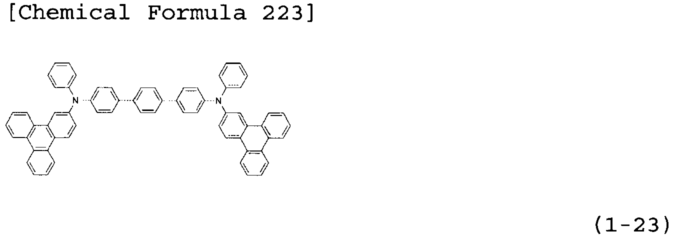

The structure of the obtained pale yellowish green powder was identified by NMR.

¹H-NMR (THF-d₈) detected 44 hydrogen signals, as follows.

δ (ppm) = 8.72-8.62 (8H), 8.45 (2H), 8.36 (2H), 7.75 (4H), 7.70-7.21 (26H), 7.09 (2H).

### Example 11

### <Synthesis of 4,4"-bis{di(naphthalen-2-yl)aminol-1,1':4',1"-terphenyl (Compound 1-24) > (not forming part of the claimed invention)

Di(naphthalen-2-yl)amine (12.2 g), 4,4''-diiodo-1,1' :4',1"-terphenyl (9.49 g), a copper powder (0.14 g), potassium carbonate (8.2 g), 3,5-di-tert-butylsalicylic acid (0.51 g), sodium bisulfite (0.69 g), dodecylbenzene (15 ml), and toluene (20 ml) were added into a reaction vessel and heated up to 210°C while removing the toluene by distillation. After the obtained product was stirred for 28 hours, the product was cooled, and 1,2-dichlorobenzene (20 ml) and methanol (20 ml) were added. A precipitated solid was collected by filtration and washed with a methanol/water (1/4, v/v) mixed solution (200 ml). Then, the solid was dissolved under heat after adding 1,2-dichlorobenzene (100 ml), and after silica gel was added, insoluble matter was removed by filtration. After the filtrate was left to cool, methanol (250 ml) was added, and a precipitated solid was collected by filtration. The precipitated solid was crystallized with a 1,2-dichlorobenzene/methanol mixed solvent followed by washing under reflux with methanol to obtain a yellowish white powder of 4,4"-bis{di(naphthalen-2-yl)amino}-1,1':4',1"-terphenyl (Compound 1-24; 10.5 g; yield 70%).

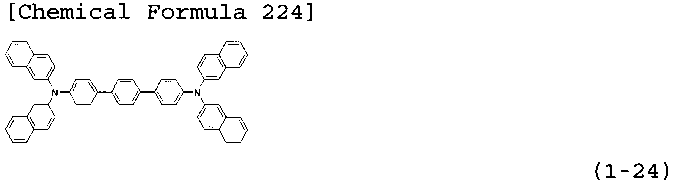

The structure of the obtained yellowish white powder was identified by NMR.

¹H-NMR (THF-d₈) detected 40 hydrogen signals, as follows.

δ (ppm) = 7.82-7.75 (6H), 7.72 (4H), 7.68-7.60 (8H), 7.56 (4H), 7.40-7.30 (14H), 7.24 (4H).

### Example 12

### <Synthesis of 4,4"-bis[14-(naphthalen-2-yl)phenyl}-phenylamino]-1,1':4',1"-terphenyl (Compound 1-25)>

{4-(Naphthalen-2-yl)phenyl}-phenylamine (16.6 g), 4,4"-diiodo-1,1':4',1"-terphenyl (11.8 g), a copper powder (0.18 g), potassium carbonate (10.5 g), 3,5-di-tert-butylsalicylic acid (0.61 g), sodium bisulfite (0.83 g), dodecylbenzene (15 ml), and toluene (20 ml) were added into a reaction vessel and heated up to 210°C while removing the toluene by distillation. After the obtained product was stirred for 19 hours, the product was cooled, and toluene (20 ml) and methanol (20 ml) were added. A precipitated solid was collected by filtration, washed with a methanol/water (1/4, v/v) mixed solution (180 ml), and further washed with methanol (100 ml). An obtained brownish yellow powder was heated after adding 1,2-dichlorobenzene (175 ml), and insoluble matter was removed by filtration. After the filtrate was left to cool, methanol (200 ml) was added, and a precipitated solid was collected by filtration. The precipitated solid was crystallized with a 1,2-dichlorobenzene/methanol mixed solvent followed by washing under reflux with methanol to obtain a brownish white powder of 4,4"'-bis[{4-(naphthalen-2-yl)phenyl}-phenylamino]-1,1':4',1"-terphenyl (Compound 1-25; 11.9 g; yield 53%).

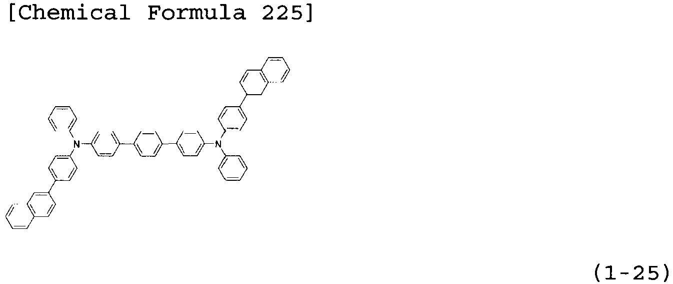

The structure of the obtained brownish white powder was identified by NMR.

¹H-NMR (THF-d₈) detected 44 hydrogen signals, as follows.

δ (ppm) = 8.10 (2H), 7.93-7.78 (8H), 7.76-7.70 (8H), 7.62 (4H), 7.44 (4H), 7.30 (4H), 7.25-7.16 (12H), 7.05 (2H).

### Example 13

### <Synthesis of 4-{(biphenyl-4-yl)-phenylamino}-4"-[{4-(1-phenyl-indol-4-yl)phenyl}-phenylamino]-1,1':4',1"-terphenyl (Compound 1-26)>

(4'-Bromo-1,1'-biphenyl-4-yl)-{4-(1-phenyl-indol-4 - yl)phenyl}-phenylamine (7.25 g), {4-(4,4,5,5-tetramethyl-1,3,2-dioxabororan-2-yl)phenyl}-(1,1'-biphenyl-4-yl)-phenylamine (5.76 g), a 2 M potassium carbonate aqueous solution (12.3 ml), toluene (80 ml), and ethanol (20 ml) were added into a reaction vessel and aerated with nitrogen gas for 40 minutes under ultrasonic irradiation. After adding tetrakistriphenylphosphinepalladium (0.43 g), the mixture was heated and refluxed for 7 hours while being stirred. After the mixture was left to cool, water (50 ml) and toluene (100 ml) were added, and insoluble matter was removed by filtration. An organic layer was collected by liquid separation, then dried over anhydrous magnesium sulfate and concentrated under reduced pressure to obtain a crude product. After the crude product was purified by column chromatography (support: silica gel, eluent: toluene/heptane), the purified product was crystallized with THF followed by crystallization with methanol to obtain a pale yellow powder of 4-{(biphenyl-4-yl)-phenylamino}-4"-[{4-(1-phenyl-indol-4-yl)phenyl}-phenylamino]-1,1':4',1"-terphenyl (Compound 1-26; 6.80 g; yield 67%).

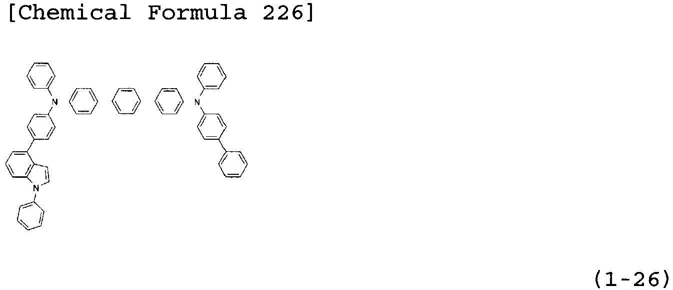

The structure of the obtained pale yellow powder was identified by NMR.

¹H-NMR (THF-d₈) detected 45 hydrogen signals, as follows.

δ (ppm) = 7.70 (4H), 7.68-7.50 (16H), 7.42-7.11 (23H), 7.05 (1H), 6.88 (1H).

### Example 14

### <Synthesis of 4,"-bis{(biphenyl-4-yl)-phenylamino}-1,1':3',1"-terphenyl (Compound 1-27)>

3-Bromoiodobenzene (8.83 g), (biphenyl-4-yl)-{4-(4,4,5,5-tetramethyl-[1,3,2]dioxaborolan-2-yl)phenyl}-phenylamine (30.5 g), potassium carbonate (13.0 g), water (30 ml), toluene (300 ml), and ethanol (75 ml) were added into a nitrogen-substituted reaction vessel and aerated with nitrogen gas under ultrasonic irradiation for 30 minutes. The mixture was heated after adding tetrakis(triphenylphosphine)palladium (1.1 g), and stirred at 80°C for 16 hours. The mixture was cooled to a room temperature, and methanol (300 ml) was added. A precipitated solid was collected by filtration, and the solid was dissolved under heat after adding 1,2-dichlorobenzene (270 ml). Silica gel (16 g) was added, and the mixture was stirred for 30 minutes. After insoluble matter was removed by filtration, a crude product precipitated by adding methanol (300 ml) was collected by filtration. The crude product was washed under reflux with methanol (200 ml) to obtain a white powder of 4,4"-bis{(biphenyl-4-yl)-phenylamino}-1,1':3',1"-terphenyl (Compound 1-27; 14.3 g; yield 71%).

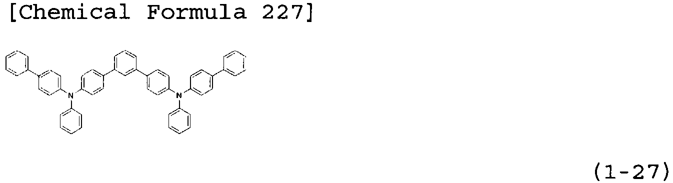

The structure of the obtained white powder was identified by NMR.

¹H-NMR (CDCl₃) detected 40 hydrogen signals, as follows.

δ (ppm) = 7.87 (1H), 7.64-7.50 (12H), 7.48-7.32 (6H), 7.31-6.98 (21H).

### Example 15

### <Synthesis of 4,4"-bis{(biphenyl-4-yl)-(phenyl-d₅)amino}-1,1':3',1"-terphenyl (Compound 1-28)>

1,3-dibromobenzene (6.51 g), (biphenyl-4-yl)-{4-(4,4,5,5-tetramethyl-[1,3,2]dioxaborolan-2-yl)phenyl}-(phenyl-d₅)amine (26.9 g), potassium carbonate (11.4 g), water (50 ml), toluene (200 ml), and ethanol (50 ml) were added into a nitrogen-substituted reaction vessel and aerated with nitrogen gas under ultrasonic irradiation for 30 minutes. The mixture was heated after adding tetrakis(triphenylphosphine)palladium (0.95 g), and stirred at 70°C for 12 hours. The mixture was cooled to a room temperature, and methanol (200 ml) was added. A precipitated solid was collected by filtration, and the solid was dissolved under heat after adding 1,2-dichlorobenzene (400 ml). Silica gel (20 g) was added, and the mixture was stirred for 30 minutes. After insoluble matter was removed by filtration, a precipitate formed by adding methanol (500 ml) was collected by filtration. The precipitate was dissolved by adding 1,2-dichlorobenzene (100 ml), and a crude product precipitated by adding toluene (100 ml) and methanol (100 ml) was collected by filtration. The crude product was washed under reflux with methanol (250 ml) to obtain a white powder of 4,4"-bis{(biphenyl-4-yl)-(phenyl-d₅)amino}-1,1':3',1"-terphenyl (Compound 1-28; 18.3 g; yield 91%).

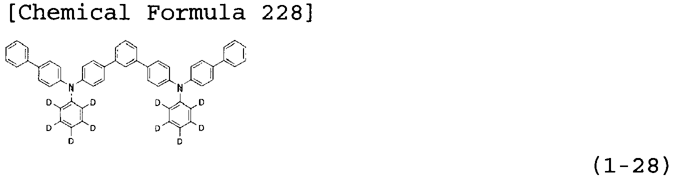

The structure of the obtained white powder was identified by NMR.

¹H-NMR (CDCl₃) detected 30 hydrogen signals, as follows.

δ (ppm) = 7.87 (1H), 7.64-7.32 (18H), 7.31-6.98 (11H).

### Example 16

### <Synthesis of 4,"-bis{(naphthalen-1-yl)-phenylamino}-1,1':3',1"-terphenyl (Compound 1-29)> (not forming part of the claimed invention)

The reaction was carried out under the same conditions as those of Example 15, except that (biphenyl-4-yl)-{4-(4,4,5,5-tetramethyl-[1,3,2]dioxaborolan-2-yl)phenyl}-(phenyl-d₅)amine was replaced with (naphthalen-1-yl)-{4-(4,4,5,5-tetramethyl-[1,3,2]dioxaborolan-2-yl)phenyl}-phenylamine. As a result, a white powder of 4,4"-bis{(naphthalen-1-yl)-phenylamino}-1,1':3',1"-terphenyl (Compound 1-29; 8.8 g; yield 59%) was obtained.

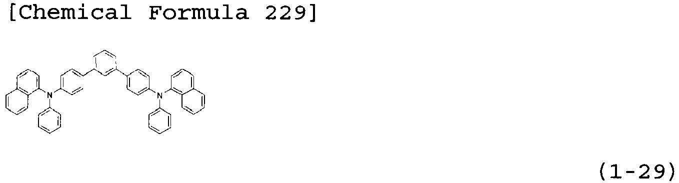

The structure of the obtained white powder was identified by NMR.

¹H-NMR (CDCl₃) detected 36 hydrogen signals, as follows.

δ (ppm) = 7.99 (2H), 7.92 (2H), 7.81 (2H), 7.72 (1H), 7.57-6.92 (29H).

### Example 17

### <Synthesis of 4,4-bis[{4-(dibenzofuran-4-yl)phenyl)-phenylaminol-1,1':3',1"-terphenyl (Compound 1-32)>

The reaction was carried out under the same conditions as those of Example 15, except that (biphenyl-4-yl)-{4-(4,4,5,5-tetramethyl-[1,3,2]dioxaborolan-2-yl)phenyl}-(phenyl-d₅)amine was replaced with {4-(dibenzofuran-4-yl)phenyl}-{4-(4,4,5,5-tetramethyl-[1,3,2]dioxaborolan-2-yl)phenyl}-phenylamine. As a result, a white powder of 4,4"-bis[{4-(dibenzofuran-4-yl)phenyl}-phenylamino]-1,1':3',1"-terphenyl (Compound 1-32; 6.8 g; yield 86%) was obtained.

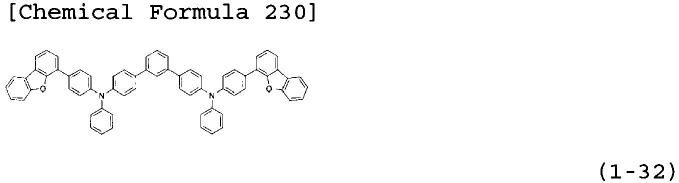

The structure of the obtained white powder was identified by NMR.

¹H-NMR (CDCl₃) detected 44 hydrogen signals, as follows.

δ (ppm) = 8.01 (2H), 7.97-7.82 (8H), 7.67-7.24 (34H).

### Example 18

### <Synthesis of 2,4"-bis{(biphenyl-4-yl)-phenylamino}-1,1':4',1"-terphenyl (Compound 1-50)>

4-Bromo-4'-{(biphenyl-4-yl)-phenylamino}-biphenyl (16.8 g), (biphenyl-4-yl)-{2-(4,4,5,5-tetramethyl-[1,3,2]dioxaborolan-2-yl)phenyl}-phenylamine (19.0 g), potassium carbonate (7.4 g), water (26 ml), toluene (200 ml), and ethanol (50 ml) were added into a nitrogen-substituted reaction vessel and aerated with nitrogen gas under ultrasonic irradiation for 30 minutes. After adding tetrakis(triphenylphosphine)palladium (0.87 g), the mixture was heated and refluxed for 20 hours while being stirred. After the mixture was cooled to a room temperature, an organic layer was collected by liquid separation, then dried over anhydrous magnesium sulfate and concentrated to obtain a crude product. After the crude product was purified by column chromatography (support: silica gel, eluent: heptane/toluene), the purified product was crystallized with an ethyl acetate/methanol mixed solvent to obtain a white powder of 2,4"-bis{(biphenyl-4-yl)-phenylamino}-1,1':4',1"-terphenyl (Compound 1-50; 20.8 g; yield 82%).

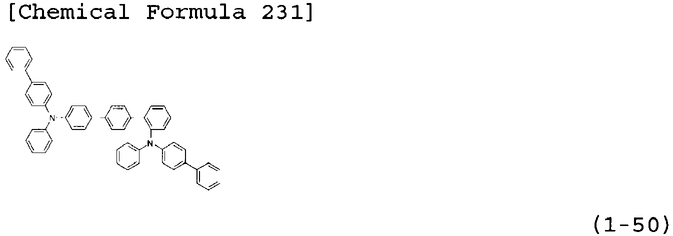

The structure of the obtained white powder was identified by NMR.

¹H-NMR (CDCl₃) detected 40 hydrogen signals, as follows.

δ (ppm) = 7.61 (2H), 7.56-6.83 (38H).

### Example 19

### <Synthesis of 4,4"-bis{(triphenylen-2-yl)-phenylamino}-1,1':3',1"-terphenyl (Compound 1-51)>

4,"-Dibromo-1,1':3',1"-terphenyl (8.2 g), (triphenylen-2-yl)-phenylamine (15.4 g), tert-butoxy sodium (5.1 g), and toluene (180 ml) were added into a nitrogen-substituted reaction vessel and aerated with nitrogen gas under ultrasonic irradiation for 30 minutes. Palladium acetate (0.11 g) and a toluene solution (0.31 ml) containing 50% (w/v) tri-tert-butylphosphine were added, and the mixture was heated and refluxed for 5 hours while being stirred.

The mixture was cooled to a room temperature and subjected to an extraction procedure using 1,2-dichlorobenzene and then to purification by adsorption with a silica gel, followed by crystallization with a 1,2-dichlorobenzene/methanol mixed solvent to obtain a yellowish white powder of 4,4-bis{(triphenylen-2-yl)-phenylamino}-1,1':3',1"-terphenyl (Compound 1-51; 11.67 g; yield 64%).

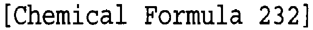

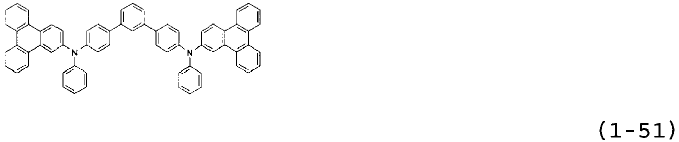

The structure of the obtained yellowish white powder was identified by NMR.

¹H-NMR (CDCl₃) detected 44 hydrogen signals, as follows.

δ (ppm) = 8.67 (4H), 8.57 (4H), 8.41 (2H), 8.36(2H), 7.88 (1H), 7.70-7.10 (31H).

### Example 20

### <Synthesis of 4,4-bis{(phenanthren-9-yl)-phenylamino}-1,1':3',1"-terphenyl (Compound 1-52)>

The reaction was carried out under the same conditions as those of Example 19, except that (triphenylen-2-yl)-phenylamine was replaced with (phenanthren-9-yl)-phenylamine. As a result, a yellowish white powder of 4,4"-bis{(phenanthren-9-yl)-phenylamino)-1,1':3',1"-terphenyl (Compound 1-52; 8.0 g; yield 50%) was obtained.

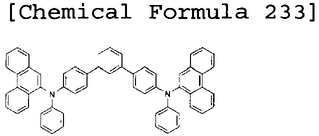

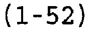

The structure of the obtained yellowish white powder was identified by NMR.

¹H-NMR (CDCl₃) detected 40 hydrogen signals, as follows.

δ (ppm) = 8.81-8.71 (4H), 8.10 (2H), 7.83-7.39 (20H), 7.29-6.97 (14H).

### Example 21

### <Synthesis of 4-{bis(biphenyl-4-yl)amino}-2"-{(biphenyl-4-yl)-phenylamino}-1,1':4',1"-terphenyl (Compound 1-53)>

2-{(biphenyl-4-yl)-phenylamino}-4"-bromo-1,1':4',1"-terphenyl (12.1 g), bis(biphenyl-4-yl)amine (8.0 g), tris(dibenzylideneacetone)palladium (0.6 g), tri-tert-butylphosphine (0.22 g), and tert-butoxy sodium (6.3 g) were added into a nitrogen-substituted reaction vessel, heated and refluxed for 3 hours while being stirred. After the mixture was cooled to a room temperature, methanol (600 ml) was added, and a precipitated crude product was collected by filtration. The crude product was dissolved in toluene, and after insoluble matter was removed by filtration, purification by crystallization with methanol was carried out. Then, recrystallization with a THF/methanol mixed solvent was carried out to obtain a white powder of 4-{bis(biphenyl-4-yl)amino}-2"-{(biphenyl-4-yl)-phenylamino}-1,1':4',1"-terphenyl (Compound 1-53; 15 g; yield 87%).

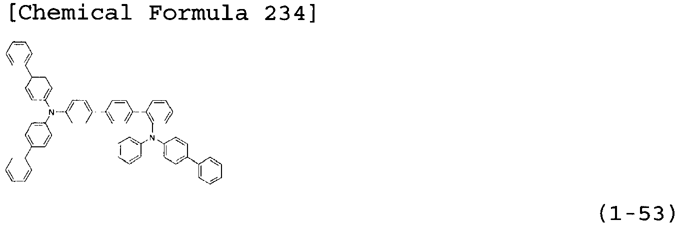

The structure of the obtained white powder was identified by NMR.

¹H-NMR (CDCl₃) detected 44 hydrogen signals, as follows.

δ (ppm) = 7.62 (4H), 7.58-6.91 (38H), 6.87 (2H).

### Example 22

### <Synthesis of 4,4"-bis{(naphthalen-1-yl)-(phenyl-d₅)amino}-1,1':3',1"-terphenyl (Compound 1-54)> (not forming part of the claimed invention)

The reaction was carried out under the same conditions as those of Example 15, except that (biphenyl-4-yl)-{4-(4,4,5,5-tetramethyl-[1,3,2]dioxaborolan-2-yl)phenyl}-(phenyl-d₅)amine was replaced with (naphthalen-1-yl)-{4-(4,4,5,5-tetramethyl-[1,3,2]dioxaborolan-2-yl)phenyl}-(phenyl-d₅)amine. As a result, a white powder of 4,4"-bis{(naphthalen-1-yl)-(phenyl-d₅)amino}-1,1':3',1"-terphenyl (Compound 1-54; 5.2 g; yield 30%) was obtained.

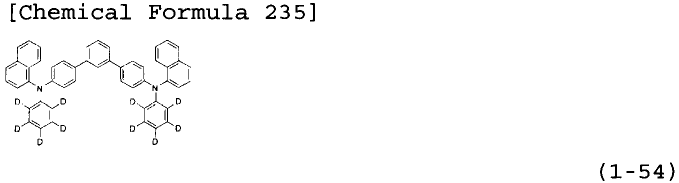

The structure of the obtained white powder was identified by NMR.

¹H-NMR (CDCl₃) detected 26 hydrogen signals, as follows.

δ (ppm) = 7.99 (2H), 7.92 (2H), 7.81 (2H), 7.72 (1H), 7.55-7.36 (15H), 7.13-7.07 (4H).

### Example 23

### <Synthesis of 2-{bis(biphenyl-4-yl)amino}-4"-{(biphenyl-4-yl)-phenylamino}-1,1':4',1"-terphenyl (Compound 1-56)>

The reaction was carried out under the same conditions as those of Example 18, except that (biphenyl-4-yl)-(2-(4,4,5,5-tetramethyl-[1,3,2]dioxaborolan-2-yl)phenyl}-phenylamine was replaced with bis(biphenyl-4-yl)-{2-(4,4,5,5-tetramethyl-[1,3,2]dioxaborolan-2-yl)phenyl}amine. As a result, a white powder of 2-{bis(biphenyl-4-yl)amino}-4"-{(biphenyl-4-yl)-phenylamino}-1,1':4',1"-terphenyl (Compound 1-56; 15.7 g; yield 94%) was obtained.

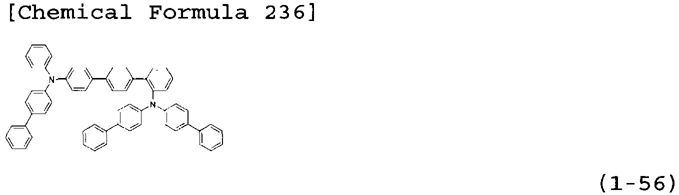

The structure of the obtained white powder was identified by NMR.

¹H-NMR (CDCl₃) detected 44 hydrogen signals, as follows.

δ (ppm) = 7.60 (2H), 7.56-6.97 (42H).

### Example 24

### <Synthesis of 2,4"-bis{bis(biphenyl-4-yl)amino}-1,1':4',1"-terphenyl (Compound 1-57) > (not forming part of the claimed invention)

The reaction was carried out under the same conditions as those of Example 18, except that 4-bromo-4'-{(biphenyl-4-yl)-phenylamino}-biphenyl was replaced with 4-bromo-4'-{bis(biphenyl-4-yl)amino}-biphenyl, and (biphenyl-4-yl)-{2-(4,4,5,5-tetramethyl-[1,3,2]dioxaborolan-2-yl)phenyl}-phenylamine was replaced with 2-{bis(biphenyl-4-yl)amino}phenylboronic acid. As a result, a white powder of 2,4"-bis(bis(biphenyl-4-yl)amino}-1,1':4',1"-terphenyl (Compound 1-57; 12 g; yield 76%) was obtained.

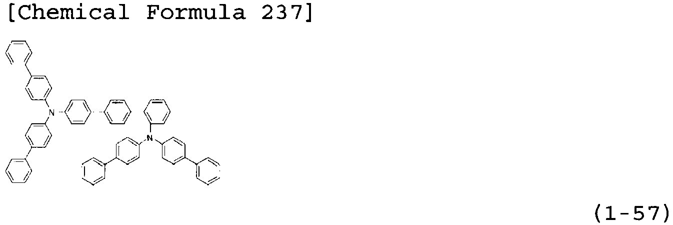

The structure of the obtained white powder was identified by NMR.

¹H-NMR (CDCl₃) detected 48 hydrogen signals, as follows.

δ (ppm) = 7.65-6.98 (48H).

### Example 25

### <Synthesis of 4,4"-bis{(biphenyl-4-yl)-(naphthalen-1-yl)amino}-1,1':3',1"-terphenyl (Compound 1-59) > (not forming part of the claimed invention)

The reaction was carried out under the same conditions as those of Example 19, except that (triphenylen-2-yl)-phenylamine was replaced with (biphenyl-4-yl)-(naphthalen-1-yl)amine. As a result, a white powder of 4,4"-bis{(biphenyl-4-yl)-(naphthalen-1-yl)amino}-1,1':3',1"-terphenyl (Compound 1-59; 6.4 g; yield 36%) was obtained.

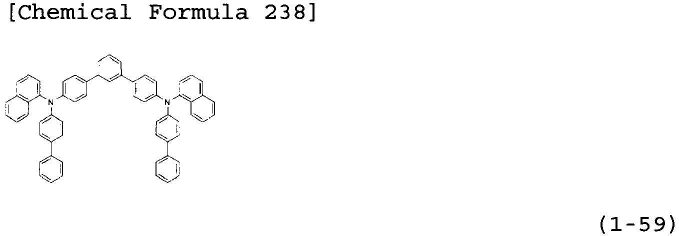

The structure of the obtained white powder was identified by NMR.

¹H-NMR (CDCl₃) detected 44 hydrogen signals, as follows.

δ (ppm) = 8.02 (2H), 7.94 (2H), 7.84 (2H), 7.76 (1H), 7.62-7.38 (27H), 7.33 (2H), 7.19-7.13 (8H).

### Example 26

### <Synthesis of 4,4"-bis{(9,9-dimethyl-9H-fluoren-2-yl)-phenylamino}-1,1':3',1"-terphenyl (Compound 1-60)>

The reaction was carried out under the same conditions as those of Example 19, except that (triphenylen-2-yl)-phenylamine was replaced with (9,9-dimethyl-9H-fluoren-2-yl)-phenylamine. As a result, a white powder of 4,4"-bis{(9,9-dimethyl-9H-fluoren-2-yl)-phenylamino}-1,1':3',1"-terphenyl (Compound 1-60; 14.6 g; yield 80%) was obtained.

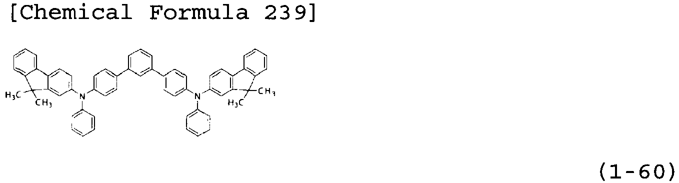

The structure of the obtained white powder was identified by NMR.

¹H-NMR (CDCl₃) detected 48 hydrogen signals, as follows.

δ (ppm) = 7.84 (1H), 7.70-7.03 (35H), 1.48 (12H).

### Example 27

### <Synthesis of 2-{bis(biphenyl-4-yl)amino}-4"-{(naphthalen-1-yl)-phenylamino}-1,1':4',1"-terphenyl (Compound 1-62)>

The reaction was carried out under the same conditions as those of Example 18, except that 4-bromo-4'-{(biphenyl-4-yl)-phenylamino}-biphenyl was replaced with 4-bromo-4'-{(naphthalen-1-yl)-phenylamino}-biphenyl, and (biphenyl-4-yl)-{2-(4,4,5,5-tetramethyl-[1,3,2]dioxaborolan-2-yl)phenyl}-phenylamine was replaced with 2-{bis(biphenyl-4-yl)amino}phenylboronic acid. As a result, a white powder of 2-{bis(biphenyl-4-yl)amino}-4"-{(naphthalen-1-yl)-phenylamino}-1,1':4',1"-terphenyl (Compound 1-62; 12.8 g; yield 75%) was obtained.

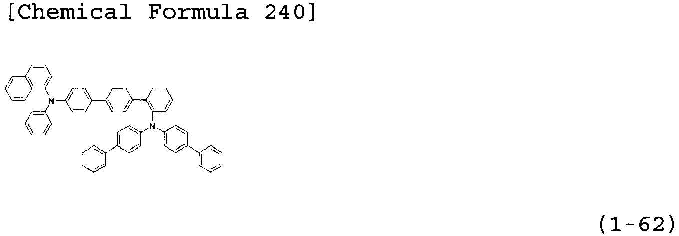

The structure of the obtained white powder was identified by NMR.

¹H-NMR (CDCl₃) detected 42 hydrogen signals, as follows.

δ (ppm) = 7.99 (2H), 7.93 (2H), 7.81 (2H), 7.57-6.96 (36H).

### Example 28

### <Synthesis of 2-{(biphenyl-4-yl)-phenylamino}-4"-{(9,9-dimethyl-9H-fluoren-2-yl)-phenylamino}-1,1':4',1"-terphenyl (Compound 1-63)>

The reaction was carried out under the same conditions as those of Example 18, except that 4-bromo-4'-{(biphenyl-4-yl)-phenylamino}-biphenyl was replaced with 4-bromo-4'-{(9,9-dimethyl-9H-fluoren-2-yl)-phenylamino}-biphenyl. As a result, a white powder of 2-{(biphenyl-4-yl)-phenylamino}-4"-{(9,9-dimethyl-9H-fluoren-2-yl)-phenylamino}-1,1':4',1"-terphenyl (Compound 1-63; 11.7 g; yield 73%) was obtained.

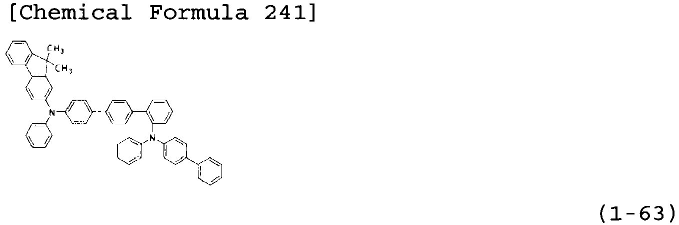

The structure of the obtained white powder was identified by NMR.

¹H-NMR (CDCl₃) detected 44 hydrogen signals, as follows.

δ (ppm) = 7.68 (1H), 7.64-6.84 (37H), 1.48 (6H).

### Example 29

### <Synthesis of 4,4"-bis{(biphenyl-4-yl)-(naphthalen-1-yl)amino}-1,1':2',1"-terphenyl (Compound 1-67)> (not forming part of the claimed invention)

The reaction was carried out under the same conditions as those of Example 19, except that 4,4"-dibromo-1,1':3',1"-terphenyl was replaced with 4,4"-dibromo-1,1':2',1"-terphenyl, and (triphenylen-2-yl)-phenylamine was replaced with (biphenyl-4-yl)-(naphthalen-1-yl)amine. As a result, a white powder of 4,4"-bis{(biphenyl-4-yl)-(naphthalen-1-yl)amino}-1,1':2',1"-terphenyl (Compound 1-67; 5.0 g; yield 30%) was obtained.

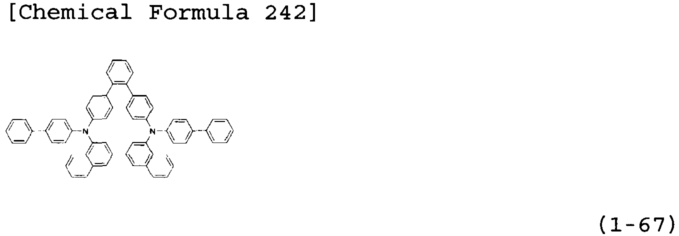

The structure of the obtained white powder was identified by NMR.

¹H-NMR (CDCl₃) detected 44 hydrogen signals, as follows.

δ (ppm) = 7.93-7.84 (4H), 7.79 (2H), 7.60-7.26 (24H), 7.25-6.92 (14H).

### Example 30

### <Synthesis of 4,4"-bis[{4-(naphthalen-1-yl)phenyl}-phenylamino]-1,1':2',1"-terphenyl (Compound 1-68)>

The reaction was carried out under the same conditions as those of Example 19, except that 4,4"-dibromo-1,1'3',1"-terphenyl was replaced with 4,4"-dibromo-1,1:2',1"-terphenyl, and (triphenylen-2-yl)-phenylamine was replaced with {4-(naphthalen-1-yl)phenyl}-phenylamine. As a result, a white powder of 4,4"-bis[{4-(naphthalen-1-yl)phenyl}-phenylamino]-1,1':2',1"-terphenyl (Compound 1-68; 7.3 g; yield 43%) was obtained.

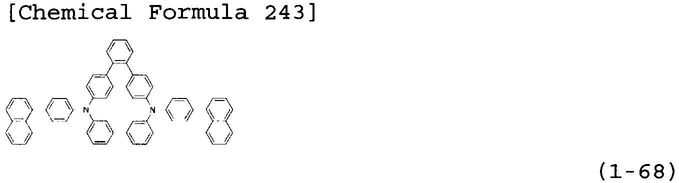

The structure of the obtained white powder was identified by NMR.

¹H-NMR (CDCl₃) detected 44 hydrogen signals, as follows.

δ (ppm) = 8.01 (2H), 7.91 (2H), 7.84 (2H), 7.53-6.98 (38H) .

### Example 31

### <Synthesis of 2,2"-bis[{4-(naphthalen-1-yl)phenyl}-phenylamino]-1,1':3',1"-terphenyl (Compound 1-69)>

The reaction was carried out under the same conditions as those of Example 14, except that 3-bromoiodobenzene was replaced with 1,3-diiodobenzene, and (biphenyl-4-yl)-{4-(4,4,5,5-tetramethyl-[1,3,2]dioxaborolan-2-yl)phenyl}-phenylamine was replaced with 2-[{4-(naphthalen-1-yl)phenyl}-phenylamino]-phenylboronic acid. As a result, a white powder of 2,2"-bis[{4-(naphthalen-1-yl)phenyl}-phenylamino]-1,1':3',1"-terphenyl (Compound 1-69; 7.3 g; yield 43%) was obtained.

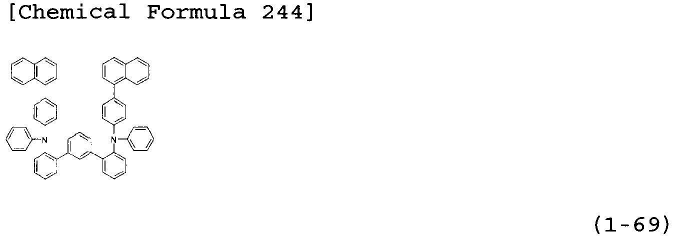

The structure of the obtained white powder was identified by NMR.

¹H-NMR (CDCl₃) detected 44 hydrogen signals, as follows.

δ (ppm) = 7.94-6.85 (44H).

### Example 32

### <Synthesis of 4,4"-bis[{4-(naphthalen-1-yl)phenyl}-phenylamino]-1,1':3',1"-terphenyl (Compound 1-71)>

The reaction was carried out under the same conditions as those of Example 19, except that (triphenylen-2-yl)-phenylamine was replaced with {4-(naphthalen-1-yl)phenyl}-phenylamine. As a result, a white powder of 4,4"-bis[{4-(naphthalen-1-yl)phenyl)-phenylamino]-1,1':3',1"-terphenyl (Compound 1-71; 16.7 g; yield 79%) was obtained.

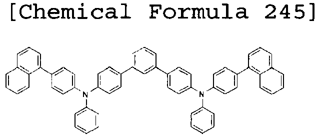

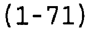

The structure of the obtained white powder was identified by NMR.

¹H-NMR (CDCl₃) detected 44 hydrogen signals, as follows.

δ (ppm) = 8.08 (2H), 7.94 (2H), 7.90-7.80 (3H), 7.65-7.00 (37H).

### Example 33

### <Synthesis of 2,2"-bis{(9,9-dimethyl-9H-fluoren-2-yl)-phenylamino}-1,1':4',1"-terphenyl (Compound 1-75)>

The reaction was carried out under the same conditions as those of Example 15, except that 1,3-dibromobenzene was replaced with 1,4-dibromobenzene, and (biphenyl-4-yl)-{4-(4,4,5,5-tetramethyl-[1,3,2]dioxaborolan-2-yl)phenyl}-(phenyl-d₅)amine was replaced with 2-{(9,9-dimethyl-9H-fluoren-2-yl)-phenylamino}-phenylboronic acid. As a result, a white powder of 2,2"-bis{(9,9-dimethyl-9H-fluoren-2-yl)-phenylamino}-1,1':4',1"-terphenyl (Compound 1-75; 13.7 g; yield 76%) was obtained.

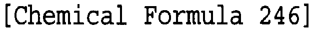

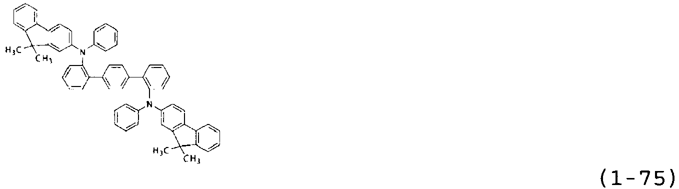

The structure of the obtained white powder was identified by NMR.

¹H-NMR (THF-d₈) detected 48 hydrogen signals, as follows.

δ (ppm) = 7.53 (2H), 7.35-6.81 (30H), 6.76 (2H), 6.67 (2H), 1.29 (12H).

### Example 34

### <Synthesis of 2,2"-bis{bis(biphenyl-4-yl)amino}-1,1':4',1"-terphenyl (Compound 1-76)> (not forming part of the claimed invention)

The reaction was carried out under the same conditions as those of Example 15, except that 1,3-dibromobenzene was replaced with 1,4-dibromobenzene, and (biphenyl-4-yl)-{4-(4,4,5,5-tetramethyl-[1,3,2]dioxaborolan-2-yl)phenyl}-(phenyl-d₅)amine was replaced with 2-{bis(biphenyl-4-yl)amino}-phenylboronic acid. As a result, a white powder of 2,2"-bis{bis(biphenyl-4-yl)amino}-1,1':4',1"-terphenyl (Compound 1-76; 15.7 g; yield 78%) was obtained.

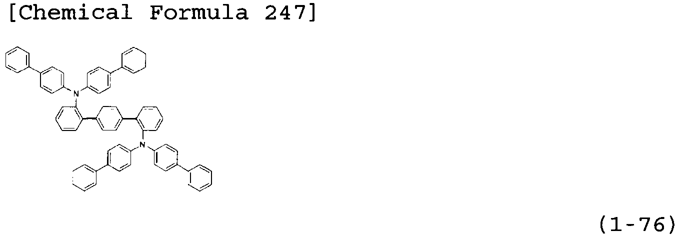

The structure of the obtained white powder was identified by NMR.

¹H-NMR (THF-d₈) detected 48 hydrogen signals, as follows.

δ (ppm) = 7.51-7.45 (8H), 7.33-7.18 (28H), 7.00 (4H), 6.90-6.82 (8H).

### Example 35

### <Synthesis of 2-{(9,9-dimethyl-9H-fluoren-2-yl)-phenylamino}-2"-[(4-(naphthalen-1-yl)phenyl)-phenylamino]-1,1':4',1"-terphenyl (Compound 1-81)>

The reaction was carried out under the same conditions as those of Example 18, except that 4-bromo-4'-{(biphenyl-4-yl)-phenylamino}-biphenyl was replaced with 4-bromo-2'-{4-(naphthalen-1-yl)phenyl}-phenylamino}-biphenyl, and (biphenyl-4-yl)-{2-(4,4,5,5-tetramethyl-[1,3,2]dioxaborolan-2-yl)phenyl}-phenylamine was replaced with 2-{(9,9-dimethyl-9H-fluoren-2-yl)-phenylamino}-phenylboronic acid. As a result, a white powder of 2-{(9,9-dimethyl-9H-fluoren-2-yl)-phenylamino}-2"-[{4-(naphthalen-1-yl)phenyl}-phenylamino]-1,1':4',1"-terphenyl (Compound 1-81; 7.3 g; yield 48%) was obtained.

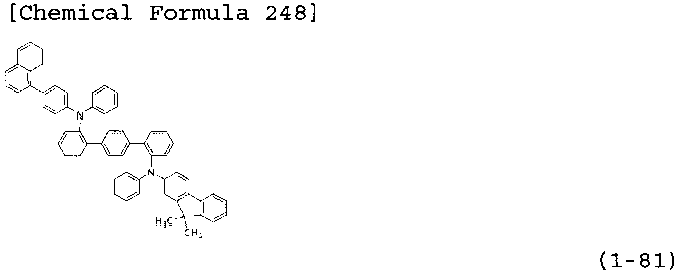

The structure of the obtained white powder was identified by NMR.

¹H-NMR (THF-d₈) detected 46 hydrogen signals, as follows.

δ (ppm) = 7.89-7.76 (3H), 7.55-6.69 (37H), 1.29 (6H).

### Example 36

The melting points and the glass transition points of the following arylamine compounds were measured using a high-sensitive differential scanning calorimeter (DSC3100SA produced by Bruker AXS).

| | Melting point | Glass transition point |
|---|---|---|
| Compound of Example 1 | 263°C | 111°C |
| Compound of Example 2 | 210°C | 113°C |
| Compound of Example 3 | 265°C | 111°C |
| Compound of Example 4 | 279°C | 107°C |
| Compound of Example 5 | 266°C | 104°C |
| Compound of Example 6 | 263°C | 111°C |
| Compound of Example 7 | 262°C | 117°C |
| Compound of Example 8 | 303°C | 149°C |
| Compound of Example 10 | 365°C | 163°C |
| Compound of Example 11 | 289°C | 138°C |
| Compound of Example 13 | not observed | 125°C |
| Compound of Example 14 | 252°C | 108°C |
| Compound of Example 15 | 252°C | 108°C |
| Compound of Example 16 | not observed | 106°C |
| Compound of Example 17 | not observed | 135°C |
| Compound of Example 18 | not observed | 107°C |
| Compound of Example 19 | 323°C | 159°C |
| Compound of Example 20 | 290°C | 146°C |
| Compound of Example 21 | not observed | 119°C |
| Compound of Example 22 | not observed | 106°C |
| Compound of Example 23 | not observed | 118°C |
| Compound of Example 24 | not observed | 133°C |
| Compound of Example 25 | not observed | 136°C |
| Compound of Example 26 | 286°C | 124°C |
| Compound of Example 27 | not observed | 117°C |
| Compound of Example 28 | 218°C | 114°C |
| Compound of Example 29 | not observed | 127°C |
| Compound of Example 31 | not observed | 110°C |
| Compound of Example 32 | not observed | 122°C |
| Compound of Example 33 | 269°C | 117°C |
| Compound of Example 34 | 277°C | 122°C |
| Compound of Example 35 | not observed | 117°C |

The above mentioned arylamine compounds have glass transition points of 100°C or higher, demonstrating that the compounds have a stable thin-film state.

### Example 37

A 100 nm-thick vapor-deposited film was fabricated on an ITO substrate using the following arylamine compounds, and a work function was measured using an ionization potential measuring device (PYS-202 produced by Sumitomo Heavy Industries, Ltd.).

| | Work function |
|---|---|
| Compound of Example 1 | 5.65 eV |
| Compound of Example 3 | 5.65 eV |
| Compound of Example 4 | 5.67 eV |
| Compound of Example 5 | 5.66 eV |
| Compound of Example 6 | 5.69 eV |
| Compound of Example 7 | 5.63 eV |
| Compound of Example 8 | 5.70 eV |
| Compound of Example 9 | 5.72 eV |
| Compound of Example 10 | 5.62 eV |
| Compound of Example 11 | 5.61 eV |
| Compound of Example 12 | 5.62 eV |
| Compound of Example 13 | 5.67 eV |
| Compound of Example 14 | 5.75 eV |
| Compound of Example 15 | 5.75 eV |
| Compound of Example 16 | 5.79 eV |
| Compound of Example 17 | 5.68 eV |
| Compound of Example 18 | 5.76 eV |
| Compound of Example 19 | 5.70 eV |
| Compound of Example 20 | 5.79 eV |
| Compound of Example 21 | 5.71 eV |
| Compound of Example 22 | 5.79 eV |
| Compound of Example 23 | 5.72 eV |
| Compound of Example 24 | 5.70 eV |
| Compound of Example 25 | 5.71 eV |
| Compound of Example 26 | 5.65 eV |
| Compound of Example 27 | 5.70 eV |
| Compound of Example 28 | 5.67 eV |
| Compound of Example 29 | 5.69 eV |
| Compound of Example 32 | 5.76 eV |

As the results show, the above mentioned arylamine compounds have desirable energy levels (5.6 to 5.8 eV) compared to the work function 5.4 eV of common hole transport materials such as NPD and TPD, and thus possess desirable hole transportability.

### Example 38

The organic EL device, as shown in FIG. 1, was fabricated by vapor-depositing a hole injection layer 3, a hole transport layer 4, a light emitting layer 5, an electron transport layer 6, an electron injection layer 7, and a cathode (aluminum electrode) 8 in this order on a glass substrate 1 on which an ITO electrode was formed as a transparent anode 2 beforehand.

Specifically, the glass substrate 1 having ITO having a film thickness of 150 nm formed thereon was subjected to ultrasonic washing in isopropyl alcohol for 20 minutes and then dried for 10 minutes on a hot plate heated to 200°C. Thereafter, after performing an UV ozone treatment for 15 minutes, the glass substrate with ITO was installed in a vacuum vapor deposition apparatus, and the pressure was reduced to 0.001 Pa or lower. Subsequently, as the hole injection layer 3 covering the transparent anode 2, an electron acceptor (Acceptor-1) of the structural formula below and Compound (1-1) of Example 1 were formed in a film thickness of 7 nm by dual vapor deposition at a vapor deposition rate ratio of Acceptor-1/Compound (1-1) = 2/98. As the hole transport layer 4 on the hole injection layer 3, Compound 1-1 of Example 1 was formed in a film thickness of 138 nm. As the light emitting layer 5 on the hole transport layer 4, Compound EMD-1 of the structural formula below and Compound EMH-1 of the structural formula below were formed in a film thickness of 20 nm by dual vapor deposition at a vapor deposition rate ratio of EMD-1/EMH-1 = 3/97. As the electron transport layer 6 on the light emitting layer 5, Compound (5b-1) having an anthracene ring structure of the structural formula below and Compound ETM-1 of the structural formula below were formed in a film thickness of 30 nm by dual vapor deposition at a vapor deposition rate ratio of Compound (5b-1)/ETM-1 = 50/50. As the electron injection layer 7 on the electron transport layer 6, lithium fluoride was formed in a film thickness of 1 nm. Finally, aluminum was vapor-deposited in a thickness of 100 nm to form the cathode 8. The characteristics of the thus fabricated organic EL device were measured in the atmosphere at an ordinary temperature. Table 1 summarizes the results of the measurement of driving voltages performed by applying a direct current voltage to the fabricated organic EL device. The fabricated organic EL device provided a good luminous efficiency by applying a direct current voltage thereto.

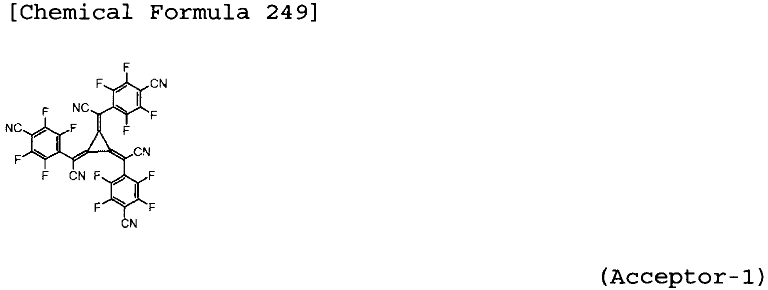

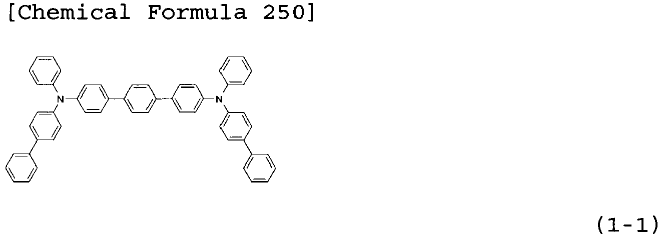

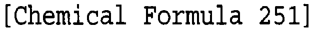

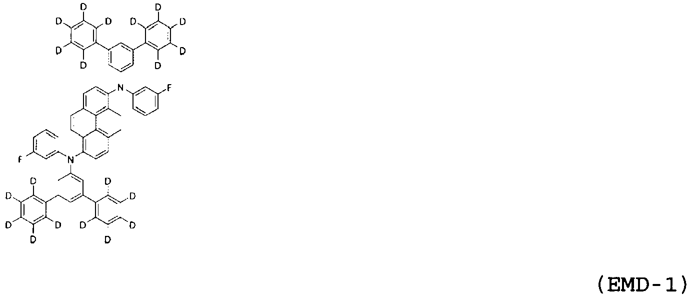

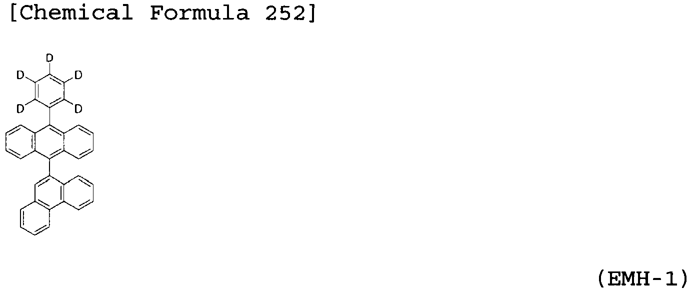

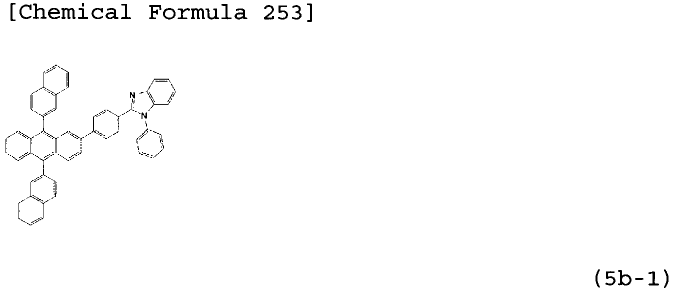

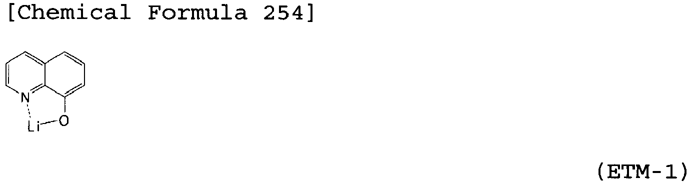

### Example 39

An organic EL device was fabricated under the same condition as in Example 38 except that the hole injection layer 3 was formed in a film thickness of 7 nm by dual vapor deposition, in which the vapor deposition rate ratio of the electron acceptor (Acceptor-1) of the aforementioned structural formula and the compound (1-1) of Example 1 was changed to Acceptor-1/Compound (1-1) = 3/97 instead of Acceptor-1/Compound (1-1) = 2/98. The characteristics of the organic EL device were measured in the atmosphere at ordinary temperature. Table 1 summarizes the results of the measurement of driving voltages performed by applying a direct current voltage to the fabricated organic EL device. The fabricated organic EL device provided a good luminous efficiency by applying a direct current voltage thereto. Example 40 (not forming part of the claimed invention)

An organic EL device was fabricated under the same condition as in Example 38 except that the compound (1-2) of Example 4 was used as the material for the hole injection layer 3 instead of the compound (1-1) of Example 1, and the hole injection layer 3 was formed in a film thickness of 7 nm by dual vapor deposition of the electron acceptor (Acceptor-1) of the aforementioned structural formula and the compound (1-2) of Example 4 at a vapor deposition rate ratio of Acceptor-1/Compound (1-2) = 2/98, and on the hole injection layer 3, the hole transport layer 4 was formed with the compound (1-2) of Example 4 in a film thickness of 138 nm. The characteristics of the organic EL device were measured in the atmosphere at ordinary temperature. Table 1 summarizes the results of the measurement of driving voltages performed by applying a direct current voltage to the fabricated organic EL device. The fabricated organic EL device provided a good luminous efficiency by applying a direct current voltage thereto.

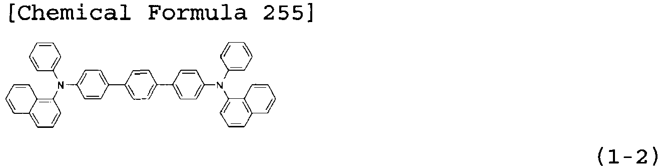

### Example 41 (not forming part of the claimed invention)

An organic EL device was fabricated under the same condition as in Example 40 except that the hole injection layer 3 was formed in a film thickness of 7 nm by dual vapor deposition, in which the vapor deposition rate ratio of the electron acceptor (Acceptor-1) of the aforementioned structural formula and the compound (1-2) of Example 4 was changed to Acceptor-1/Compound (1-2) = 3/97 instead of Acceptor-1/Compound (1-2) = 2/98. The characteristics of the organic EL device were measured in the atmosphere at ordinary temperature. Table 1 summarizes the results of the measurement of driving voltages performed by applying a direct current voltage to the fabricated organic EL device. The fabricated organic EL device provided a good luminous efficiency by applying a direct current voltage thereto.

### Example 42

An organic EL device was fabricated under the same condition as in Example 39 except that the film thickness of the hole injection layer 3 was changed to 5 nm instead of 7 nm, and the film thickness of the hole transport layer 4 was changed to 140 nm instead of 138 nm, i.e., the hole injection layer 3 was formed in a film thickness of 5 nm by dual vapor deposition of the electron acceptor (Acceptor-1) of the aforementioned structural formula and the compound (1-1) of Example 1 at a vapor deposition rate ratio of Acceptor-1/Compound (1-1) = 3/97, and on the hole injection layer 3, the hole transport layer 4 was formed with the compound (1-1) of Example 1 in a film thickness of 140 nm. The characteristics of the organic EL device were measured in the atmosphere at ordinary temperature. Table 1 summarizes the results of the measurement of driving voltages performed by applying a direct current voltage to the fabricated organic EL device. The fabricated organic EL device provided a good luminous efficiency by applying a direct current voltage thereto.

### Example 43

An organic EL device was fabricated under the same condition as in Example 39 except that the film thickness of the hole injection layer 3 was changed to 10 nm instead of 7 nm, and the film thickness of the hole transport layer 4 was changed to 135 nm instead of 138 nm, i.e., the hole injection layer 3 was formed in a film thickness of 10 nm by dual vapor deposition of the electron acceptor (Acceptor-1) of the aforementioned structural formula and the compound (1-1) of Example 1 at a vapor deposition rate ratio of Acceptor-1/Compound (1-1) = 3/97, and on the hole injection layer 3, the hole transport layer 4 was formed with the compound (1-1) of Example 1 in a film thickness of 135 nm. The characteristics of the organic EL device were measured in the atmosphere at ordinary temperature. Table 1 summarizes the results of the measurement of driving voltages performed by applying a direct current voltage to the fabricated organic EL device. The fabricated organic EL device provided a good luminous efficiency by applying a direct current voltage thereto.

### Example 44

An organic EL device was fabricated under the same condition as in Example 39 except that the film thickness of the hole injection layer 3 was changed to 30 nm instead of 7 nm, and the film thickness of the hole transport layer 4 was changed to 115 nm instead of 138 nm, i.e., the hole injection layer 3 was formed in a film thickness of 30 nm by dual vapor deposition of the electron acceptor (Acceptor-1) of the aforementioned structural formula and the compound (1-1) of Example 1 at a vapor deposition rate ratio of Acceptor-1/Compound (1-1) = 3/97, and on the hole injection layer 3, the hole transport layer 4 was formed with the compound (1-1) of Example 1 in a film thickness of 115 nm. The characteristics of the organic EL device were measured in the atmosphere at ordinary temperature. Table 1 summarizes the results of the measurement of driving voltages performed by applying a direct current voltage to the fabricated organic EL device. The fabricated organic EL device provided a good luminous efficiency by applying a direct current voltage thereto.

### Example 45 (not forming part of the claimed invention)

An organic EL device was fabricated under the same condition as in Example 41 except that the film thickness of the hole injection layer 3 was changed to 5 nm instead of 7 nm, and the film thickness of the hole transport layer 4 was changed to 140 nm instead of 138 nm, i.e., the hole injection layer 3 was formed in a film thickness of 5 nm by dual vapor deposition of the electron acceptor (Acceptor-1) of the aforementioned structural formula and the compound (1-2) of Example 4 at a vapor deposition rate ratio of Acceptor-1/Compound (1-2) = 3/97, and on the hole injection layer 3, the hole transport layer 4 was formed with the compound (1-2) of Example 4 in a film thickness of 140 nm. The characteristics of the organic EL device were measured in the atmosphere at ordinary temperature. Table 1 summarizes the results of the measurement of driving voltages performed by applying a direct current voltage to the fabricated organic EL device. The fabricated organic EL device provided a good luminous efficiency by applying a direct current voltage thereto.

### Example 46 (not forming part of the claimed invention)

An organic EL device was fabricated under the same condition as in Example 41 except that the film thickness of the hole injection layer 3 was changed to 10 nm instead of 7 nm, and the film thickness of the hole transport layer 4 was changed to 135 nm instead of 138 nm, i.e., the hole injection layer 3 was formed in a film thickness of 10 nm by dual vapor deposition of the electron acceptor (Acceptor-1) of the aforementioned structural formula and the compound (1-2) of Example 4 at a vapor deposition rate ratio of Acceptor-1/Compound (1-2) = 3/97, and on the hole injection layer 3, the hole transport layer 4 was formed with the compound (1-2) of Example 4 in a film thickness of 135 nm. The characteristics of the organic EL device were measured in the atmosphere at ordinary temperature. Table 1 summarizes the results of the measurement of driving voltages performed by applying a direct current voltage to the fabricated organic EL device. The fabricated organic EL device provided a good luminous efficiency by applying a direct current voltage thereto.

### Example 47 (not forming part of the claimed invention)

An organic EL device was fabricated under the same condition as in Example 41 except that the film thickness of the hole injection layer 3 was changed to 30 nm instead of 7 nm, and the film thickness of the hole transport layer 4 was changed to 115 nm instead of 138 nm, i.e., the hole injection layer 3 was formed in a film thickness of 30 nm by dual vapor deposition of the electron acceptor (Acceptor-1) of the aforementioned structural formula and the compound (1-2) of Example 4 at a vapor deposition rate ratio of Acceptor-1/Compound (1-2) = 3/97, and on the hole injection layer 3, the hole transport layer 4 was formed with the compound (1-2) of Example 4 in a film thickness of 115 nm. The characteristics of the organic EL device were measured in the atmosphere at ordinary temperature. Table 1 summarizes the results of the measurement of driving voltages performed by applying a direct current voltage to the fabricated organic EL device. The fabricated organic EL device provided a good luminous efficiency by applying a direct current voltage thereto.

### Comparative Example 1

For comparison, an organic EL device was fabricated under the same condition as in Example 43 except that the hole injection layer 3 was formed in a film thickness of 10 nm by dual vapor deposition, in which the vapor deposition rate ratio of the electron acceptor (Acceptor-1) of the aforementioned structural formula and the compound (1-1) of Example 1 was changed to Acceptor-1/Compound (1-1) = 1/99 instead of Acceptor-1/Compound (1-1) = 3/97. The characteristics of the organic EL device were measured in the atmosphere at ordinary temperature. Table 1 summarizes the results of the measurement of driving voltages performed by applying a direct current voltage to the fabricated organic EL device. The fabricated organic EL device provided a good luminous efficiency by applying a direct current voltage thereto.

### Comparative Example 2

For comparison, an organic EL device was fabricated under the same condition as in Example 46 except that the hole injection layer 3 was formed in a film thickness of 10 nm by dual vapor deposition, in which the vapor deposition rate ratio of the electron acceptor (Acceptor-1) of the aforementioned structural formula and the compound (1-2) of Example 4 was changed to Acceptor-1/Compound (1-2) = 1/99 instead of Acceptor-1/Compound (1-2) = 3/97. The characteristics of the organic EL device were measured in the atmosphere at ordinary temperature. Table 1 summarizes the results of the measurement of driving voltages performed by applying a direct current voltage to the fabricated organic EL device. The fabricated organic EL device provided a good luminous efficiency by applying a direct current voltage thereto.

By using the organic EL devices fabricated in Examples 38 to 47 and Comparative Examples 1 and 2, the driving voltage on applying an electric current of a current density of 10 mA/cm² (i.e., the driving voltage at the start of driving, V) and the driving voltage immediately after continuously applying an electric current of a current density of 10 mA/cm² for 100 hours (i.e., the driving voltage after driving for 100 hours, V) were measured, and the difference thereof (i.e., the driving voltage rise = (driving voltage after driving for 100 hours) - (driving voltage at the start of driving), V) was calculated. Table 1 summarizes the measurement results. Examples 40, 41 and 45-47 in Table 1 are not forming part of the claimed invention.

**[Table 1]**

| | Hole injection layer | | | Hole transport layer | | Driving voltage (V) (@10 mA/cm²) | Driving voltage rise (V) |
|---|---|---|---|---|---|---|---|
| | Compound | Doping concentration (%) | Film thickness (nm) | Compound | Film thickness (nm) | | |
| Example 38 | Compound 1-1 | 2 | 7 | Compound 1-1 | 138 | 3.73 | 0.41 |
| Example 39 | Compound 1-1 | 3 | 7 | Compound 1-1 | 138 | 3.66 | 0.21 |
| Example 40 | Compound 1-2 | 2 | 7 | Compound 1-2 | 138 | 3.84 | 0.56 |
| Example 41 | Compound 1-2 | 3 | 7 | Compound 1-2 | 138 | 3.70 | 0.19 |
| Example 42 | Compound 1-1 | 3 | 5 | Compound 1-1 | 140 | 3.66 | 0.32 |
| Example 43 | Compound 1-1 | 3 | 10 | Compound 1-1 | 135 | 3.64 | 0.10 |
| Example 44 | Compound 1-1 | 3 | 30 | Compound 1-1 | 115 | 3.61 | 0.03 |
| Example 45 | Compound 1-2 | 3 | 5 | Compound 1-2 | 140 | 3.76 | 0.37 |
| Example 46 | Compound 1-2 | 3 | 10 | Compound 1-2 | 135 | 3.64 | 0.08 |
| Example 47 | Compound 1-2 | 3 | 30 | Compound 1-2 | 115 | 3.60 | 0.03 |
| Comparative Example 1 | Compound 1-1 | 1 | 10 | Compound 1-1 | 135 | 3.93 | 0.90 |
| Comparative Example 2 | Compound 1-2 | 1 | 10 | Compound 1-2 | 135 | 4.01 | 0.98 |

As shown in Table 1, the driving voltage on applying an electric current of a current density of 10 mA/cm² was as low as 3.60 to 3.84 V for all the organic EL devices of Examples 38 to 47, as compared to 3.93 to 4.01 V for the organic EL devices of Comparative Examples 1 and 2. The driving voltage rise ((driving voltage after driving for 100 hours) - (driving voltage at the start of driving)) was 0.03 to 0.56 V for the organic EL devices of Examples 38 to 47, as compared to 0.90 to 0.98 V for the organic EL devices of Comparative Examples 1 and 2, from which it was understood that the low driving voltage was retained, or the driving voltage rise was effectively suppressed.

The organic EL device of the invention can retain a low driving voltage or can be effectively suppressed in the driving voltage rise, by controlling the doping concentration of the electron acceptor and/or the film thickness of the organic layer containing the electron acceptor.

It has been found that in the organic EL device of the invention, the particular arylamine compound (having the particular structure and ionization potential) is selected as a material of a hole injection layer and doped with an electron acceptor to enable efficient injection and transport of holes from an anode, the particular arylamine compound (having the particular structure) that is not doped with an electron acceptor is combined as a material of a hole transport layer therewith, and further the doping concentration of the electron acceptor and/or the film thickness of the organic layer containing the electron acceptor are controlled, thereby achieving an organic EL device with high efficiency that retains a low driving voltage or is effectively suppressed in the driving voltage rise.

### Industrial Applicability

The organic EL device with high efficiency of the invention, in which the particular arylamine compound (having the particular structure and ionization potential) is doped with an electron acceptor to enable efficient injection and transport of holes from an electrode, and the doping concentration of the electron acceptor and/or the film thickness of the organic layer containing the electron acceptor are controlled, thereby retaining a low driving voltage or effectively suppressing the driving voltage rise, can reduce the electric power consumption and can be applied, for example, to the purposes of electric home appliances and illuminations.

### Reference Signs List

- 1: Glass substrate
- 2: Transparent anode
- 3: Hole injection layer
- 4: Hole transport layer
- 5: Light emitting layer
- 6: Electron transport layer
- 7: Electron injection layer
- 8: Cathode

## Claims

1. An organic electroluminescent device of the top emission type comprising at least an anode (2), a hole injection layer (3), a hole transport layer (4), a light emitting layer (5), an electron transport layer (6), and a cathode (8), in this order, wherein the hole injection layer (3) includes an arylamine compound represented by the following general formula (1) and an electron acceptor: 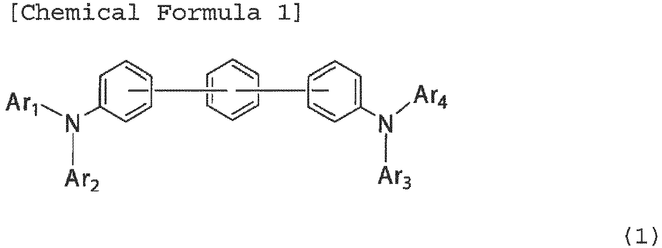
wherein Ar₁ to Ar₄ may be the same or different, and represent a substituted or unsubstituted aromatic hydrocarbon group, a substituted or unsubstituted aromatic heterocyclic group, or a substituted or unsubstituted condensed polycyclic aromatic group,
wherein Ar₁ and Ar₂ are selected from the group consisting of phenyl, biphenylyl, terphenylyl, anthracenyl, phenanthrenyl, fluorenyl, indenyl, pyrenyl, perylenyl, fluoranthenyl, triphenylenyl, pyridyl, pyrimidinyl, triazinyl, furyl, pyrrolyl, thienyl, quinolyl, isoquinolyl, benzofuranyl, benzothienyl, indolyl, carbazolyl, benzoxazolyl, benzothiazolyl, quinoxalinyl, benzoimidazolyl, pyrazolyl, dibenzofuranyl, dibenzothienyl, naphthyridinyl, phenanthrolinyl, acridinyl, and carbolinyl,
wherein Ar₃ is selected from the group consisting of phenyl, terphenylyl, naphthyl, anthracenyl, phenanthrenyl, fluorenyl, indenyl, pyrenyl, perylenyl, fluoranthenyl, triphenylenyl, pyridyl, pyrimidinyl, triazinyl, furyl, pyrrolyl, thienyl, quinolyl, isoquinolyl, benzofuranyl, benzothienyl, indolyl, carbazolyl, benzoxazolyl, benzothiazolyl, quinoxalinyl, benzoimidazolyl, pyrazolyl, dibenzofuranyl, dibenzothienyl, naphthyridinyl, phenanthrolinyl, acridinyl, and carbolinyl, and
wherein the hole transport layer (4) has a film thickness of 100 to 200 nm,
wherein the hole transport layer (4) has the same arylamine compound as the hole injection layer (3), but no electron acceptor,
wherein the electron acceptor is contained in an amount of 0.5 to 30% by weight based on the total hole injection layer (3), and
wherein the hole injection layer (3) has a film thickness of 5 to 150 nm.

2. The organic electroluminescent device according to claim 1, wherein the layers that are adjacent to the light emitting layer (5) do not include an electron acceptor.

3. The organic electroluminescent device according to claim 1 or 2, wherein the electron acceptor is an electron acceptor selected from trisbromophenylamine hexachloroantimony, tetracyanoquinodimethane (TCNQ), 2,3,5,6-tetrafluoro-tetracyano-1,4-benzoquinodimethane (F4TCNQ), and a radialene derivative.

4. The organic electroluminescent device according to any one of claims 1 to 3, wherein the electron acceptor is a radialene derivative represented by the following general formula (2): 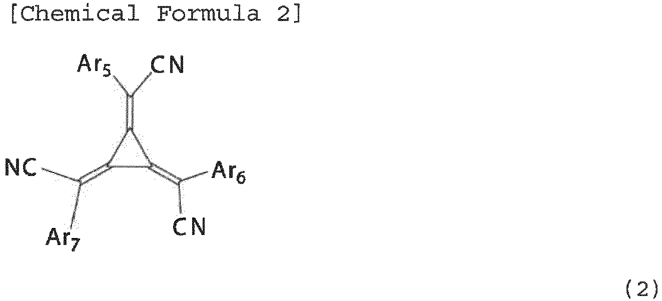 wherein Ar₅ to Ar₇ may be the same or different, and represent an aromatic hydrocarbon group, an aromatic heterocyclic group, or a condensed polycyclic aromatic group, having an electron acceptor group as a substituent.

5. The organic electroluminescent device according to any one of claims 1 to 4, wherein the arylamine compound of the general formula (1) has an ionization potential of 5.4 to 5.8 eV, as determined using an ionization potential measuring device PYS-202 produced by Sumitomo Heavy Industries, Ltd.

6. The organic electroluminescent device according to any one of claims 1 to 5, wherein the hole transport layer (4) includes an arylamine compound having a structure in which two to six triphenylamine structures are joined within a molecule via a single bond or a divalent group that does not contain a heteroatom.

7. The organic electroluminescent device according to claim 6, wherein the arylamine compound having a structure in which two to six triphenylamine structures are joined within a molecule via a single bond or a divalent group that does not contain a heteroatom is an arylamine compound represented by the following general formula (3): 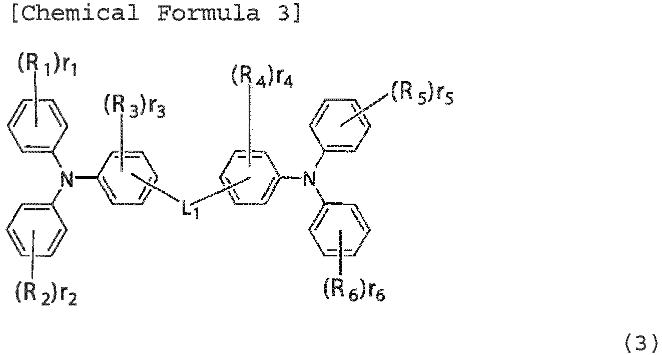 wherein R₁ to R₆ represent a deuterium atom, a fluorine atom, a chlorine atom, cyano, nitro, linear or branched alkyl of 1 to 6 carbon atoms that may have a substituent, cycloalkyl of 5 to 10 carbon atoms that may have a substituent, linear or branched alkenyl of 2 to 6 carbon atoms that may have a substituent, linear or branched alkyloxy of 1 to 6 carbon atoms that may have a substituent, cycloalkyloxy of 5 to 10 carbon atoms that may have a substituent, a substituted or unsubstituted aromatic hydrocarbon group, a substituted or unsubstituted aromatic heterocyclic group, a substituted or unsubstituted condensed polycyclic aromatic group, or substituted or unsubstituted aryloxy; r₁ to r₆ may be the same or different, r₁, r₂, r₅, and r₆ representing an integer of 0 to 5, and r₃ and r₄ representing an integer of 0 to 4, where when r₁, r₂, r₅, and r₆ are an integer of 2 to 5, or when r₂ and r₄ are an integer of 2 to 4, R₁ to R₆, a plurality of which bind to the same benzene ring, may be the same or different and may bind to each other via a single bond, substituted or unsubstituted methylene, an oxygen atom, or a sulfur atom to form a ring; and L₁ represents a divalent linking group or a single bond.

8. The organic EL device according to claim 6, wherein the arylamine compound having a structure in which two to six triphenylamine structures are joined within a molecule via a single bond or a divalent group that does not contain a heteroatom is an arylamine compound of the following general formula (4): 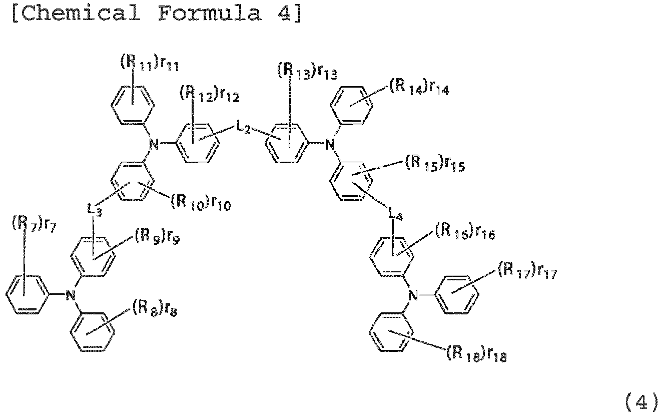 wherein R₇ to R₁₈ represent a deuterium atom, a fluorine atom, a chlorine atom, cyano, nitro, linear or branched alkyl of 1 to 6 carbon atoms that may have a substituent, cycloalkyl of 5 to 10 carbon atoms that may have a substituent, linear or branched alkenyl of 2 to 6 carbon atoms that may have a substituent, linear or branched alkyloxy of 1 to 6 carbon atoms that may have a substituent, cycloalkyloxy of 5 to 10 carbon atoms that may have a substituent, a substituted or unsubstituted aromatic hydrocarbon group, a substituted or unsubstituted aromatic heterocyclic group, a substituted or unsubstituted condensed polycyclic aromatic group, or substituted or unsubstituted aryloxy; r₇ to r₁₈ may be the same or different, r₇, r₈, r₁₁, r₁₄, r₁₇, and r₁₈ representing an integer of 0 to 5, and r₉, r₁₀, r₁₂, r₁₃, r₁₅, and r₁₆ representing an integer of 0 to 4. where when r₇, r₈, r₁₁, r₁₄, r₁₇, and r₁₈ are an integer of 2 to 5, or when r₉, r₁₀, r₁₂, r₁₃, r₁₅, and r₁₆ are an integer of 2 to 4, R₇ to R₁₈, a plurality of which bind to the same benzene ring, may be the same or different and may bind to each other via a single bond, substituted or unsubstituted methylene, an oxygen atom, or a sulfur atom to form a ring; and L₂, L₃, and L₄ may be the same or different, and represent a divalent linking group or a single bond.

9. The organic EL device according to any one of claims 1 to 8, wherein the electron transport layer (6) includes a compound represented by the following general formula (5) having an anthracene ring structure: 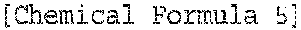 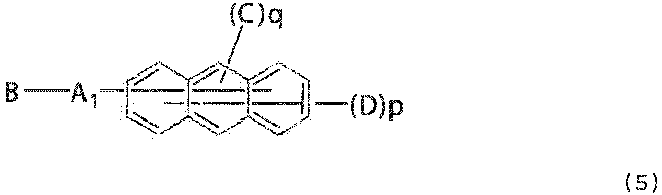 wherein A₁ represents a divalent group of a substituted or unsubstituted aromatic hydrocarbon, a divalent group of a substituted or unsubstituted aromatic heterocyclic ring, a divalent group of substituted or unsubstituted condensed polycyclic aromatics, or a single bond; B represents a substituted or unsubstituted aromatic heterocyclic group; C represents a substituted or unsubstituted aromatic hydrocarbon group, a substituted or unsubstituted aromatic heterocyclic group, or a substituted or unsubstituted condensed polycyclic aromatic group; D may be the same or different, and represents a hydrogen atom, a deuterium atom, a fluorine atom, a chlorine atom, cyano, trifluoromethyl, linear or branched alkyl of 1 to 6 carbon atoms, a substituted or unsubstituted aromatic hydrocarbon group, a substituted or unsubstituted aromatic heterocyclic group, or a substituted or unsubstituted condensed polycyclic aromatic group; and p represents 7 or 8, and q represents 1 or 2 while maintaining a relationship that a sum of p and q is 9.

10. The organic electroluminescent device according to any one of claims 1 to 9, wherein the light emitting layer (5) includes a blue light emitting dopant.

11. The organic electroluminescent device according to claim 10, wherein the light emitting layer (5) includes a pyrene derivative, which is a blue light emitting dopant.

12. The organic electroluminescent device according to any one of claims 1 to 11, wherein the light emitting layer (5) includes an anthracene derivative.

13. The organic EL device according to claim 12, wherein the light emitting layer (5) includes a host material which is the anthracene derivative.

## Patentansprüche

1. Organische Elektrolumineszenzvorrichtung vom Top-Emissionstyp, umfassend mindestens eine Anode (2), eine Lochinjektionsschicht (3), eine Lochtransportschicht (4), eine lichtemittierende Schicht (5), eine Elektronentransportschicht (6) und eine Kathode (8) in dieser Reihenfolge, wobei die Lochinjektionsschicht (3) eine Arylaminverbindung, dargestellt durch die folgende allgemeine Formel (1), und einen Elektronenakzeptor enthält: 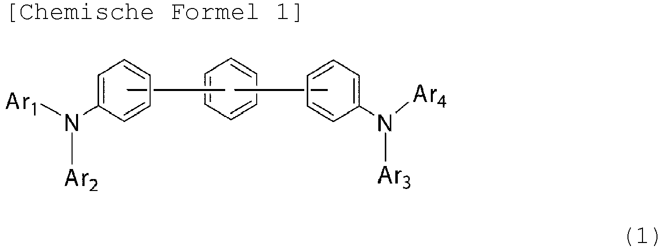
worin Ar ₁ bis Ar ₄ gleich oder verschieden sein können und eine substituierte oder unsubstituierte aromatische Kohlenwasserstoffgruppe, eine substituierte oder unsubstituierte aromatische heterocyclische Gruppe oder eine substituierte oder unsubstituierte kondensierte polycyclische aromatische Gruppe darstellen,
worin Ar ₁ und Ar ₂ ausgewählt sind aus der Gruppe, bestehend aus Phenyl, Biphenylyl, Terphenylyl, Anthracenyl, Phenanthrenyl, Fluorenyl, Indenyl, Pyrenyl, Perylenyl, Fluoranthenyl, Triphenylenyl, Pyridyl, Pyrimidinyl, Triazinyl, Furyl, Pyrrolyl, Thienyl, Chinolyl, Isochinolyl, Benzofuranyl, Benzothienyl, Indolyl, Carbazolyl, Benzoxazolyl, Benzothiazolyl, Chinoxalinyl, Benzoimidazolyl, Pyrazolyl, Dibenzofuranyl, Dibenzothienyl, Naphthyridinyl, Phenanthrolinyl, Acridinyl und Carbolinyl,
worin Ar₃ ausgewählt ist aus der Gruppe, bestehend aus Phenyl, Terphenylyl, Naphthyl, Anthracenyl, Phenanthrenyl, Fluorenyl, Indenyl, Pyrenyl, Perylenyl, Fluoranthenyl, Triphenylenyl, Pyridyl, Pyrimidinyl, Triazinyl, Furyl, Pyrrolyl, Thienyl, Chinolyl, Isochinolyl, Benzofuranyl, Benzothienyl, Indolyl, Carbazolyl, Benzoxazolyl, Benzothiazolyl, Chinoxalinyl, Benzoimidazolyl, Pyrazolyl, Dibenzofuranyl, Dibenzothienyl, Naphthyridinyl, Phenanthrolinyl, Acridinyl und Carbolinyl, und
wobei die Lochtransportschicht (4) eine Schichtdicke von 100 bis 200 nm aufweist,
wobei die Lochtransportschicht (4) die gleiche Arylaminverbindung wie die Lochinjektionsschicht (3), aber keinen Elektronenakzeptor aufweist,
wobei der Elektronenakzeptor in einer Menge von 0,5 bis 30 Gew.-%, bezogen auf die gesamte Lochinjektionsschicht (3), enthalten ist, und
wobei die Lochinjektionsschicht (3) eine Filmdicke von 5 bis 150 nm aufweist.

2. Organische Elektrolumineszenzvorrichtung nach Anspruch 1, wobei die Schichten, die an die lichtemittierende Schicht (5) angrenzen, keinen Elektronenakzeptor enthalten.

3. Organische Elektrolumineszenzvorrichtung nach Anspruch 1 oder 2, wobei der Elektronenakzeptor ein Elektronenakzeptor ist, der aus Trisbromphenylaminhexachlorantimon, Tetracyanochinodimethan (TCNQ), 2,3,5,6-Tetrafluortetracyano-1,4-benzochinodimethan (F4TCNQ) und einem Radialenderivat ausgewählt ist.

4. Organische Elektrolumineszenzvorrichtung nach einem der Ansprüche 1 bis 3, wobei der Elektronenakzeptor ein Radialenderivat ist, das durch die folgende allgemeine Formel (2) dargestellt wird: 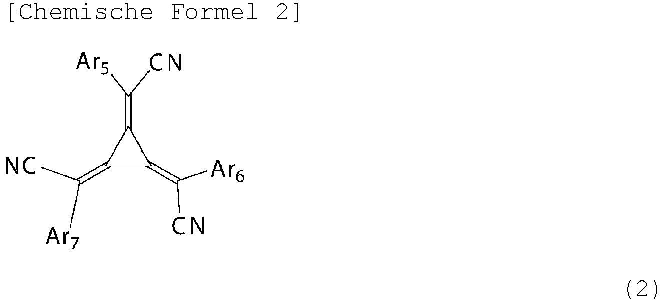 worin Ar₅ bis Ar₇ gleich oder verschieden sein können und eine aromatische Kohlenwasserstoffgruppe, eine aromatische heterocyclische Gruppe oder eine kondensierte polycyclische aromatische Gruppe mit einer Elektronenakzeptorgruppe als Substituent darstellen.

5. Organische Elektrolumineszenzvorrichtung nach einem der Ansprüche 1 bis 4, wobei die Arylaminverbindung der allgemeinen Formel (1) ein Ionisationspotential von 5,4 bis 5,8 eV aufweist, bestimmt unter Verwendung eines Ionisationspotential-Messgeräts von PYS-202 Sumitomo Heavy Industries, Ltd..

6. Organische Elektrolumineszenzvorrichtung nach einem der Ansprüche 1 bis 5, wobei die Lochtransportschicht (4) eine Arylaminverbindung mit einer Struktur enthält, in der zwei bis sechs Triphenylaminstrukturen innerhalb eines Moleküls über eine Einfachbindung oder eine zweiwertige Gruppe, die kein Heteroatom enthält, verbunden sind.

7. Organische Elektrolumineszenzvorrichtung nach Anspruch 6, wobei die Arylaminverbindung mit einer Struktur, in der zwei bis sechs Triphenylaminstrukturen innerhalb eines Moleküls über eine Einfachbindung oder eine zweiwertige Gruppe, die kein Heteroatom enthält, verbunden sind, eine Arylaminverbindung ist, dargestellt durch die folgende allgemeine Formel (3): 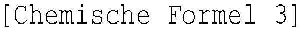 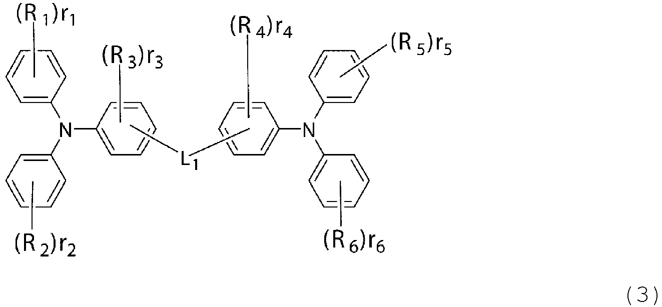 worin R₁ bis R₆ ein Deuteriumatom, ein Fluoratom, ein Chloratom, Cyano, Nitro, lineares oder verzweigtes Alkyl mit 1 bis 6 Kohlenstoffatomen, das einen Substituenten aufweisen kann, Cycloalkyl mit 5 bis 10 Kohlenstoffatomen, das einen Substituenten aufweisen kann, lineares oder verzweigtes Alkenyl mit 2 bis 6 Kohlenstoffatomen, das einen Substituenten aufweisen kann, lineares oder verzweigtes Alkyloxy mit 1 bis 6 Kohlenstoffatomen, das einen Substituenten aufweisen kann, Cycloalkyloxy mit 5 bis 10 Kohlenstoffatomen, das einen Substituenten aufweisen kann, eine substituierte oder unsubstituierte aromatische Kohlenwasserstoffgruppe, eine substituierte oder unsubstituierte aromatische heterocyclische Gruppe, eine substituierte oder unsubstituierte kondensierte polycyclische aromatische Gruppe oder substituiertes oder unsubstituiertes Aryloxy darstellen; r₁ bis r₆ gleich oder verschieden sein können, r₁ , r₂ , r₅ und r₆ eine ganze Zahl von 0 bis 5 darstellen und r₃ und r₄ eine ganze Zahl von 0 bis 4 darstellen, wobei wenn r₁, r₂, r₅ und r₆ eine ganze Zahl von 2 bis 5 sind oder r₃ und r₄ eine ganze Zahl von 2 bis 4 sind, R₁ bis R₆, von denen mehrere an denselben Benzolring binden, gleich oder verschieden sein können und über eine Einfachbindung, substituiertes oder unsubstituiertes Methylen, ein Sauerstoffatom oder ein Schwefelatom unter Bildung eines Rings aneinander binden können; und L ₁ eine zweiwertige Verknüpfungsgruppe oder eine Einfachbindung darstellt.

8. Organische EL-Vorrichtung nach Anspruch 6, wobei die Arylaminverbindung mit einer Struktur, in der zwei bis sechs Triphenylaminstrukturen innerhalb eines Moleküls über eine Einfachbindung oder eine zweiwertige Gruppe, die kein Heteroatom enthält, verbunden sind, eine Arylaminverbindung der folgenden allgemeine Formel (4) ist: 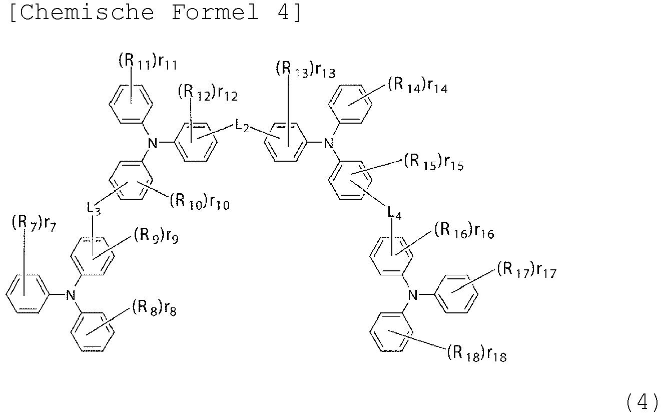 worin R₇ bis R₁₈ ein Deuteriumatom, ein Fluoratom, ein Chloratom, Cyano, Nitro, lineares oder verzweigtes Alkyl mit 1 bis 6 Kohlenstoffatomen, das einen Substituenten aufweisen kann, Cycloalkyl mit 5 bis 10 Kohlenstoffatomen, das einen Substituenten aufweisen kann, lineares oder verzweigtes Alkenyl mit 2 bis 6 Kohlenstoffatomen, das einen Substituenten aufweisen kann, lineares oder verzweigtes Alkyloxy mit 1 bis 6 Kohlenstoffatomen, das einen Substituenten aufweisen kann, Cycloalkyloxy mit 5 bis 10 Kohlenstoffatomen, das einen Substituenten aufweisen kann, eine substituierte oder unsubstituierte aromatische Kohlenwasserstoffgruppe, eine substituierte oder unsubstituierte aromatische heterocyclische Gruppe, eine substituierte oder unsubstituierte kondensierte polycyclische aromatische Gruppe oder substituiertes oder unsubstituiertes Aryloxy darstellen; r₇ bis r₁₈ gleich oder verschieden sein können, r₇ , r₈ , r₁₁, r₁₄, r₁₇ und r₁₈ eine ganze Zahl von 0 bis 5 darstellen, und r₉, r₁₀, r₁₂, r₁₃, r₁₅ und r₁₆ eine ganze Zahl von 0 bis 4 darstellen, wobei, wenn r₇, r₈ , r₁₁, r₁₄, r₁₇ und r₁₈ eine ganze Zahl von 2 bis 5 sind oder r₉, r₁₀, r₁₂, r₁₃, r₁₅ und r₁₆ eine ganze Zahl von 2 bis 4 sind, R₇ bis R₁₈, von denen mehrere an denselben Benzolring binden, gleich oder verschieden sein können und über eine Einfachbindung, substituiertes oder unsubstituiertes Methylen, ein Sauerstoffatom oder ein Schwefelatom aneinander binden können, um einen Ring zu bilden; und L₂, L₃ und L₄ gleich oder verschieden sein können und eine zweiwertige Verknüpfungsgruppe oder eine Einfachbindung darstellen.

9. Organische EL-Vorrichtung nach einem der Ansprüche 1 bis 8, wobei die Elektronentransportschicht (6) eine Verbindung der folgenden allgemeinen Formel (5) mit einer Anthracenringstruktur enthält: 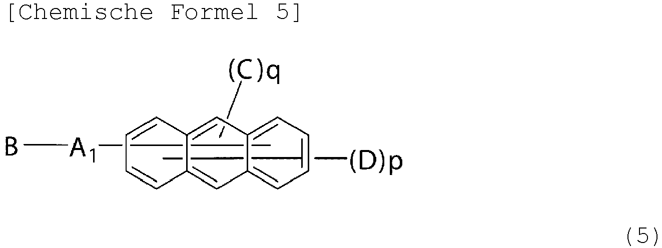 worin A₁ eine zweiwertige Gruppe eines substituierten oder unsubstituierten aromatischen Kohlenwasserstoffs, eine zweiwertige Gruppe eines substituierten oder unsubstituierten aromatischen heterocyclischen Rings, eine zweiwertige Gruppe eines substituierten oder unsubstituierten kondensierten polycyclischen Aromaten oder eine Einfachbindung darstellt; B eine substituierte oder unsubstituierte aromatische heterocyclische Gruppe darstellt; C eine substituierte oder unsubstituierte aromatische Kohlenwasserstoffgruppe, eine substituierte oder unsubstituierte aromatische heterocyclische Gruppe oder eine substituierte oder unsubstituierte kondensierte polycyclische aromatische Gruppe darstellt; D gleich oder verschieden sein kann und ein Wasserstoffatom, ein Deuteriumatom, ein Fluoratom, ein Chloratom, Cyano, Trifluormethyl, lineares oder verzweigtes Alkyl mit 1 bis 6 Kohlenstoffatomen, eine substituierte oder unsubstituierte aromatische Kohlenwasserstoffgruppe darstellt, eine substituierte oder unsubstituierte aromatische heterocyclische Gruppe oder eine substituierte oder unsubstituierte kondensierte polycyclische aromatische Gruppe darstellt; und p 7 oder 8 darstellt und q 1 oder 2 darstellt, während eine Beziehung aufrechterhalten wird, dass eine Summe von p und q 9 ist.

10. Organische Elektrolumineszenzvorrichtung nach einem der Ansprüche 1 bis 9, wobei die lichtemittierende Schicht (5) einen blaues Licht emittierenden Dotierstoff enthält.

11. Organische Elektrolumineszenzvorrichtung nach Anspruch 10, wobei die lichtemittierende Schicht (5) ein Pyrenderivat enthält, das ein blaues Licht emittierendes Dotierungsmittel ist.

12. Organische Elektrolumineszenzvorrichtung nach einem der Ansprüche 1 bis 11, wobei die lichtemittierende Schicht (5) ein Anthracenderivat enthält.

13. Organische EL-Vorrichtung nach Anspruch 12, wobei die lichtemittierende Schicht (5) ein Wirtsmaterial enthält, welches das Anthracenderivat ist.

## Revendications

1. Dispositif électroluminescent organique du type à émission par le haut comprenant au moins une anode (2), une couche d'injection de trous (3), une couche de transport de trous (4), une couche d'émission lumineuse (5), une couche de transport d'électrons (6) et une cathode (8), dans cet ordre, dans lequel la couche d'injection de trous (3) inclut un composé d'arylamine représenté par la formule générale suivante (1) et un accepteur d'électrons : 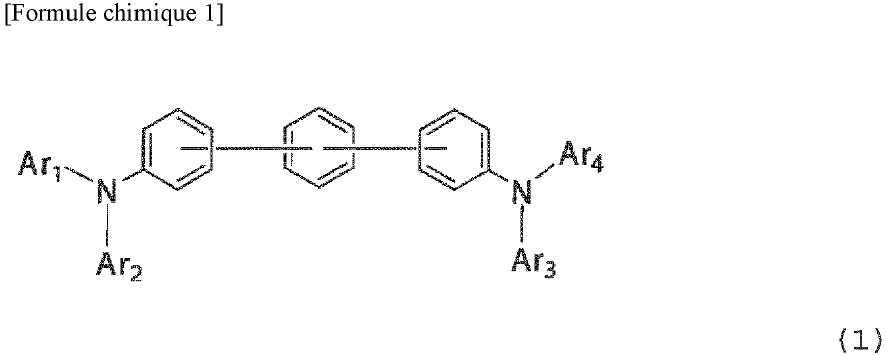
dans laquelle Ar₁ à Ar₄ peuvent être identiques ou différents, et représentent un groupe hydrocarbure aromatique substitué ou non substitué, un groupe hétérocyclique aromatique substitué ou non substitué, ou un groupe aromatique polycyclique condensé substitué ou non substitué,
dans laquelle Ar₁ et Ar₂ sont sélectionnés parmi le groupe constitué de phényle, biphénylyle, terphénylyle, anthracényle, phénanthrényle, fluorényle, indényle, pyrényle, pérylényle, fluoranthényle, triphénylényle, pyridyle, pyrimidinyle, triazinyle, furyle, pyrrolyle, thiényle, quinolyle, isoquinolyle, benzofuranyle, benzothiényle, indolyle, carbazolyle, benzoxazolyle, benzothiazolyle, quinoxalinyle, benzoimidazolyle, pyrazolyle, dibenzofuranyle, dibenzothiényle, naphtyridinyle, phénanthrolinyle, acridinyle, et carbolinyle,
dans laquelle Ar₃ est sélectionné parmi le groupe constitué de phényle, terphénylyle, naphtyle, anthracényle, phénanthrényle, fluorényle, indényle, pyrényle, pérylényle, fluoranthényle, triphénylényle, pyridyle, pyrimidinyle, triazinyle, furyle, pyrrolyle, thiényle, quinolyle, isoquinolyle, benzofuranyle, benzothiényle, indolyle, carbazolyle, benzoxazolyle, benzothiazolyle, quinoxalinyle, benzoimidazolyle, pyrazolyle, dibenzofuranyle, dibenzothiényle, naphtyridinyle, phénanthrolinyle, acridinyle, et carbolinyle, et
dans lequel la couche de transport de trous (4) a une épaisseur de film de 100 à 200 nm,
dans lequel la couche de transport de trous (4) a le même composé d'arylamine que la couche d'injection de trous (3), mais aucun accepteur d'électrons,
dans lequel l'accepteur d'électrons est contenu en une quantité de 0,5 à 30 % en poids basée sur la couche d'injection de trous totale (3), et
dans lequel la couche d'injection de trous (3) a une épaisseur de film de 5 à 150 nm.

2. Dispositif électroluminescent organique selon la revendication 1, dans lequel les couches qui sont adjacentes à la couche d'émission lumineuse (5) n'incluent pas d'accepteur d'électrons.

3. Dispositif électroluminescent organique selon la revendication 1 ou 2, dans lequel l'accepteur d'électrons est un accepteur d'électrons sélectionné parmi la trisbromophénylamine, l'hexachloroantimoine, le tétracyanoquinodiméthane (TCNQ), le 2,3,5,6-tétrafluoro-tétracyano-1,4-benzoquinodiméthane (F4TCNQ) et un dérivé de radialène.

4. Dispositif électroluminescent organique selon l'une quelconque des revendications 1 à 3, dans lequel l'accepteur d'électrons est un dérivé de radialène représenté par la formule générale suivante (2) : 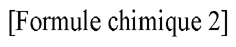 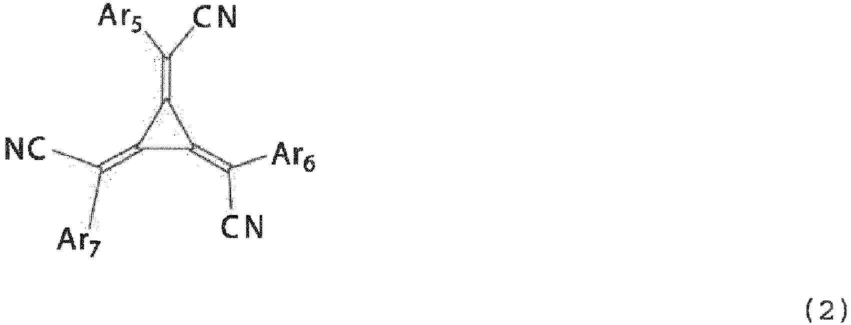 dans laquelle Ar₅ à Ar₇ peuvent être identiques ou différents, et représentent un groupe hydrocarbure aromatique, un groupe hétérocyclique aromatique, ou un groupe aromatique polycyclique condensé, ayant un groupe accepteur d'électrons en tant que substituant.

5. Dispositif électroluminescent organique selon l'une quelconque des revendications 1 à 4, dans lequel le composé d'arylamine de la formule générale (1) a un potentiel d'ionisation de 5,4 à 5,8 eV, tel que déterminé en utilisant un dispositif de mesure de potentiel d'ionisation PYS-202 produit par Sumitomo Heavy Industries, Ltd.

6. Dispositif électroluminescent organique selon l'une quelconque des revendications 1 à 5, dans lequel la couche de transport de trous (4) inclut un composé d'arylamine ayant une structure dans laquelle deux à six structures de triphénylamine sont reliées au sein d'une molécule par le biais d'une liaison simple ou d'un groupe divalent qui ne contient pas d'hétéroatome.

7. Dispositif électroluminescent organique selon la revendication 6, dans lequel le composé d'arylamine ayant une structure dans laquelle deux à six structures de triphénylamine sont reliées au sein d'une molécule par le biais d'une liaison simple ou d'un groupe divalent qui ne contient pas d'hétéroatome est un composé d'arylamine représenté par la formule générale suivante (3) : 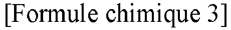 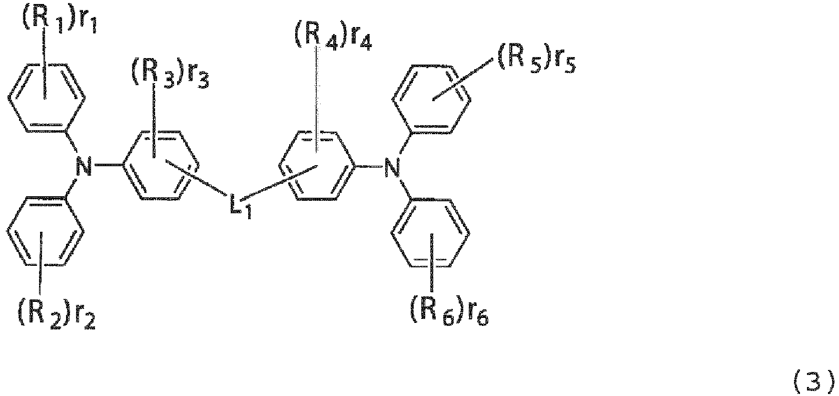 dans laquelle R₁ à R₆ représentent un atome de deutérium, un atome de fluor, un atome de chlore, cyano, nitro, alkyle linéaire ou ramifié de 1 à 6 atomes de carbone qui peuvent avoir un substituant, cycloalkyle de 5 à 10 atomes de carbone qui peuvent avoir un substituant, alkényle linéaire ou ramifié de 2 à 6 atomes de carbone qui peuvent avoir un substituant, alkyloxy linéaire ou ramifié de 1 à 6 atomes de carbone qui peuvent avoir un substituant, cycloalkyloxy de 5 à 10 atomes de carbone qui peuvent avoir un substituant, un groupe hydrocarbure aromatique substitué ou non substitué, un groupe hétérocyclique aromatique substitué ou non substitué, un groupe aromatique polycyclique condensé substitué ou non substitué, ou aryloxy substitué ou non substitué ; r₁ à r₆ peuvent être identiques ou différents, r₁, r₂, r₅ et r₆ représentant un entier de 0 à 5, et r₃ et r₄ représentant un entier de 0 à 4, où lorsque r₁, r₂, r₅ et r₆ sont un entier de 2 à 5, ou lorsque r₃ et r₄ sont un entier de 2 à 4, R₁ à R₆ dont une pluralité se lient au même anneau benzénique, peuvent être identiques ou différents et peuvent se lier l'un à l'autre par le biais d'une liaison simple, de méthylène substitué ou non substitué, d'un atome d'oxygène ou d'un atome de soufre pour former un anneau ; et L₁ représente un groupe de liaison divalent ou une liaison simple.

8. Dispositif EL organique selon la revendication 6, dans lequel le composé d'arylamine ayant une structure dans laquelle deux à six structures de triphénylamine sont reliées au sein d'une molécule par le biais d'une liaison simple ou d'un groupe divalent qui ne contient pas d'hétéroatome est un composé d'arylamine de la formule générale suivante (4) : 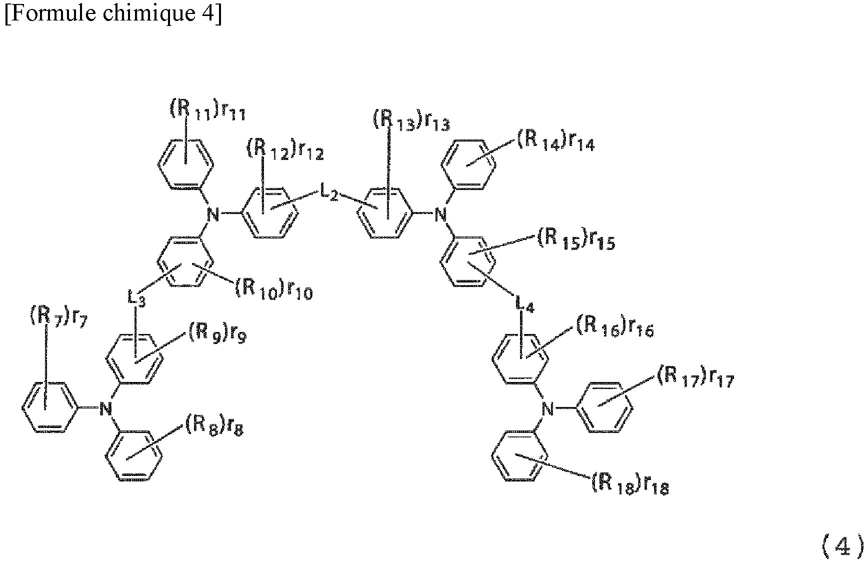 dans laquelle R₇ à R₁₈ représentent un atome de deutérium, un atome de fluor, un atome de chlore, cyano, nitro, alkyle linéaire ou ramifié de 1 à 6 atomes de carbone qui peuvent avoir un substituant, cycloalkyle de 5 à 10 atomes de carbone qui peuvent avoir un substituant, alkényle linéaire ou ramifié de 2 à 6 atomes de carbone qui peuvent avoir un substituant, alkyloxy linéaire ou ramifié de 1 à 6 atomes de carbone qui peuvent avoir un substituant, cycloalkyloxy de 5 à 10 atomes de carbone qui peuvent avoir un substituant, un groupe hydrocarbure aromatique substitué ou non substitué, un groupe hétérocyclique aromatique substitué ou non substitué, un groupe aromatique polycyclique condensé substitué ou non substitué, ou aryloxy substitué ou non substitué ; r₇ à r₁₈ peuvent être identiques ou différents, r₇, r₈, r₁₁, r₁₄, r₁₇ et r₁₈ représentant un entier de 0 à 5, et r₉, r₁₀, r₁₂, r₁₃, r₁₅ et r₁₆ représentant un entier de 0 à 4, où lorsque r₇, r₈, r₁₁, r₁₄, r₁₇ et r₁₈ sont un entier de 2 à 5, ou lorsque r₉, r₁₀, r₁₂, r₁₃, r₁₅ et r₁₆ sont un entier de 2 à 4, R₇ à R₁₈ dont une pluralité se lient au même anneau benzénique, peuvent être identiques ou différents et peuvent se lier l'un à l'autre par le biais d'une liaison simple, de méthylène substitué ou non substitué, d'un atome d'oxygène ou d'un atome de soufre pour former un anneau ; et L₂, L₃ et L₄ peuvent être identiques ou différents, représentent un groupe de liaison divalent ou une liaison simple.

9. Dispositif EL organique selon l'une quelconque des revendications 1 à 8, dans lequel la couche de transport d'électrons (6) inclut un composé représenté par la formule générale suivante (5) ayant une structure d'anneau d'anthracène : 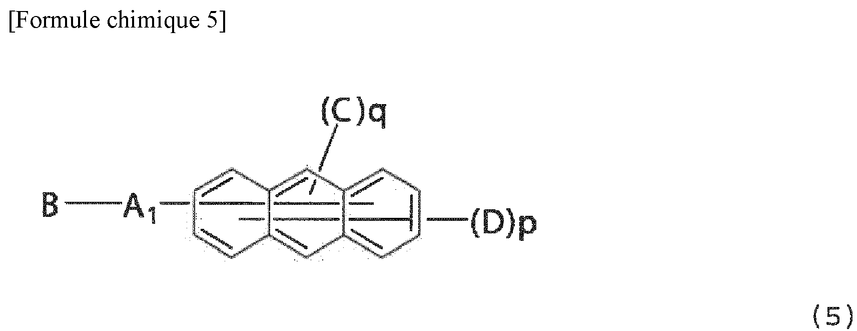 dans laquelle A₁ représente un groupe divalent d'un hydrocarbure aromatique substitué ou non substitué, un groupe divalent d'un anneau hétérocyclique aromatique substitué ou non substitué, un groupe divalent de composés aromatiques polycycliques condensés substitués ou non substitués, ou une liaison simple ; B représente un groupe hétérocyclique aromatique substitué ou non substitué ; C représente un groupe hydrocarbure aromatique substitué ou non substitué, un groupe hétérocyclique aromatique substitué ou non substitué, ou un groupe aromatique polycyclique condensé substitué ou non substitué ; D peut être identique ou différent, et représente un atome d'hydrogène, un atome de deutérium, un atome de fluor, un atome de chlore, cyano, trifluorométhyle, alkyle linéaire ou ramifié de 1 à 6 atomes de carbone, un groupe hydrocarbure aromatique substitué ou non substitué, un groupe hétérocyclique aromatique substitué ou non substitué, ou un groupe aromatique polycyclique condensé substitué ou non substitué ; et p représente 7 ou 8, et q représente 1 ou 2 tout en maintenant une relation selon laquelle une somme de p et q est de 9.

10. Dispositif électroluminescent organique selon l'une quelconque des revendications 1 à 9, dans lequel la couche d'émission lumineuse (5) inclut un dopant d'émission de lumière bleue.

11. Dispositif électroluminescent organique selon la revendication 10, dans lequel la couche d'émission lumineuse (5) inclut un dérivé de pyrène qui est un dopant d'émission de lumière bleue.

12. Dispositif électroluminescent organique selon l'une quelconque des revendications 1 à 11, dans lequel la couche d'émission lumineuse (5) inclut un dérivé d'anthracène.

13. Dispositif EL organique selon la revendication 12, dans lequel la couche d'émission lumineuse (5) inclut un matériau hôte qui est le dérivé d'anthracène.
